# EUROPEAN PATENT APPLICATION

(11) **EP 4 703 374 A1**
(43) Date of publication of application: **04.03.2026**
(21) Application number: 24796232.7
(22) Date of filing: 26.04.2024
(51) Int. Cl.: C07K 14/705, C07K 16/28, A61K 38/17, A61K 39/395, C07K 14/725, C12N 15/88, A61K 48/00, A61K 31/713

(54) **MODIFIED DELIVERY VECTOR AND USE THEREOF**

(30) Priority: 28.04.2023 WO PCT/CN2023/091679; 09.04.2024 WO PCT/CN2024/086804
(71) Applicant: Shenzhen Shenxin Biotechnology Co., Ltd., Shenzhen, Guangdong 518000 (CN)
(72) Inventor: LI, Linxian, Shenzhen, Guangdong 518000 (CN); JIN, Liang, Shenzhen, Guangdong 518000 (CN)
(74) Representative: Dentons Patent Solutions Rechtsanwaltsgesellschaft mbH
(86) International application number: PCT/CN2024/090028
(87) International publication number: WO 2024/222859

(57) **Abstract**

The present invention relates to a modified delivery carrier and use thereof. The modified delivery carrier comprises a delivery carrier and a targeting domain coupled to the delivery carrier, wherein the delivery carrier is used for loading an agent, and the targeting domain is capable of specifically targeting a surface antigen of an immune cell.

## Description

### Technical Field

The present invention belongs to the field of biotechnology, and relates to a modified delivery carrier and use thereof.

### Background Art

Cell therapy is a therapeutic means of injecting living cells into a patient to treat diseases, for example, T cell-based cell therapy (for example, CAR-T cell therapy) involves collecting autologous T cells from human body followed by in vitro culturing and modification to expand them by thousands of times and enhance their targeting killing ability, and then infusing them back into patients so as to kill pathogens, mutated cells (such as cancer cells) and others in blood and tissues. For another example, B-cell based cell therapy utilizes gene editing to modify B cells by inserting at a specific location in its genome a gene encoding a protein that needs to be expressed to obtain reprogrammed B cells which then are expanded and differentiated into plasma cells that secrete a therapeutic protein (including a therapeutic antibody) and finally these plasma cells are infused into patients, thereby treating a rare disease due to deficiency of a particular protein.

At present, although the therapeutic effect of cell therapy has been supported by some clinical trials, the preparation of personalized cells is complicated, costly and time-consuming, and patient's vein is required to be connected to acquisition devices for a long time when collecting autologous cells, resulting in poor patient comfort.

### Summary

Based on this, in order to solve the problems of complex operation and poor patient comfort brought by in vitro cell engineering, the present invention provides a modified delivery carrier comprising a delivery carrier for loading agents and a targeting domain coupled to the delivery carrier. Compared with traditional cell therapy, when the modified delivery carrier of the present invention is used for prevention or treatment, it is not necessary to collect autologous cells from body, or modify the collected cells in vitro, so the operation is simpler, and the patient comfort is higher. In addition, the present invention further provides a method of preparing the modified delivery carrier, a kit for preparing the modified delivery carrier, a pharmaceutical composition comprising the modified delivery carrier, and its use in the preparation of a medicament.

A modified delivery carrier comprising a delivery carrier and a targeting domain coupled to the delivery carrier, wherein the delivery carrier is for loading an agent and the targeting domain is capable of specifically targeting a surface antigen of an immune cell.

In some embodiments, the delivery carrier is one selected from lipid nanoparticle, liposome, cationic protein, vesicle, microparticle, polymer, and micelle.

In some embodiments, the immune cell includes one or more of T cell, B cell, NK cell, DC cell, neutrophil, eosinophil, basophil, mast cell, monocyte, and macrophage; in some embodiments, the immune cell is B cell, T cell, or NK cell.

In some embodiments, the surface antigen comprises one or more of the following:
a pan-T cell antigen, a pan-B cell antigen, a pan-NK cell antigen, a pan-DC cell antigen, a pan-neutrophil antigen, a pan-eosinophil antigen, a pan-basophil antigen, a pan-monocyte antigen, and a pan-macrophage antigen; in some embodiments, the surface antigen is a pan-B cell antigen.

In some embodiments, the surface antigen comprises one or more of:
CD1, CD2, CD5, CD6, CD9, CD10, CD11, CD17, CD18, CD19, CD20, CD21, CD22, CD23, CD24, CD25, CD26, CD27, CD29, CD30, CD31, CD32a, CD32b, CD35, CD37, CD38, CD39, CD40, CD44, CD45, CD45RA, CD45RB, CD45RC, CD46, CD47, CD48, CD49b, CD49c, CD49d, CD50, CD52, CD53, CD54, CD55, CD58, CD60a, CD62L, CD63, CD68, CD69, CD70, CD72, CD73, CD74, CD75, CD75S, CD77, CD79a, CD79b, CD80, CD81, CD82, CD83, CD84, CD85E, CD85I, CD85J, CD85L, CD85M, CD86, CD92, CD95, CD97, CD98, CD99, CD100, CD102, CD108, CD119, CD120, CD121, CD122, CD124, CD125, CD126, CD127, CD130, CD132, CD137, CD138, CD139, CD147, CD148, CD150, CD151, CD152, CD162, CD164, CD166, CD167a, CD170, CD171, CD175, CD175s, CD179, CD180, CD184, CD185, CD192, CD196, CD197, CD200, CD205, CD210, CD212, CD213a1, CD213a2, CD215, CD217, CD218, CD220, CD221, CD222, CD224, CD225, CD226, CD227, CD229, CD230, CD232, CD245, CD252, CD253, CD257, CD258, CD261, CD262, CD263, CD264, CD267, CD268, CD269, CD270, CD272, CD274, CD275, CD277, CD279, CD283, CD289, CD290, CD295, CD298, CD300a, CD300c, CD305, CD306, CD307, CD314, CD315, CD316, CD317, CD319, CD321, CD327, CD328, CD329, CD338, CD351, CD352, CD353, CD354, CD355, CD357, CD358, CD360, CD361, CD362, CD363, CD367, CD369 and GPRC5D;

In some embodiments, the surface antigen comprises one or more of: CD10, CD19, CD20, CD21, CD22, CD23, CD27, CD32b, CD38, CD40, CD49d, CD52, CD79a, CD79b, CD80, CD86, CD126, CD138, CD267, CD268, CD269, CD275, CD351, CD360 and GPRC5D; in some embodiments, the surface antigen comprises one or two of CD19 and CD22.

In some embodiments, the targeting domain comprises one or more of a nucleic acid, a polypeptide, an antibody, and a small molecule.
In some embodiments, the targeting domain is an antibody;
In some embodiments, the antibody is one or more selected from: an anti-CD10 antibody, an anti-CD19 antibody, an anti-CD20 antibody, an anti-CD21 antibody, an anti-CD22 antibody, an anti-CD23 antibody, an anti-CD27 antibody, an anti-CD32b antibody, an anti-CD38 antibody, an anti-CD40 antibody, an anti-CD49d antibody, CD52 antibody, an anti-CD79a antibody, an anti-CD79b antibody, an anti-CD80 antibody, an anti-CD86 antibody, an anti-CD126 antibody, an anti-CD138 antibody, an anti-CD267 antibody, an anti-CD268 antibody, an anti-CD269 antibody, an anti-CD275 antibody, an anti-CD351 antibody, an anti-CD360 antibody and an anti-GPRC5D antibody; in some embodiments, the targeting domain is one or two of an anti-CD19 antibody and an anti-CD22 antibody.

In some embodiments, the targeting domain is coupled to the delivery carrier in a coupling manner of covalent coupling or non-covalent coupling;
In some embodiments, the non-covalent coupling includes one or more of electrostatic interaction, van der Waals force, ring stacking, and hydrophobic interaction.

In some embodiments, the targeting domain has a first coupling group, and the delivery carrier has a second coupling group, wherein the first coupling group and the second coupling group are independently selected from or derived from one or more of sulfhydryl, azido, amino, amido, a polypeptide whose amino acid sequence is LPXTG, carbonyl, an avidin, a sulfhydryl reactive functional group, alkynyl, alkenyl, an amino reactive functional group, polyglycine, aminooxy (-O-NH₂) and biotin, wherein X is any amino acid, and in some embodiments, the avidin is streptavidin;
In some embodiments, one of the first coupling group and the second coupling group is selected from or derived from one or more of sulfhydryl, azido, amino, amido, a polypeptide whose amino acid sequence is LPXTG, carbonyl, and an avidin, and the other is selected from or derived from one or more of a sulfhydryl reactive functional group, alkynyl, alkenyl, an amino reactive functional group, polyglycine, aminooxy (-O-NH₂), and biotin;
In some embodiments, one of the first coupling group and the second coupling group is sulfhydryl, and the other is a sulfhydryl-reactive functional group; or one of the first coupling group and the second coupling group is azido, and the other is alkenyl or alkynyl; or one of the first coupling group and the second coupling group is amino, and the other is an amino-reactive functional group; or one of the first coupling group and the second coupling group is amido, and the other is amino; or one of the first coupling group and the second coupling group is a polypeptide whose amino acid sequence is LPXTG, and the other is polyglycine; or one of the first coupling group and the second coupling group is avidin or streptavidin, and the other is biotin; or one of the first coupling group and the second coupling group is carbonyl, and the other is aminooxy (-O-NH₂).

In some embodiments, the delivery carrier is a lipid nanoparticle;
In some embodiments, the lipid nanoparticle comprises a polymer-lipid, and the targeting domain is coupled to the polymer-lipid;
In some embodiments, the polymer used to form the polymer-lipid comprises one or both of: a hydrophilic polymer and an amphoteric polymer;
In some embodiments, the hydrophilic polymer comprises one or more of polyethylene glycol, polyoxazoline, polyglycerol, poly(hydroxypropyl methacrylate), poly(2-hydroxyethyl methacrylate), poly(N-(2-hydroxypropyl) methacrylamide), poly(vinylpyrrolidone), poly(N,N-dimethylacrylamide), poly(N-acryloyl morpholine), glycosaminoglycan, heparin, hyaluronic acid, polysialic acid, elastin like polypeptide, serum albumin and CD47;
In some embodiments, the amphoteric polymer comprises one or more of poly(carboxybetaine), poly(sulfobetaine), phosphobetaine-based polymer, and phosphorylcholine polymer.

In some embodiments, the delivery carrier is loaded with an agent; in some embodiments, the agent is encapsulated within the delivery carrier;
In some embodiments, the agent includes one or more of: a therapeutic agent, an imaging agent, a diagnostic agent, a contrast agent, a labeling agent, a detection agent, and a disinfectant.

In some embodiments, the agent comprises a therapeutic agent comprising one or more of: a nucleic acid, a polypeptide, a protein, an antibody, and a small molecule.

In some embodiments, the agent comprises a therapeutic agent, and an active ingredient of the therapeutic agent comprises a nucleic acid;
In some embodiments, the nucleic acid comprises DNA and/or RNA; in some embodiments, the nucleic acid comprises one or more of mRNA, nucleic acid based on RNAi technology and sgRNA; in some embodiments, the nucleic acid based on RNAi technology is one or more selected from siRNA, antisense oligonucleotide and miRNA;
In some embodiments, the DNA or the mRNA comprises one or more of a DNA or mRNA encoding a Cas protein, a DNA or mRNA encoding a CAR, a DNA or mRNA encoding a TCR, and a DNA or mRNA encoding a therapeutic protein or polypeptide;
In some embodiments, the mRNA is an mRNA encoding a Cas protein, an mRNA of CAR, or an mRNA of TCR;
In some embodiments, the DNA is a DNA encoding a therapeutic protein or polypeptide;
In some embodiments, the therapeutic protein is an antibody, or the therapeutic protein is a protein or polypeptide for treating a rare disease;
In some embodiments, the nucleic acid comprises a gRNA and an mRNA encoding a Cas protein;
In some embodiments, the Cas protein is Cas9;
In some embodiments, the nucleic acid comprises a modified nucleotide.

In some embodiments, the mRNA comprised in the agent further comprises a regulatory element;
In some embodiments, the regulatory element comprises a microRNA binding site;
In some embodiments, the mRNA comprised in the agent comprises one or more microRNA binding sites; in some embodiments, the microRNA binding sites are the same or at least partially different;
In some embodiments, the microRNA binding sites are different, and the microRNA binding sites bind to the same microRNA or different microRNAs;
In some embodiments, the microRNA binding sites bind to different microRNAs, the different microRNAs are from the same cell and/or tissue, or the different microRNAs are from different cells and/or tissues;
In some embodiments, the mRNA in the modified delivery carrier comprises 3'-UTR, and the position of the microRNA binding site is selected from one or more of 5' end close to 3'-UTR, a region between 5' end and 3' end of 3'-UTR, and 3' end close to 3'-UTR;
In some embodiments, the mRNA in the modified delivery carrier comprises 5'-UTR, and the position of the microRNA binding site is selected from one or more of 5' end close to 5'-UTR, a region between 5' end and 3' end of 5'-UTR, and 3' end close to 5'-UTR;
In some embodiments, the mRNA in the modified delivery carrier comprises 5'-UTR, 3'-UTR, and a microRNA binding site, wherein the position of the microRNA binding site is one selected from a 5' end closed to 3'-UTR, a region between the 5' and 3' ends of 3'-UTR, a 3' end closed to 3'-UTR, a 5' end closed to 5'-UTR, a region between the 5' and 3' ends of the 5' - UTR, and a 3' end close to 5'-UTR;
In some embodiments, the mRNA in the modified delivery carrier comprises 5'-UTR, 3'-UTR, and microRNA binding sites, wherein the positions of the microRNA binding sites are one or more selected from a 5' end close to 3'-UTR, a region between 5' end and 3' end of 3'-UTR, a 3' end close to 3'-UTR, a 5' end close to 5'-UTR, a region between 5' end and the 3' end of the 5'-UTR, and a 3' end close to the 5'-UTR.

In some embodiments, the modified delivery carrier comprises a plurality of targeting domains coupled to the delivery carrier;
In some embodiments, the plurality of targeting domains are independently coupled to the delivery carrier, or at least one of the plurality of targeting domains is coupled to the delivery carrier, and at least one targeting domain of the remaining is coupled to the targeting domain coupled to the delivery carrier.

A preparation method of the modified delivery carrier, comprising steps of:
mixing raw materials for preparing a delivery carrier with an agent to prepare an agent-loaded delivery carrier; and
coupling a targeting domain to the agent-loaded delivery carrier to produce a modified delivery carrier, wherein the targeting domain is capable of specifically targeting a surface antigen of an immune cell.

A pharmaceutical composition, the modified delivery carrier described above.

In some embodiments, the pharmaceutical composition comprises at least two of the modified delivery carriers, the modified delivery carriers each loaded with different agents or the targeting domains of the delivery carriers each are different.

Use of the delivery carrier or the pharmaceutical composition described above in the preparation of a gene-edited immune cell;
In some embodiments, the gene-edited immune cell comprises one or more of: B cell, T cell, NK cell, DC cell, neutrophil, eosinophil, basophil, mast cell, monocyte, and macrophage; in some embodiments, the gene-edited immune cell is B cell, T cell, or NK cell;
In some embodiments, the gene-edited immune cell is CAR-T cell, TCR-T cell or CAR-NK cell, and the delivery carrier of the modified delivery carrier is loaded with a nucleic acid encoding a CAR or TCR; in some embodiments, the nucleic acid encoding a CAR or TCR is an mRNA encoding a CAR or TCR;
or, the gene-edited immune cell is B cell, and the modified delivery carrier is loaded with sgRNA, nucleic acid encoding a Cas protein, and nucleic acid encoding a therapeutic protein; in some embodiments, the therapeutic protein is a protein for treating a rare disease, or the therapeutic protein is an antibody; in some embodiments, the nucleic acid encoding a Cas protein is an mRNA encoding a Cas protein, and the nucleic acid encoding a therapeutic protein is a DNA.

Use of the modified delivery carrier or the pharmaceutical composition described above in the preparation of a medicament;
In some embodiments, the medicament is used for treating and/or preventing a disease;
In some embodiments, the modified delivery carrier targets B cell, and the medicament is used for treating a B-cell tumor;
or the modified delivery carrier targets T cell, and the medicament is used for treating a T cell tumor;
or the modified delivery carrier targets NK cell, and the medicament is used for treating a NK cell tumor;
In some embodiments, the modified delivery carrier comprises one or more of: a nucleic acid, a polypeptide, a protein, and a small molecule;
In some embodiments, the nucleic acid comprises one or two of RNA and DNA;

In some embodiments, the nucleic acid comprises mRNA, or the nucleic acid comprises a nucleic acid based on RNAi technology; in some embodiments, the nucleic acid based on RNAi technology is selected from one or more of siRNA, antisense oligonucleotide, and miRNA.
Use of the modified delivery carrier or the pharmaceutical composition described above in the preparation of a medicament;
In some embodiments, the medicament is used for treating and/or preventing a disease;
In some embodiments, the modified delivery carrier targets B cell, and the modified delivery carrier is loaded with an sgRNA, a nucleic acid encoding a Cas protein, and a nucleic acid encoding a therapeutic protein; in some embodiments, the therapeutic protein is a protein for treating a rare disease, or the therapeutic protein is an antibody;
In some embodiments, the nucleic acid encoding a Cas protein is an mRNA encoding a Cas protein; and/or the nucleic acid encoding a therapeutic protein is a DNA.

Use of the modified delivery carrier or the pharmaceutical composition described above in the preparation of a medicament;
In some embodiments, the medicament is used for treating and/or preventing a disease;
In some embodiments, the modified delivery carrier targets T cell, and the modified delivery carrier is loaded with a nucleic acid encoding a desired protein; in some embodiments, the nucleic acid is mRNA. In some embodiments, the desired protein is CAR or TCR; furthermore, the modified delivery carrier is loaded with an mRNA encoding a CAR or TCR;
or, the modified delivery carrier targets NK cell, and the modified delivery carrier is loaded with a nucleic acid encoding a desired protein; in some embodiments, the nucleic acid is mRNA. In some embodiments, the desired protein is CAR; furthermore, the modified delivery carrier is loaded with an mRNA encoding a CAR.

In some embodiments, the medicament is used for treating and/or preventing one or more of a rare disease, cancer, infectious disease, autoimmune disease, metabolic disease, neuropathic disease, cardiovascular disease, transplant rejection response, inflammatory response, hereditary disease and musculoskeletal disease;
In some embodiments, the rare disease includes one or more of Brittle Bone Disease, Wilson disease, spinal muscular atrophy, Huntington's disease, Rett syndrome, amyotrophic lateral sclerosis, Duchenne Type Muscular Dystrophy, Friedrichs ataxia, Methylmalonic Acidemia, cystic fibrosis, glycogen storage disease 1a, glycogen storage disease III, Crigler-Najjar syndrome, ornithine transcarbamylase deficiency, Propionic Acidemia, phenylketonuria, hemophilia A, hemophilia B, β-thalassemia, Lafora disease, Dravet syndrome, Alexander disease, Leber's congenital amaurosis, myelodysplastic syndromes, and Homocystinuria due to CBS deficiency;
the cancer comprises one or more of hematological malignancy, lung cancer, liver cancer, kidney cancer, head and neck cancer, esophageal cancer, gastric cancer, colorectal cancer, pancreatic cancer, brain cancer, prostate cancer, gallbladder cancer, ovarian cancer, breast cancer, cervical cancer, endometrial cancer, bladder cancer, melanoma;
the infectious disease comprises a disease caused by infection with one or more of virus, fungus, and bacterium;
the autoimmune disease comprises one or more of acute idiopathic thrombocytopenic purpura, chronic idiopathic thrombocytopenic purpura, systemic lupus erythematosus, rheumatoid arthritis, psoriasis, inflammatory bowel disease, multiple sclerosis, celiac disease, type 1 diabetes mellitus, diffuse toxic goiter;
the hereditary disease comprises one or more of hemophilia, thalassemia, and Gaucher's disease;
The neuropathic disease includes one or more of amyotrophic lateral sclerosis, Alzheimer's disease, and glioma.

A kit for preparing a modified delivery carrier, comprising:
reagents for preparing a delivery carrier;
an agent comprising or preparing a targeting domain, wherein the targeting domain is capable of specifically targeting a surface antigen of an immune cell; and
a linking agent for coupling the targeting domain to the delivery carrier.

### Brief Description of Figures

FIG. 1 shows representative flow cytometry results of eGFP expression levels in B cells of PBMCs in the groups LNP, LNP-eGFP, and anti-CD19/LNP-eGFP;
FIG. 2 shows the eGFP expression level in B cells of PBMC in the groups LNP, LNP-eGFP and anti-CD19/LNP-eGFP, wherein the ordinate is the content of eGFP⁺ in B cells in a unit of %, and the abscissa is the group;
FIG. 3 shows the eGFP expression level in B cells and T cells in the groups LNP, LNP-eGFP and anti-CD19/LNP-eGFP, wherein the ordinate is the content of eGFP⁺ in the cells in a unit of %, and the abscissa is the group.
FIGs. 4A to 4B show the TdTomato expression level in T cells in the groups LNP, LNP-Cre and T-LNP-Cre, wherein the ordinate is the TdTomato content in T cells in a unit of %, and the abscissa is the group. FIG. 4A shows the TdTomato expression level in T cells of the spleen in the groups LNP, LNP-Cre and T-LNP-Cre, and FIG. 4B shows the TdTomato expression level in the T cells of the PBMC in the groups LNP, LNP-Cre and T-LNP-Cre.
FIGs. 5A to 5B show the comparison of the expression levels of TdTomato in T cells and non-T cells in the groups LNP, LNP-Cre and T-LNP-Cre, wherein the ordinate is the content of TdTomato in the cells in a unit of %, and the abscissa is the group. FIG. 5A shows the comparison of TdTomato expression levels in T cells and non-T cells of spleen in the groups LNP, LNP-Cre and T-LNP-Cre, and FIG. 5B shows the comparison of TdTomato expression levels in T cells and non-T cells of PBMC in the groups LNP, LNP-Cre, and T-LNP-Cre.

### Detailed description of the invention

### I. Definitions

All patents, patent applications, scientific publications, manufacturers' instructions and guidelines, etc. cited herein are hereby incorporated by reference in their entirety, irrespective of the foregoing and following context. Nothing herein is to be construed as an admission that the invention is not entitled to precede such disclosure.

Unless otherwise specified, scientific and technical terms used herein have the meanings commonly understood by those skilled in the art. Moreover, the protein and nucleic acid chemistry, molecular biology, cell and tissue culture, microbiology related terms used herein are all widely used in the respective fields (see, e.g., Molecular Cloning: A Laboratory Manual, 2nd Edition, J. Sambrook et al. eds., Cold Spring Harbor Laboratory Press, Cold Spring Harbor 1989). Meanwhile, in order to better understand the invention, definitions and explanations of related terms are provided below.

As used herein, the expressions "comprise", "include", "contain" and "have" are openended, meaning that the listed elements, steps or components are included but other unrecited elements, steps or components are not excluded. The expression "consist of' excludes any element, step, or component not specified. The expression "consist essentially of" means that the range is limited to the specified elements, steps, or components, plus optional elements, steps, or components that do not significantly affect the basic and novel properties of the claimed subject matter. It will be understood that the expressions "consist essentially of" and "consist of' are encompassed within the meaning of the expression "comprise".

As used herein, the singular form "a", "an", "this" or "the" and similar referents in the context of describing the invention (especially in the context of the claims) are to be construed to cover both the singular and the plural, unless clearly indicated otherwise from the context. The terms "one or more" or "at least one" encompass 1, 2, 3, 4, 5, 6, 7, 8, 9 or more.

The numerical ranges recited herein are understood to encompass any and all sub-ranges subsumed therein. For example, a range of "1 to 10" should be understood to include not only the explicitly recited values 1 and 10, but also any single value in the range of 1 to 10 (e.g., 2, 3, 4, 5, 6, 7, 8 and 9) and sub-ranges (e.g., 1 to 2, 1.5 to 2.5, 1 to 3, 1.5 to 3.5, 2.5 to 4, 3 to 4.5, etc.). This principle also applies to ranges that use only one value as the minimal or maximal value.

All methods described herein can be performed in any suitable order unless otherwise indicated.

As used herein, the term "wild-type" or "WT" refers to a biomolecule that is the same as a biological molecule (e.g., polynucleotide, polypeptide, or protein) occurred in nature, meaning that the sequence is naturally occurring and is not artificially modified, including naturally occurring mutants.

The term "fragment" or "fragment of nucleic acid" refers to a portion of a nucleic acid. For example, nucleic acids shortened at 5' and/or 3' ends. The fragment of nucleic acid comprises at least 50%, 60%, 70%, or 80% from the nucleic acid. Preferably, the fragment of nucleic acid comprises at least 70% or 80% from the nucleic acid. Preferred are at least 90%, 95%, 96%, 97%, 98% or 99% of nucleotide residues. Generally, it may be a shorter portion of the full length of a nucleic acid.

The term "variant" in reference to a nucleic acid refers to a nucleic acid variant, at least one nucleotide of which is different from a reference nucleic acid (or "parent"). Variant nucleic acid includes a single one or more nucleotide deletions, additions, mutations, and/or insertions as compared to a reference nucleic acid, wherein: the deletion includes removal of one or more nucleotides from the reference nucleic acid; the addition includes fusing one or more nucleotides (e.g., 1, 2, 3, 5, 10, 20, 30, 50 or more nucleotides) to 5' and/or 3' ends of the reference nucleic acid; the mutation may include, but not limited to, substitution (e.g., at least one nucleotide is removed and another nucleotide is inserted at its position (e.g., transversion and transformation)); the insertion includes adding at least one nucleotide. The term "nucleic acid variant" as used herein includes naturally occurring variants and engineered variants. Thus, "nucleic acid variant" as defined herein can be derived from a reference nucleic acid, isolated from a reference nucleic acid, correlated to, based on, or homologous to a reference nucleic acid sequence. A "nucleic acid variant" may optionally have a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98% or 99%; preferably at least 70%, more preferably at least 80%, even more preferably at least 85%, even more preferably at least 90%, most preferably at least 95% or even 97% to the corresponding naturally occurring (wild-type) nucleic acid or homologue, fragment or derivative thereof. It will be appreciated that for a nucleic acid molecule, the term "variant" includes a degenerate nucleic acid sequence in which the degenerate nucleic acid sequence according to the invention is a nucleic acid that is different from the reference nucleic acid in respect of codon sequences due to the degeneracy of genetic code.

As used herein, the term "% identity" refers to the percentage of nucleotides or amino acids that are the same in the optimal alignment between sequences to be compared. The difference between two sequences may be distributed over a local region (segment) or the entire length of the sequence to be compared. The identity between two sequences is typically determined after the optimal alignment over the segment or "comparison window". Optimal alignment may be performed manually, or by means of algorithms known in the art, including, but not limited to, the local homology algorithm described by Smith and Waterman, 1981, Ads App. Math. 2, 482 and Needleman and Wunsch, 1970, J. Mol. Biol. 48, 443, the similarity search method described by Pearson and Lipman, 1988, Proc. Natl Acad. Sci. USA 88, 2444, or using a computer program such as GAP, BESTFIT, FASTA, BLASTP, BLASTN, and TFASTA in Wisconsin Genetics Software Package, Genetics Computer Group, 575 Science Drive, Madison, Wis. For example, the percentage identity of two sequences may be determined using BLASTN or BLASTP algorithm publicly available in the National Center for Biotechnology Information (NCBI) website.

"% identity" may be obtained by determining the number of identical positions corresponding to the sequence to be compared, divided by the number of the positions compared (e.g., the number of positions in the reference sequence), and then multiplied by 100 to obtain % identity. In some embodiments, at least about 50%, at least about 55%, at least about 60%, at least about 65%, at least about 70%, at least about 75%, at least about 80%, at least about 85%, at least about 90%, at least about 95%, or about 100% of the area gives the degree of identity. In some embodiments, the degree of identity is given over the full length of the reference sequence. Alignment of sequence identity may be determined using tools known in the art, preferably with optimal sequence alignment, e.g., using Align, in standard settings, preferably EMBOSS::needle, Matrix:Blosum62, Gap Open 10.0, Gap Extend 0.5.

As used herein, "nucleotide" includes deoxyribonucleotides, ribonucleotides, derivatives of deoxyribonucleotides, and derivatives of ribonucleotides. As used herein, "ribonucleotide" is a constituent of ribonucleic acid (RNA), consisting of one molecule base, one molecule pentose and one molecule phosphate, which refers to a nucleotide having a hydroxyl group at 2' position of β-D-ribofuranosyl group, and "deoxyribonucleotide" is a constituent of deoxyribonucleic acid (DNA), also consisting of one molecule base, one molecule pentose and one molecule phosphate, which refers to a nucleotide with hydrogen substituting for hydroxyl at 2' position of β-D-ribofuranosyl group, and is the main chemical component of chromosome.

"Nucleotide" is generally referred to by a single letter representing the base thereof: "A" or "A nucleotide" refers to adenine deoxyribonucleotide or adenine ribonucleotide containing adenine, "C" or "C nucleotide" refers to cytosine deoxyribonucleotide or cytosine ribonucleotide containing cytosine, "G" or "G nucleotide" refers to guanine deoxyribonucleotide or guanine ribonucleotide containing guanine, "U" or "U nucleotide" refers to uracil ribonucleotide containing uracil, "T" or "T nucleotide" refers to thymine deoxyribonucleotide containing thymine.

As used herein, the term "nucleic acid" refers generally to a polymer comprising deoxyribonucleotides (deoxyribonucleic acid, abbreviated as DNA) or a polymer comprising ribonucleotides (ribonucleic acid, abbreviated as RNA) or any compound of the combination thereof. In addition, nucleic acid herein also includes derivatives of nucleic acid. The term "derivatives of nucleic acid" includes chemical derivatization of nucleic acids on base, sugar and/or phosphate of nucleotides, and/or nucleic acids containing non-natural nucleotides and nucleotide analogs. In addition, as used herein, a nucleic acid may be in a form of single-stranded or double-stranded linear or covalently closed cyclic molecule.

"Polynucleotide sequence," "nucleic acid sequence," and "nucleotide sequence" are used interchangeably to denote the ordering of nucleotides in a polynucleotide. Those skilled in the art should understand that the DNA coding strand (sense strand) and the RNA encoded thereby can be regarded as having the same nucleotide sequence, and deoxythymidylate in the DNA coding strand sequence corresponds to the uridylate in the RNA sequence encoded thereby.

As used herein, the term "expression" includes transcription and/or translation of a nucleotide sequence. Thus, expression may involve the production of transcripts and/or polypeptides. The term "transcription" involves the process of transcribing a genetic code in a DNA sequence into RNA (transcript). The term "in vitro transcription" refers to the in vitro synthesis of RNA, in particular mRNA, in a cell free system (see, e.g., Pardi N., Muramatsu H., Weissman D., Karikó K. (2013). In: Rabinovich P. (eds) Synthetic Messenger RNA and Cell Metabolism Modulation. Methods in Molecular Biology (Methods and Protocols), vol 969. Humana Press, Totowa, NJ.). A vector that may be used to produce transcripts is also known as a "transcription vector," which contains regulatory sequences required for transcription. The term "transcription" encompasses "in vitro transcription."

As used herein, the term "host cell" refers to a cell for receiving, holding, replicating, expressing a polynucleotide or vector. The term "host cell" encompasses prokaryotic cells (e.g., *E. coli)* or eukaryotic cells (e.g., yeast cells and insect cells), e.g. cells from humans, mice, hamsters, pigs, goats, primates. The cells may be derived from a variety of tissue types and include primary cells and cell lines. Some specific examples include keratinocytes, peripheral blood leukocytes, bone marrow stem cells, and embryonic stem cells. In other embodiments, the host cell is an antigen presenting cell, in particular dendritic cell, monocyte, or macrophage. Nucleic acids may be present in the host cell in a single copy or in multiple copies. In some embodiments, the host cell may be a cell in which the polypeptide of the invention is expressed.

In the context of the present invention, the term "plasmid" generally refers to a cyclic DNA molecule, and may also encompass linearized DNA molecules. In particular, the term "plasmid" also encompasses a molecule obtained by linearizing a cyclic plasmid by digesting the cyclic plasmid, e.g., with a restriction enzyme, thereby converting the cyclic plasmid molecule into a linear molecule. Plasmid can be replicated, i.e., amplified in cells independently of genetic information stored as chromosomal DNA, and can be used for cloning, i.e., for amplification of genetic information in bacterial cells. Preferably, the DNA plasmid is a medium-copy or a high-copy plasmid, more preferably a high-copy plasmid. Examples of such high-copy plasmid include, for example, pUC and pTZ plasmid or any other plasmids containing an origin of replication supporting high copy number of plasmid (e.g. pMB1, pCoIE1).

As used herein, the term "treatment" or the like is used herein to generally mean obtaining a desired pharmacological and/or physiological effect. Thus, the treatment of the invention may involve the treatment of a state of a certain disease, but may also involve prophylactic treatment in terms of complete or partial prevention of a disease or a symptom thereof. Preferably, the term "treatment" is understood to be therapeutic in partial or complete cure of a disease and/or an adverse effect and/or a symptom resulted from the disease. The treatment may also be a prophylactic or preventive treatment, i.e., a measure taken to prevent a disease, e.g., to prevent infection and/or the onset of disease.

As used herein, the term "antibody" is used in its broadest sense, and specifically includes, but not limited to, monoclonal antibodies (including full-length monoclonal antibodies), polyclonal antibodies, multispecific antibodies (e.g., bispecific antibodies), and antibody fragments as long as they exhibit the desired antigen-binding activity.

The term "CDR" refers to complementary determining region (CDR), which is a part of variable domains in an immunoglobulin (antibody), and is also referred to as an antigen binding site where an antigen is in contact with an antibody. CDRs are where the variability in immunoglobulins (antibodies) are greatest to capture different antigens and are also commonly referred to as hypervariable regions. The amino acid sequence of an immunoglobulin (antibody) variable domain has three CDRs (CDR1, CDR2 and CDR3) which are discontinuous and distributed on two polypeptide chains (light chain and heavy chain). HCDR1, HCDR2 and HCDR3 refer to three CDRs on the heavy chain, and LCDR1, LCDR2 and LCDR3 refer to three CDRs on the light chain.

The terms "full-length antibody," "intact antibody," and "whole antibody" are used interchangeably herein to refer to an antibody in a substantially intact form rather than an antibody fragment as defined below. An "antibody fragment" refers to an antigen-binding fragment of an antibody and an antibody analog, which generally includes an antigen-binding region or a variable region (e.g., one or more CDRs) of at least a portion of parental antibody. The antibody fragment retains at least some binding specificity of parental antibody. Generally, antibody fragment retains at least 10% of binding activity of parental antibody when activity is expressed in mol. Preferably, antibody fragment retains at least 20%, 50%, 70%, 80%, 90%, 95%, or 100% or more of binding affinity of parental antibody to target. Examples of antibody fragment include: Fab fragments, Fab' fragments, F(ab')₂ fragments, Fv fragments, and single-chain antibody molecules (e.g., scFv); diabodies; linear antibodies; unibodies (techniques from Genmab); heavy chain antibodies, nanobodies (techniques from Domantis); single-domain antibodies (techniques from Abbynx); and multi-specific antibodies formed from antibody fragments.

"Fab fragment" consists of a light chain and a CH1 and a variable region of a heavy chain. The heavy chain of Fab cannot form a disulfide bond with the heavy chain of another Fab. "Fc fragment" comprises two heavy chain fragments comprising the CH2 and CH3 domains of antibody that are held together by one or more disulfide bonds and by the hydrophobic action of the CH3 domains. "Fab' fragment" comprises a light chain and a partial heavy chain comprising a VH domain, a CH1 domain, and a region between the CH1 and CH2 domains, whereby an interchain disulfide bond can be formed between the two heavy chains of the two Fab' to form F(ab')₂ fragment. Fab' fragment differs from Fab fragment in that Fab' fragment adds some residues at the carboxy terminus of the heavy chain CH1 domain, including one or more cysteines from antibody hinge region. "F(ab')₂ fragment" comprises two light chains and two partial heavy chains comprising the VH domains, CH1 domains, and the regions between CH1 and CH2 domains, thereby forming an interchain disulfide bond between the two heavy chains. Thus, the F(ab')₂ fragment consists of two Fab' fragments held together by disulfide bonds between the two heavy chains. For example, an antibody digested with papain produces two identical antigen-binding fragments (each fragment has a single antigen-binding site, which may be referred to as "Fab fragment") and a left "Fc fragment" (its name reflecting its ability to crystallize readily). For example, pepsin treatment can produce F(ab')2 fragments that have two antigen binding sites and can still be cross-linked with antigen.

"Fv fragment" comprises variable regions from both the heavy and light chains, but lacks constant regions, which is a minimal antibody fragment comprising a complete antigen-binding site.

"Single-chain Fv fragment" or "scFv fragment" comprises the VH and VL domains of an antibody, wherein these domains are present in a single polypeptide chain. Generally, the scFv fragment further comprises a polypeptide linker between the VH and VL domains, allowing the scFv to form the desired antigen binding structure. For review of scFv, see, for example, The Pharmacology of Monoclonal Antibodies of Pluckthun, Volume 113, eds by Rosenburg and Moore, (Springer-Verlag, New York, 1994), pages 269-315.

The term "diabody" or "double-stranded antibody" is artificial antibody, which is formed by connecting two single-stranded molecules in a certain manner, and can bind two antigens at the same time. The diabody may be bivalent or bispecific antibody. Diabody is more fully described, for example, in EP 404,097; WO 1993/01161; Hudson et al., Nat. Med. 9:129-134 (2003); and Hollinger et al., Proc. Natl. Acad. Sci. USA 90:6444-6448(1993). Hudson et al. also describe tribody and tetrabody in Nat. Med. 9:129-134(2003).

"Heavy chain antibody" refers to an antibody fragment comprising only VH, CH2, and CH3 and having immunological function.

"Single-domain antibody" or "SdAb" is an immunologically functional antibody fragment comprising only heavy chain variable region or light chain variable region. In some cases, two or more VHs are covalently linked to a peptide linker to form a divalent single-domain antibody. The two VH of the divalent single-domain antibody can target the same or different antigens.

"Nanobody" refers to an antibody fragment comprising only VH and having immunological function, also known as VHH antibody.

As used herein, the term "monoclonal antibody" refers to an antibody obtained from a substantially homogeneous population of antibody, i.e., the individual antibodies that make up the population are consistent except for possible naturally occurring mutations that may be present in small amounts. Monoclonal antibodies are highly specific and can be directed to a single antigenic site. In addition, in contrast to conventional (polyclonal) antibody preparations which typically include a plurality of different antibodies against a plurality of different determinants (epitopes), each monoclonal antibody is directed only to a single determinant on antigen. The modifier "monoclonal" refers to a characteristic of an antibody obtained from a substantially homogeneous population of antibody and cannot be understood as requiring the preparation of the antibody by any particular method. For example, monoclonal antibodies for use in the present invention can be prepared by hybridoma method described first by Kohler et al. (1975, Nature 256:495), or can be prepared by recombinant DNA methods (see, e.g., U.S. Pat. No. 4,816,567). "Monoclonal antibodies" can also be isolated from phage antibody libraries using the techniques described in such as Crackson et al. (1991, Nature352:624-628) and Marks et al(1991, J. Mol. Biol. 222:581-597). The monoclonal antibodies herein expressly include "chimeric" antibodies. The term "chimeric antibody" is intended to mean an antibody in which the variable region sequence is derived from one species and the constant region sequence is derived from another species, such as an antibody in which the variable region sequence is derived from a mouse antibody and the constant region sequence is derived from a human antibody.

The term "couple" refers to an association between atoms or molecules. The association may be direct (e.g., via a covalent bond) or indirect (e.g., via a non-covalent bond). Non-covalent bond includes, but not limited to, electrostatic interaction (e.g., ionic bond, hydrogen bond, halogen bond), Van der Waals force, ring stacking (π effect), and hydrophobic interaction.

As used herein, the term "administer" refers to providing or giving an agent to a subject via any effective route. Exemplary routes of administration include, but not limited to, one or more of injection (e.g., subcutaneous, intramuscular, intradermal, intraperitoneal, intrathecal, intracerebroventricular, or intravenous), oral, intraluminal, sublingual, rectal, transdermal, intranasal, vaginal, and inhalation. When used to treat a disease, disorder, condition or symptom thereof, administration of substance is typically performed following the onset of the disease, disorder, condition or symptom thereof. When used to prevent a disease, disorder, condition or symptom, administration of substance is typically performed prior to the onset of the disease, disorder, condition or symptom.

As used herein, the term "therapeutic agent" refers to any medicament that can be used to treat, prevent, or alleviate a disease, disorder, or condition, including for treating, preventing or alleviating one or more symptoms of a disease, disorder, or condition and associated symptoms.

Some elements of the invention will be described herein. These elements and specific embodiments are set forth together, it should be understood, however, that they may be combined in any manner and in any number to generate additional embodiments. For example, in one embodiment, the polymer-lipid of the modified delivery carrier comprises polyethylene glycol-PEG, in another embodiment, the polymer-lipid and the targeting domain are coupled via action of biotin and avidin, the following embodiment is one embodiment to be protected in the invention: the polymer-lipid of the modified delivery carrier comprises polyethylene glycol-PEG, and the polymer-lipid and the targeting domain are coupled via action of biotin and avidin. Furthermore, the examples and preferred embodiments described differently shall not be construed as limiting the invention to the specifically described embodiments. This specification should be understood as supporting and including embodiments that combine specifically described embodiments with any number of the disclosed and/or preferred elements. In addition, unless the context indicates otherwise, any permutation and combination of all the described elements in the present invention should be considered as being disclosed by the specification of the present invention.

### II. Modified Delivery Carrier

The present invention provides a modified delivery carrier comprising a delivery carrier for loading an agent and a targeting domain for specifically targeting a surface antigen of an immune cell, wherein the targeting domain is coupled to the delivery carrier.

In some embodiments, the delivery carrier is a non-viral carrier.

In some embodiments, the delivery carrier is selected from one or a combination of a plurality of lipid nanoparticles (LNPs), liposomes, cationic proteins, vesicles, microparticles, polymers, and micelles. In some embodiments, the delivery carrier is one selected from the group consisting of lipid nanoparticles, liposomes, cationic proteins, vesicles, microparticles, polymers, and micelles.

In some embodiments, the delivery carrier is lipid nanoparticles (LNPs).

In some embodiments, lipid nanoparticles refer to particles at a nanometer scale (e.g., 1 nm to 1000 nm), which includes one or more lipids. It should be noted that "a plurality of" herein means at least two.

In some embodiments, the lipid nanoparticles have an average diameter of 20nm to 800nm, 20nm to 500nm, 20nm to 400nm, 20nm to 300nm, 20nm to 200nm, 20nm to 100nm, 30nm to 700nm, 30nm to 500nm, 30nm to 300nm, 30nm to 200nm, 30nm to 100nm, 40nm to 800nm, 40nm to 600nm, 40nm to 500nm, 40nm to 300nm, 40nm to 200nm, 40nm to 100nm, 50nm to 800nm, 50nm to 600nm, 50nm to 500nm, 50nm to 500nm, 50nm to 400nm, 50nm to 500nm, 50nm to 400nm, 50nm to 300nm, 50nm to 200nm, 50nm to 100nm, 60nm to 800nm, 60nm to 600nm, 60nm to 500nm, 60nm to 400nm, 60nm to 300nm, 60 nm to 200 nm or 60 nm to 100 nm. In some optional specific examples, the lipid nanoparticles have an average diameter of 26nm, 31nm, 36nm, 41nm, 46nm, 51nm, 56nm, 61nm, 66nm, 71nm, 76nm, 81nm, 86nm, 91nm, 96nm, 101nm, 106nm, 111nm, 116nm, 121nm, 126nm, 131nm, 136nm, 141nm, 146nm, 151nm, 156nm, 161nm, 166nm, 171nm, 176nm, 181nm, 186nm, 191nm, 196nm, 201nm, 206nm, 211nm, 216nm, 221nm, 226nm, 231nm, 236nm, 241nm, 246 nm or 249 nm. Herein, the average diameter of the lipid nanoparticles may be represented by z-average determined by dynamic light scattering.

In some embodiments, the lipid nanoparticles include one of cationic lipid nanoparticles, solid lipid nanoparticles (SLN), nanostructured lipid carriers (NLC), nonlamellar lipid nanoparticles. In an optional specific example, the lipid nanoparticles are cationic lipid nanoparticles.

In some embodiments, the lipid nanoparticles comprise one or more of a cationic lipid, a helper lipid, a structural lipid, and a polymer-lipid.

The term "cationic lipid" refers to a positively charged lipid when pH is lowered below the pKa of the ionizable group of the lipid, but gradually becomes neutral at a higher pH, and a positively charged lipid can bind to a negatively charged nucleic acid when pH is below the pKa. In certain embodiments, the cationic lipid comprises zwitterionic lipid.

In some embodiments, the cationic lipid comprises the compound (I), an N-oxide thereof, a salt thereof, or an isomer thereof: 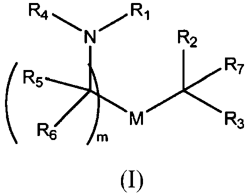 wherein:
R₁ is selected from the group consisting of C₅-C₃₀ alkyl, C₅-C₂₀ alkenyl, -R*YR", -YR", and -R"M'R';
R₂ and R₃ are independently selected from the group consisting of H, C₁-C₁₄ alkyl, C₂-C₁₄ alkenyl, -R*YR", -YR" and -R*OR", or R₂ and R₃ together with the atoms to which they are attached form a heterocycle or a carbocyclic ring;
R₄ is selected from the group consisting of hydrogen, C₃-C₆ carbocyclic ring, -(CH₂)ₙQ, -(CH₂)ₙCHQR, -(CH₂)ₒC(R₁₀)₂(CH₂)ₙ₋ₒQ, -CHQR, -CQ(R)₂ and unsubstituted C₁-C₆ alkyl, wherein Q is selected from the group consisting of carbocyclic ring, heterocycle, -OR, - O(CH₂)ₙN(R)₂, -C(O)OR, -OC(O)R, -CX₃, -CX₂H, -CXH₂, -CN, -N(R)₂, -C(O)N(R)₂, - N(R)C(O)R, -N(R)S(O)₂R, -N(R)C(O)N(R)₂, -N(R)C(S)N(R)₂, N(R)R₈, -N(R)S(O)₂R₈, - O(CH₂)ₙOR, -N(R)C(=NR₉)N(R)₂, -N(R)C(=CHR₉)N(R)₂, -OC(O)N(R)₂, -N(R)C(O)OR, - N(OR)C(O)R, -N(OR)S(O)₂R, -N(OR)C(O)OR, -N(OR)C(O)N(R)₂, -N(OR)C(S)N(R)₂, - N(OR)C(=NR₉)N(R)₂, -N(OR)C(=CHR₉)N(R)₂, -C(=NR₉)N(R)₂, -C(=NR₉)R, - C(O)N(R)OR and -C(R)N(R)₂C(O)OR, each o is independently selected from 1, 2, 3 and 4, and each n is independently selected from 1, 2, 3, 4 and 5;
each R₅ is independently selected from the group consisting of OH, C₁-C₃ alkyl, C₂-C₃ alkenyl, and H;
each R₆ is independently selected from the group consisting of OH, C₁-C₃ alkyl, C₂-C₃ alkenyl, and H;
M and M' are independently selected from the group consisting of -C(O)O-, -OC(O)-, - OC(O)-M"-C(O)O-, -C(O)N(R')-, -N(R')C(O)-, -C(O)-, -C(S)-, -C(S)S-, -SC(S)-, -CH(OH)-, -P(O)(OR')O-, -S(O)₂-, -S-S-, aryl and heteroaryl group, wherein M" is a bond, C₁-C₁₃ alkyl, or C₂₋-C₁₃ alkenyl;
R₇ is selected from the group consisting of C₁₋₃ alkyl, C₂-C₃ alkenyl, and H;
R₈ is selected from the group consisting of a C₃₋₆ carbocyclic ring and a heterocycle;
R₉ is selected from the group consisting of H, CN, NO₂, C₁-C₆ alkyl, -OR, -S(O)₂R, - S(O)₂N(R)₂, C₂-C₆ alkenyl, C₃-C₆ carbocyclic ring, and heterocycle;
R₁₀ is selected from the group consisting of H, C₁-C₃ alkyl and C₂-C₃ alkenyl;
each R is independently selected from the group consisting of C₁-C₃ alkyl, C₂-C₃ alkenyl, (CH₂)_{q}OR*, and H,
and each q is independently selected from 1, 2 and 3;
each R' is independently selected from the group consisting of C₁-C₁₈ alkyl, C₂-C₁₈ alkenyl, -R*YR", -YR", H and 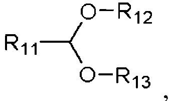 and R_{11,} R₁₂ and R₁₃ are each independently selected from the group consisting of C₁-C₁₂ alkyl and C₂-C₁₂ alkenyl;
each R" is independently selected from the group consisting of C₃-C₁₅ alkyl and C₃-C₁₅ alkenyl;
each R* is independently selected from the group consisting of absence, C₁-C₁₂ alkyl, and C₂-C₁₂ alkenyl;
each Y is independently a C₃-C₆ carbocyclic ring;
each X is independently selected from the group consisting of F, Cl, Br, and I; and
m is selected from 5, 6, 7, 8, 9, 10, 11, 12 and 13; and wherein, when R₄ is -(CH₂)ₙQ, - (CH₂)ₙCHQR, -CHQR, or -CQ(R)₂, then (i) Q is not -N(R)₂ when n is 1, 2, 3, 4 or 5; or (ii) Q is not 5, 6 or 7-membered heterocycloalkyl when n is 1 or 2.

In an optional specific example, the cationic lipid is the following compound (I), an N-oxide thereof, a salt thereof, or an isomer thereof: 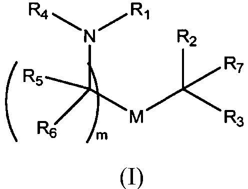 where R₁-R₇, M, and m are as previously defined.

In some embodiments, the cationic lipid comprises the following compound (II), an N-oxide thereof, a salt thereof, or an isomer thereof: 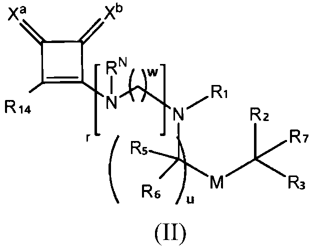 wherein:
R₁, R₂, R₃, R₅, R₆, M and R₇ are as described above,
R^{N} is H or C₁-C₃ alkyl;
X^{a} and X^{b} are each independently O or S;
R₁₄ is selected from the group consisting of H, halogen, -OH, R^{b}, -N(R^{b})₂, -CN, -N₃, - C(O)OH, -C(O)OR^{b}, -OC(O)R^{b}, -OR^{b}, -SR^{b}, -S(O)R^{b}, -S(O)OR^{b}, -S(O)₂OR^{b}, -NO₂, - S(O)₂N(R^{h})₂, -N(R^{b})S(O)₂R^{b}, -NH(CH₂)ₜ₁N(R^{b})₂, -NH(CH₂)ₚ₁O(CH₂)_{q1}N(R^{b})₂, - NH(CH₂)ₛ₁OR^{b}, -N((CH₂)_{S}OR^{b})₂, -N(R^{b})-carbocyclic ring, -N(R^{b})- heterocycle, N(R^{b})-aryl, - N(R^{b})-heteroaryl, -N(R^{b})(CH₂)ₜ₁-carbocyclic ring, -N(R^{b})(CH₂)ₜ₁-heterocycle, -N(R^{b})(CH₂)ₜ₁₋aryl, -N(R^{b})(CH₂)ₜ₁-heteroaryl, carbocyclic ring, heterocycle, aryl and heteroaryl;
each R^{b} is independently selected from the group consisting of C₁-C₃ alkyl, C₂-C₃ alkenyl, and H;
u is 5, 6, 7, 8, 9, 10, 11, 12 or 13;
w is 1, 2, 3, 4, 5, 6, 7, 8, 9 or 10;
r is 0 or 1;
t¹ is 1, 2, 3, 4 or 5;
p¹ is 1, 2, 5, 4 or 5;
q¹ is 1, 2, 5, 4 or 5; and
s¹ is 1, 2, 3, 4 or 5.

In an optional specific example, the cationic lipid is the following compound (II), an N-oxide thereof, a salt thereof, or an isomer thereof: 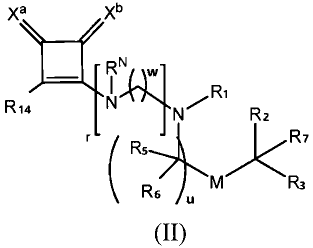 where R₁-R₃, R₅-R₇, R₁₄, X^{a}, X^{b}, R^{N}, M, u, w, and r are as previously defined.

In some embodiments, the cationic lipid comprises the following compound (III), an N-oxide thereof, a salt thereof, or an isomer thereof: 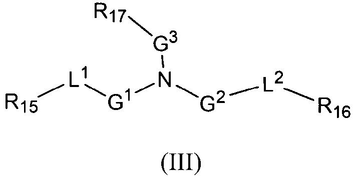 wherein:
one of L¹ or L² is-O(C=O)-, -(C=O)O-, -C(=O)-, -O-, -S(O)ₓ-, -S-S-, -C(=O)S-, - SC(=O)-, -NR^{a}C(=O)-, -C(=O)NR^{a}-, NR^{a}C(=O)NR^{a}-, -OC(=O)NR^{a}- 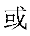 -NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or -NR^{a}C(=O)O- or a bond;
G¹ and G² are each independently an unsubstituted C₁-C₁₂ alkylene or C₁-C₁₂ alkenylene;
G³ is C₁-C₂₄ alkylene, C₁-C₂₄ alkenylene, C₃-C₈ cycloalkylene, C₃-C₈ cycloalkenylene;
R^{a} is H or C₁-C₁₂ hydrocarbyl;
R₁₅ and R₁₆ are each independently C₆-C₂₄ alkyl or C₆-C₂₄ alkenyl;
R₁₇ is H, OR₁₈, CN, -C(=O)OR₁₉, -OC(=O)R₁₉ or -NR₁₈C(=O)R₁₉;
R₁₉ is C₁-C₁₂ hydrocarbyl;
R₁₈ is H or C₁-C₆ hydrocarbyl; and
x is 0, 1 or 2.

In an optional specific example, the cationic lipid is the following compound (III), an N-oxide thereof, a salt thereof, or an isomer thereof: 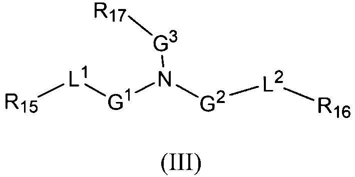 where: R₁₅-R₁₇, G¹-G³, L¹-L² are as defined above.

In some embodiments, the cationic lipid includes one or two of ALC-0315(CAS number 2036272-55-4) and SM-102 (CAS number 2089251-47-6).

In some embodiments, the helper lipid of the lipid nanoparticle comprises phospholipids. Phospholipids are typically semi-synthetic or naturally derived or chemically modified. In an alternative specific example, the helper lipid of the lipid nanoparticle is phospholipid. In some embodiments, the phospholipid of the lipid nanoparticle includes one or more of DSPC (distearoyl phosphatidylcholine), DOPE (dioleoylphosphatidylethanolamine), DOPC (dioleoylphosphatidylcholine), DOPS (dioleoylphosphatidylserine), DSPG (1,2-distearoyl-sn-glycero-3-phospho-(1'-rac-glycerol)), DPPG (dipalmitoylphosphatidylglycerol), DPPC (dipalmitoylphosphatidylcholine), DGTS (1,2-dipalmitoyl-sn-glycerol-3-O-4'-(N,N,N-trimethyl)homoserine) and lysophospholipids. In some embodiments, the helper lipid of the lipid nanoparticle is one or more selected from the group consisting of DSPC, DOPE, DOPC, and DOPS. In some embodiments, the helper lipid of the lipid nanoparticle is DSPC and/or DOPE.

In some embodiments, the helper lipid is DSPC.

In some embodiments, the structural lipid of the lipid nanoparticle comprises sterol. In an optional specific example, the structural lipid of the lipid nanoparticle is sterol. In some embodiments, the sterol of the lipid nanoparticle includes one or more of 20α-hydroxy cholesterol, cholesterol, cholesteryl ester, steroid hormone, sterol vitamin, bile acid, cholesterol, ergosterol, β-sitosterol, and oxidized cholesterol derivatives. In some embodiments, the structural lipid of the lipid nanoparticle includes at least one of cholesterol, cholesteryl ester, steroid hormone, sterol vitamin, and bile acid. In some embodiments, the structural lipid of the lipid nanoparticle is cholesterol. Cholesterol of high purity, particularly injection grade high purity cholesterol, e.g., CHO-HP (produced by AVT), is preferred. In other embodiments, the structured lipid is 20α-hydroxy cholesterol.

In some embodiments, the structural lipid is cholesterol.

Polymer-lipid refers to a conjugate comprising a polymer and a lipid coupled to the polymer. The polymer-lipid (e.g., polyethylene glycol-lipid) in the lipid nanoparticle can improve the stability of the lipid nanoparticle in vivo.

In some embodiments, the lipid of the polymer-lipid for forming the lipid nanoparticle includes one or more of myristoyl diacylglycerol (1,2-dimyristoyl-sn-glycerool, DMG), distearoyl-phosphatidylethanolamine (DSPE), diacylglycerol (DAG), dialkyloxypropyl (DAA), phospholipid, ceramide (Cer), 1,2-distearoyl-rac-glycerol (DSG) and 1,2-dipalmitoyl-rac-glycero (DPG).

In some embodiments, the polymer in the polymer-lipid for forming the lipid nanoparticle comprises one or both of hydrophilic polymer and amphoteric polymer.

In some embodiments, the polymer in the polymer-lipid for forming the lipid nanoparticle is hydrophilic polymer. In other embodiments, the polymer in the polymer-lipid for forming the lipid nanoparticle is amphoteric polymer.

In some embodiments, the hydrophilic polymer includes one or more of polyethylene glycol (PEG), polyoxazolines (POX), poly(glycerols) (PGs), poly(hydroxypropyl methacrylate) (PHPMA), poly(2-hydroxyethyl methacrylate) (PHEMA), poly(N-(2-hydroxypropyl) methacrylamide) (HPMA), poly(vinylpyrrolidone) (PVP), poly(N,N-dimethyl acrylamide) (PDMA), poly(N-acryloyl morpholine) (PAcM), polyaminoacids, glycosaminoglycans (GAGs), heparin, hyaluronic acid (HA), polysialic acid (PSA), elastin like polypeptide (ELPs), serum albumin, and CD47.

Correspondingly, the polymer-lipid includes one or more of polyethylene glycol-lipid (PEG-lipid), polyoxazoline-lipid, polyglycerol-lipid, poly(hydroxypropyl methacrylate)-lipid, poly(2-hydroxyethyl methacrylate)-lipid, poly(N-(2-hydroxypropyl) methacrylamide)-lipid, poly(vinylpyrrolidone)-lipid, poly(N,N-dimethyl acrylamide)-lipid, poly(N-acryloyl morpholine)-lipid, glycosaminoglycan-lipid, heparin-lipid, hyaluronic acid-lipid, polysialic acid-lipid, elastin like polypeptide-lipid, serum albumin-lipid, and CD47-lipid. It should be noted that "PEG-lipid" is a conjugate of polyethylene glycol and lipid, "polyoxazoline-lipid" refers to a conjugate formed by coupling polyoxazoline to lipid, and "polyglycerol-lipid" refers to a conjugate formed by coupling polyglycerol to lipid, and other polymer-lipids are the same. In one optional specific example, the hydrophilic polymer comprises polyethylene glycol.

In some embodiments, the polymer-lipid comprises PEG-lipid. In one alternative specific example, the polymer-lipid is PEG-lipid. In some embodiments, the PEG-lipid includes one or more of PEG-myristoyl diacylglycerol (PEG-DMG), PEG-distearoylphosphatidylethanolamine (PEG-DSPE), PEG-diacylglycerol (PEG-DAG), PEG-dialkyloxypropyl (PEG-DAA), PEG-phospholipid, PEG-ceramide (PEG-Cer), PEG-1,2-distearoyl-rac-glycerol (PEG-DSG), and PEG-1,2-dipalmitoyl-rac-glycerol (PEG-DPG). The PEG-lipid is preferably PEG-DMG, PEG-DSG, PEG-DPG. PEG-DMG is a polyethylene glycol derivative of glyceride 1,2-dimyristate. In some embodiments, the PEG in the PEG-lipid has an average molecular weight of about 2000 to 5000, preferably about 2000.

In some embodiments, the amphoteric polymer includes one or more of poly(carboxybetaine) (pCB), poly(sulfobetaine) (pSB), phosphobetaine-base polymers and phosphorylcholine polymer_{∘} In some embodiments, the amphoteric polymer includes one or more of poly(carboxybetaine acrylamide) (pCBAA), poly(carboxybetaine methacrylate), poly(sulfobetaine methacrylate), poly(methacryloyloxyethyl phosphorylcholine), poly(vinyl-pyridinio propanesulfonate), poly(carboxybetaine) based on vinylimidazole, poly(sulfobetaine) based on vinylimidazole, and poly(sulfobetaine) based on vinylpyridine.

Correspondingly, the polymer-lipid comprises one or more of poly(carboxybetaine)-lipid, poly(sulfobetaine)-lipid, phosphobetaine-base polymer-lipid, and phosphorylcholine polymer-lipid. In some embodiments, the polymer-lipid comprises one or more of poly(carboxybetaine acrylamide)-lipid, poly(carboxybetaine methacrylate)-lipid, poly(sulfobetaine methacrylate)-lipid, poly(methacryloyloxyethyl phosphorylcholine)-lipid, poly(vinyl-pyridinio propanesulfonate)-lipid, poly(carboxybetaine) based on vinylimidazole-lipid, poly(sulfobetaine) based on vinylimidazole-lipid, and poly(sulfobetaine) based on vinylpyridine-lipid.

Further, in some embodiments, the polymers applied to nanoparticle described in Hoang Thi, Thai Thanh et al., "The Importance of Poly(ethylene glycol) Alternatives for Overcoming PEG Immunogenicity in Drug Delivery and Bioconjugation." Polymers vol. 12,2 298, are also incorporated herein.

In some embodiments, the PEG-lipid is DSPE-PEG-Maleimide (DSPE-PEG-Mal).

In some embodiments, the PEG-lipid is DSPE-PEG2000-Mal.

In some embodiments, the lipid nanoparticle comprises cationic lipid, helper lipid, structural lipid and polymer-lipid (e.g., PEG-lipid). In some embodiments, based on the total amount of cationic lipid, helper lipid, structural lipid and polymer-lipid, the lipid nanoparticle comprises the cationic lipid in an amount (mole percentage) of about 25.0% to 75.0%, such as about 25.0% to 28.0%, 28.0%-32.0%, 32.0%-35.0%, 35.0%-40.0%, 40.0%-42.0%, 42.0%-45.0%, 45.0%-46.3%, 46.3%-48.0%, 48.0%-49.5%, 49.5%-50.0%, 50.0%-55.0%, 55.0%-60.0%, 60.0%-65.0% or 65.0% to 75.0%.

In some embodiments, based on the total amount of cationic lipid, helper lipid, structural lipid and polymer-lipid (e.g., PEG-lipid), the lipid nanoparticle described above comprises the helper lipid in an amount (mole percentage) of 5%-45%, such as 5%-9%, 9%-9.4%, 9.4%-10%, 10%-10.5%, 10.5%-11%, 11%-15%, 15%-16%, 16%-18%, 18%-20%, 20%-25%, 25%-33.5%, 33.5%-37%, 37%-40%, 40%-42% or 42%-45%.

In some embodiments, based on the total amount of cationic lipid, helper lipid, structural lipid and polymer-lipid (e.g., PEG-lipid), the lipid nanoparticle described above comprises the structural lipid in an amount (mole percentage) of 0%-50%, such as 0%-10%, 10%-15.5%, 15.5%-18.5%, 18.5%-22.5%, 22.5%-23.5%, 23.5%-28.5%, 28.5%-33.5%, 33.5%-35%, 35%-36.5%, 36.5%-38%, 38%-38.5%, 38.5%-39%, 39%-39.5%, 39.5%-40.5%, 40.5%-41.5%, 41.5%-42.5%, 42.5%-42.7%, 42.7%-43%, 43%-43.5%, 43.5%-45%, 45%-46.5%, 46.5%-48.5% or 46.5%-50%.

In some embodiments, based on the total amount of cationic lipid, helper lipid, structural lipid and polymer-lipid (e.g., PEG-lipid), the lipid nanoparticle described above comprises the polymer-lipid in an amount (mole percentage) of 0.5%-5%, such as 0.5%-1%, 1%-1.5%, 1.5%-1.6%, 1.6%-2%, 2%-2.5%, 2.5%-3%, 3%-3.5%, 3.5%-4%, 4%-4.5% or 4.5%-5%.

In some embodiments, the lipid nanoparticle comprises a cationic lipid, a helper lipid, a structural lipid and a polymer-lipid, wherein the cationic lipid is ALC-0315, the helper lipid is DSPC, the structural lipid is cholesterol, and the polymer-lipid is DSPE-PEG-Mal.

In some embodiments, the cationic lipid is ALC-0315, the helper lipid is DSPC, the structural lipid is cholesterol and the polymer-lipid is DSPE-PEG-Mal, and based on the total amount of the cationic lipid, the helper lipid, the structural lipid and the polymer-lipid, the molar ratio of the cationic lipid, the helper lipid, the structural lipid and the polymer-lipid is (25-75): (5-45): (0-50): (0.5-5).

In some embodiments, the cationic lipid is ALC-0315, the helper lipid is DSPC, the structural lipid is cholesterol and the polymer-lipid is DSPE-PEG-Mal, and based on the total amount of the cationic lipid, the helper lipid, the structural lipid and the polymer-lipid, the molar ratio of the cationic lipid, the helper lipid, the structural lipid and the polymer-lipid is (40-55): (5-15): (35-45): (0.5-2.5).

In some embodiments, the nonlamellar lipid nanoparticle is one selected from ethosomes and echogenic liposomes.

In some embodiments, the delivery carrier comprises liposome. Liposome utilizes vesicle wrapping agent (e.g., therapeutic agent) formed from phospholipid bilayer membrane, and the components of liposome include phospholipid and cholesterol. In an optional specific example, the delivery carrier is liposome. In some embodiments, the delivery carrier is a liposome loaded with an agent.

In some embodiments, the delivery carrier comprises cationic protein. In an optional specific example, the delivery carrier is a cationic protein. In some embodiments, the cationic protein includes, but not limited to, protamine.

In some embodiments, the delivery carrier comprises polymer. In some embodiments, the delivery carrier comprises lipopolyplex (LPP) and/or hyaluronic acid polymer (e.g., hyaluronic acid gel). In an optional specific example, the delivery carrier is a lipopolyplex or hyaluronic acid gel. Lipopolyplex is a bilayer structure that encapsulates nucleic acid (e.g., mRNA) using polymer as kernel and lipids (e.g., phospholipid) as shell.

In some embodiments, the immune cell targeted by the targeting domain includes one or more of T cell, B cell, natural killer (NK) cell, DC cell, neutrophil, eosinophil, basophil, mast cell, monocyte and macrophage.

In some embodiments, the T cell targeted by the targeting domain includes one or more of cytotoxic T cell, helper T cell, memory T cell and effector T-cell. In some embodiments, the helper T cell includes one or more of follicular helper T cell, conventional helper CD4+ T cell (T_{H} cell), regulatory CD4+ T cell (Treg cell), cytotoxic CD8+ T cell (CTL, killer T cell), natural killer T cell (NKT cell), mucosa-associated invariant T cell (MAIT), and γδ T cell.

In some embodiments, the B cell targeted by the targeting domain includes one or more of plasmablast, plasma cell, lymphoplasmacytoid cell, memory B cell, B-2 cell (e.g., follicular (FO)B cell, marginal zone (MZ)B cell), B-1 cell and regulatory B cell.

In some embodiments, the NK cell targeted by the targeting domain includes one or more of CD27+CD11b+NK cell, CD27-CD11b+NK cell, CD56^{dim}NK cell and CD56^{bright}NK cell.

In some embodiments, the surface antigen of the immune cell targeted by the targeting domain includes one or more of pan-T cell antigen, pan-B cell antigen, pan-NK cell antigen, pan-DC cell antigen, pan-neutrophil antigen, pan-eosinophil antigen, pan-basophil antigen, pan-monocyte antigen and pan-macrophage antigen. It should be noted that the pan-T cell antigen (pan-T antigen) refers to a cell surface antigen expressed on all T cells, such as CD2, CD3, CD5, CD7, etc. The pan-B cell antigen, pan-NK cell antigen, pan-DC cell antigen, pan-neutrophil antigen, pan-eosinophil antigen, pan-basophil antigen, pan-monocyte antigen and pan-macrophage antigen are the same.

In some embodiments, the immune cell targeted by the targeting domain is not T cell or does not merely comprise T cell (e.g., T cell and B cell, or T cell and NK cell). In an optional specific example, the immune cell is B cell or NK cell.

In some embodiments, the surface antigen of the immune cell targeted by the targeting domain is one type. When the surface antigen is one type, the targeting domain can specifically target immune cells having the surface antigen. In some other embodiments, the surface antigen of the immune cell targeted by the targeting domain is more than one types, such as two, three, four or five. When the surface antigen is more than one types, the targeting domain specifically targets an immune cell having at least one antigen of the more than one types of the surface antigens. For example, when the surface antigens of the immune cell targeted by the targeting domain are CD19 and CD20, the targeting domain can target an immune cell with CD19, an immune cell with CD20, and an immune cell with both CD19 and CD20.

In some embodiments, the surface antigen of the immune cell targeted by the targeting domain includes one or more of

CD1, CD2, CD5, CD6, CD9, CD10, CD11, CD17, CD18, CD19, CD20, CD21, CD22, CD23, CD24, CD25, CD26, CD27, CD29, CD30, CD31, CD32a(Fc y RIIa), CD32b(Fc y R IIb), CD35, CD37, CD38, CD39, CD40, CD44, CD45, CD45RA, CD45RB, CD45RC, CD46, CD47, CD48, CD49b, CD49c, CD49d(Integrin a4), CD50, CD52, CD53, CD54, CD55, CD58, CD60a, CD62L, CD63, CD68, CD69, CD70, CD72, CD73, CD74, CD75, CD75S, CD77, CD79a, CD79b, CD80, CD81, CD82, CD83, CD84, CD85E, CD85I, CD85J, CD85L, CD85M, CD86, CD92, CD95, CD97, CD98, CD99, CD100, CD102, CD108, CD119, CD120, CD121, CD122, CD124, CD125, CD126(IL-6R), CD127, CD130, CD132, CD137, CD138, CD139, CD147, CD148, CD150, CD151, CD152, CD162, CD164, CD166, CD167a, CD170, CD171, CD175, CD175s, CD179, CD180, CD184, CD185, CD192, CD196, CD197, CD200, CD205, CD210, CD212, CD213a1, CD213a2, CD215, CD217, CD218, CD220, CD221, CD222, CD224, CD225, CD226, CD227, CD229, CD230, CD232, CD245, CD252, CD253, CD257, CD258, CD261, CD262, CD263, CD264, CD267(TACI), CD268(BAFFR), CD269(BCMA), CD270, CD272, CD274, CD275(ICOSL), CD277, CD279, CD283, CD289, CD290, CD295, CD298, CD300a, CD300c, CD305, CD306, CD307, CD314, CD315, CD316, CD317, CD319, CD321, CD327, CD328, CD329, CD338, CD351(IgM), CD352, CD353, CD354, CD355, CD357, CD358, CD360(IL-21R), CD361, CD362, CD363, CD367, CD369 and GPRC5D.

In some embodiments, the surface antigen of the immune cell targeted by the targeting domain includes one or more of CD10, CD19, CD20, CD21, CD22, CD23, CD27, CD32b, CD38, CD40, CD49d, CD52, CD79a, CD79b, CD80, CD86, CD126, CD138, CD267, CD268, CD269, CD275, CD351, CD360 and GPRC5D. In some embodiments, the surface antigen of the immune cell targeted by the targeting domain is one or more of CD10, CD19, CD20, CD21, CD22, CD23, CD27, CD32b, CD38, CD40, CD49d, CD52, CD79a, CD79b, CD80, CD86, CD126, CD138, CD267, CD268, CD269, CD275, CD351, CD360 and GPRC5D. In some embodiments, the surface antigen of the immune cell targeted by the targeting domain comprises CD19 and/or CD22.

In some embodiments, the surface antigen of the immune cell targeted by the targeting domain comprises one of the combinations CD19 and CD20, CD19 and CD22, CD19 and CD269, CD19 and CD268, CD19 and CD38, CD19 and CD37, CD19 and CD123, CD19 and CD79b, CD33 and CD123, CD269 and CD38, CD269 and GPRC5D, CD269 and CD319, as well as CD269 and CD267.

In some embodiments, the surface antigen of the immune cell targeted by the targeting domain is one of the combinations CD19 and CD20, CD19 and CD22, CD19 and CD269, CD19 and CD268, CD19 and CD38, CD19 and CD37, CD19 and CD123, CD19 and CD79b, CD33 and CD123, CD269 and CD38, CD269 and GPRC5D, CD269 and CD319, as well as CD269 and CD267.

In some embodiments, the targeting domain comprises one or more of nucleic acid, polypeptide, small molecule and antibody.

In some embodiments, the targeting domain is nucleic acid. In some embodiments, the targeting domain is an aptamer. Aptamer is a single-stranded oligonucleotide (such as single-stranded DNA or single-stranded RNA) with a specific recognition function, and its role is that single-stranded oligonucleotides are adaptively folded by various interaction forces such as hydrogen bond (for example, complementary pairing of nucleotide bases), π-π stacking, and electrostatic force to form specific three-dimensional structures, which can specifically bind to target molecules through intermolecular interaction forces.

In some embodiments, the targeting domain is a polypeptide that is capable of specifically binding to a surface antigen of an immune cell.

In some embodiments, the targeting domain is a small molecule capable of specifically binding to a surface antigen of an immune cell. Small molecule refers to a molecule having a molecular weight of less than 1000 Daltons. In some embodiments, a small molecule refers to a molecule having a molecular weight of less than 400 Daltons, 500 Daltons, 600 Daltons, or 700 Daltons.

In some embodiments, the targeting domain is an antibody that is a full-length antibody, antibody fragment, full-length antibody variant or antibody fragment variant capable of specifically targeting a surface antigen of an immune cell. Antibody fragment is a surface antigen binding fragment that specifically targets an immune cell. Examples of antibody fragment include: Fab fragments, Fab' fragments, F(ab')2 fragments and Fv fragments; diabodies; linear antibodies; single-chain antibodies (scFv); unibodies; nanobodies; single-domain antibodies; and multispecific antibodies formed from antibody fragments. In some embodiments, the full-length antibody variant and antibody fragment variant are preferably functional variants.

In some embodiments, the targeting domain is an antibody that is an IgG4 antibody.

In some embodiments, the targeting domain is an antibody comprising one or more of an anti-CD1 antibody, an anti-CD2 antibody, an anti-CD5 antibody, an anti-CD6 antibody, an anti-CD9 antibody, an anti-CD10 antibody, an anti-CD11 antibody, an anti-CD17 antibody, an anti-CD18 antibody, an anti-CD19 antibody, an anti-CD20 antibody, an anti-CD21 antibody, an anti-CD22 antibody, an anti-CD23 antibody, an anti-CD24 antibody, an anti-CD25 antibody, an anti-CD26 antibody, an anti-CD27 antibody, an anti-CD29 antibody, an anti-CD30 antibody, an anti-CD31 antibody, an anti-CD32a antibody, an anti-CD32b antibody, an anti-CD35 antibody, an anti-CD37 antibody, an anti-CD38 antibody, an anti-CD39 antibody, an anti-CD40 antibody, an anti-CD44 antibody, an anti-CD45, an anti-CD45RA antibody, an anti-CD45RB antibody, an anti-CD45RC antibody, an anti-CD46 antibody, an anti-CD47 antibody, an anti-CD48 antibody, an anti-CD49b antibody, an anti-CD49c antibody, an anti-CD49d antibody, an anti-CD50 antibody, an anti-CD52 antibody, an anti-CD53 antibody, an anti-CD54 antibody, an anti-CD55 antibody, an anti-CD58 antibody, an anti-CD60a antibody, an anti-CD62L antibody, an anti-CD63 antibody, an anti-CD68 antibody, an anti-CD69 antibody, an anti-CD70 antibody, an anti-CD72 antibody, an anti-CD73 antibody, an anti-CD74 antibody, an anti-CD75 antibody, an anti-CD75S antibody, an anti-CD77 antibody, an anti-CD79a antibody, an anti-CD79b antibody, an anti-CD80 antibody, an anti-CD81 antibody, an anti-CD82 antibody, an anti-CD83 antibody, an anti-CD84 antibody, an anti-CD85E antibody, an anti-CD85I antibody, an anti-CD85J antibody, an anti-CD85L antibody, an anti-CD85M antibody, an anti-CD86 antibody, an anti-CD92 antibody, an anti-CD95 antibody, an anti-CD97 antibody, an anti-CD98 antibody, an anti-CD99 antibody, an anti-CD100 antibody, an anti-CD102 antibody, an anti-CD108 antibody, an anti-CD119 antibody, an anti-CD120 antibody, an anti-CD121 antibody, an anti-CD122 antibody, an anti-CD124 antibody, an anti-CD125 antibody, an anti-CD126 antibody, an anti-CD127 antibody, an anti-CD130 antibody, an anti-CD132 antibody, an anti-CD137 antibody, an anti-CD138 antibody, an anti-CD139 antibody, an anti-CD147 antibody, an anti-CD148 antibody, an anti-CD150 antibody, an anti-CD151 antibody, an anti-CD152 antibody, an anti-CD162 antibody, an anti-CD164 antibody, an anti-CD166 antibody, an anti-CD167a antibody, an anti-CD170 antibody, an anti-CD171 antibody, an anti-CD175 antibody, an anti-CD175s antibody, an anti-CD179 antibody, an anti-CD180 antibody, an anti-CD184 antibody, an anti-CD185 antibody, an anti-CD192 antibody, an anti-CD196 antibody, an anti-CD197 antibody, an anti-CD200 antibody, an anti-CD205 antibody, an anti-CD210 antibody, an anti-CD212 antibody, an anti-CD213a1 antibody, an anti-CD213a2 antibody, an anti-CD215 antibody, an anti-CD217 antibody, an anti-CD218 antibody, an anti-CD220 antibody, an anti-CD221 antibody, an anti-CD222 antibody, an anti-CD224 antibody, an anti-CD225 antibody, an anti-CD226 antibody, an anti-CD227 antibody, an anti-CD229 antibody, an anti-CD230 antibody, an anti-CD232 antibody, an anti-CD245 antibody, an anti-CD252 antibody, an anti-CD253 antibody, an anti-CD257 antibody, an anti-CD258 antibody, an anti-CD261 antibody, an anti-CD262 antibody, an anti-CD263 antibody, an anti-CD264 antibody, an anti-CD267 antibody, an anti-CD268 antibody, an anti-CD269 antibody, an anti-CD270 antibody, an anti-CD272 antibody, an anti-CD274 antibody, an anti-CD275 antibody, an anti-CD277 antibody, an anti-CD279 antibody, an anti-CD283 antibody, an anti-CD289 antibody, an anti-CD290 antibody, an anti-CD295 antibody, an anti-CD298 antibody, an anti-CD300a antibody, an anti-CD300c antibody, an anti-CD305 antibody, an anti-CD306 antibody, an anti-CD307 antibody, an anti-CD314 antibody, an anti-CD315 antibody, an anti-CD316 antibody, an anti-CD317 antibody, an anti-CD319 antibody, an anti-CD321 antibody, an anti-CD327 antibody, an anti-CD328 antibody, an anti-CD329 antibody, an anti-CD338 antibody, an anti-CD351 antibody, an anti-CD352 antibody, an anti-CD353 antibody, an anti-CD354 antibody, an anti-CD355 antibody, an anti-CD357 antibody, an anti-CD358 antibody, an anti-CD360 antibody, an anti-CD361 antibody, an anti-CD362 antibody, an anti-CD363 antibody, an anti-CD367 antibody, an anti-CD369 antibody and an anti-GPRC5D antibody.

In some embodiments, the targeting domain is an antibody comprising one or more of an anti-CD10 antibody, an anti-CD19 antibody, an anti-CD20 antibody, an anti-CD21 antibody, an anti-CD22 antibody, an anti-CD23 antibody, an anti-CD27 antibody, an anti-CD32b antibody, an anti-CD38 antibody, an anti-CD40 antibody, an anti-CD49d antibody, CD52 antibody, an anti-CD79a antibody, an anti-CD79b antibody, an anti-CD80 antibody, an anti-CD86 antibody, an anti-CD126 antibody, an anti-CD138 antibody, an anti-CD267 antibody, an anti-CD268 antibody, an anti-CD269 antibody, an anti-CD275 antibody, an anti-CD351 antibody, an anti-CD360 antibody and an anti-GPRC5D antibody.

In some embodiments, the targeting domain is an antibody comprising one or more of an anti-CD19 antibody and an anti-CD20 antibody. In some embodiments, the targeting domain comprises an anti-CD19 antibody or the combination of an anti-CD19 antibody and an anti-CD20 antibody. In some embodiments, the targeting domain comprises an anti-CD19 antibody.

In some embodiments, the antibody comprised in the targeting domain is one or more of an anti-CD10 antibody, an anti-CD19 antibody, an anti-CD20 antibody, an anti-CD21 antibody, an anti-CD22 antibody, an anti-CD23 antibody, an anti-CD27 antibody, an anti-CD32b antibody, an anti-CD38 antibody, an anti-CD40 antibody, an anti-CD49d antibody, CD52 antibody, an anti-CD79a antibody, an anti-CD79b antibody, an anti-CD80 antibody, an anti-CD86 antibody, an anti-CD126 antibody, an anti-CD138 antibody, an anti-CD267 antibody, an anti-CD268 antibody, an anti-CD269 antibody, an anti-CD275 antibody, an anti-CD351 antibody, an anti-CD360 antibody and an anti-GPRC5D antibody. In some embodiments, the targeting domain is an anti-CD19 antibody or the combination of an anti-CD19 antibody and an anti-CD20 antibody. In some embodiments, the targeting domain is an anti-CD19 antibody.

In some embodiments, the targeting domain is an antibody that is one or more of an anti-CD10 antibody, an anti-CD19 antibody, an anti-CD20 antibody, an anti-CD21 antibody, an anti-CD22 antibody, an anti-CD23 antibody, an anti-CD27 antibody, an anti-CD32b antibody, an anti-CD38 antibody, an anti-CD40 antibody, an anti-CD49d antibody, CD52 antibody, an anti-CD79a antibody, an anti-CD79b antibody, an anti-CD80 antibody, an anti-CD86 antibody, an anti-CD126 antibody, an anti-CD138 antibody, an anti-CD267 antibody, an anti-CD268 antibody, an anti-CD269 antibody, an anti-CD275 antibody, an anti-CD351 antibody, an anti-CD360 antibody and an anti-GPRC5D antibody. In some embodiments, the targeting domain is one or both of an anti-CD19 antibody and an anti-CD20 antibody.

In some embodiments, the targeting domain is an antibody comprising one of the combinations an anti-CD19 antibody and an anti-CD20 antibody, an anti-CD19 antibody and an anti-CD22 antibody, an anti-CD19 antibody and an anti-CD269 antibody, an anti-CD19 antibody and an anti-CD268 antibody, an anti-CD19 antibody and an anti-CD38 antibody, an anti-CD19 antibody and an anti-CD37 antibody, an anti-CD19 antibody and an anti-CD123 antibody, an anti-CD19 antibody and an anti-CD79b antibody, an anti-CD33 antibody and an anti-CD123 antibody, an anti-CD269 antibody and an anti-CD38 antibody, an anti-CD269 antibody and an anti-GPRC5D antibody, an anti-CD269 antibody and an anti-CD319 antibody, as well as an anti-CD269 antibody and an anti-CD267 antibody.

In some embodiments, the targeting domain is an antibody that is one of the combinations an anti-CD19 antibody and an anti-CD20 antibody, an anti-CD19 antibody and an anti-CD22 antibody, an anti-CD19 antibody and an anti-CD269 antibody, an anti-CD19 antibody and an anti-CD268 antibody, an anti-CD19 antibody and an anti-CD38 antibody, an anti-CD19 antibody and an anti-CD37 antibody, an anti-CD19 antibody and an anti-CD123 antibody, an anti-CD19 antibody and an anti-CD79b antibody, an anti-CD33 antibody and an anti-CD123 antibody, an anti-CD269 antibody and an anti-CD38 antibody, an anti-CD269 antibody and an anti-GPRC5D antibody, an anti-CD269 antibody and an anti-CD319 antibody, as well as an anti-CD269 antibody and an anti-CD267 antibody.

The targeting domain described above is an antibody that is commercially available or can be prepared according to published materials (e.g., patents, journals, or books). For example, in some embodiments, the anti-CD19 antibody is obtained by purchase, e.g. CD19 Monoclonal Antibody (HIB19), eBioscience^{™} (Invitrogen, Cat. No.: 14-0199-82), CD19 Monoclonal Antibody (HIB19), Functional Grade, eBioscience^{™} (Invitrogen, Cat. No.: 16-0199-85), CD19 Monoclonal Antibody (HIB19), FITC, eBioscience^{™} (Invitrogen, Cat. No.: 11-0199-41), Purified anti-human CD19 (BioLegend, Cat. No.: 302202), Purified anti-human CD19 (Maxpar^{®} Ready) (BioLegend, Cat. No.: 30)2247), InVivoMab anti-human CD19 (Bio X Cell, Cat. No.: BE0281-100MG), Anti-CD19 antibody [EPR5906] (Abcam, Cat. No.: ab134114), etc. In addition, other commercially available anti-CD19 antibodies can be obtained by querying the Labome website (https://www.labome.com). For another example, in some embodiments, the anti-CD19 antibody is derived from a patent, such as WO2008031056A2, WO2008022152A2, US20040043401A1, WO2012079000A1, WO2014153270A1, WO2009091826A3, WO2016014565A3, WO2014190273A1, US9260530B2, etc.

In some embodiments, the targeting domain is an antibody that is an anti-CD19 antibody.

In some embodiments, the anti-CD19 antibody comprises a heavy chain variable region comprising HCDR1 comprising SEQ ID NO: 1 (DYGVS), HCDR2 comprising SEQ ID NO: 2 (VIWGSETTYYNSALKS) and HCDR3 comprising SEQ ID NO: 3 (KHYYYGGSYAMDY) and a light chain variable region comprising LCDR1 comprising SEQ ID NO: 4 (RASQDISKYLN), LCDR2 comprising SEQ ID NO: 5 (HTSRLHS) and LCDR3 comprising SEQ ID NO: 6 (GNTLPYT).

In some embodiments, the anti-CD19 antibody comprises a heavy chain variable region comprising HCDR1 as set forth in SEQ ID NO: 1 (DYGVS), HCDR2 as set forth in SEQ ID NO: 2 (VIWGSETTYYNSALKS) and HCDR3 as set forth in SEQ ID NO: 3 (KHYYYGGSYAMDY) and a light chain variable region comprising LCDR1 as set forth in SEQ ID NO: 4 (RASQDISKYLN), LCDR2 as set forth in SEQ ID NO: 5 (HTSRLHS) and LCDR3 as set forth in SEQ ID NO: 6 (GNTLPYT).

In some embodiments, the anti-CD19 antibody comprises a heavy chain variable region and a light chain variable region, wherein the heavy chain variable region (VH) comprises the amino acid sequence set forth in SEQ ID NO: 7 and the light chain variable region (VL) comprises the amino acid sequence set forth in SEQ ID NO: 8. In some embodiments, the anti-CD19 antibody comprises a heavy chain variable region and a light chain variable region, wherein the amino acid sequence of the heavy chain variable region (VH) is set forth in SEQ ID NO: 7, and the amino acid sequence of the light chain variable region (VL) is set forth in SEQ ID NO: 8.
SEQ ID NO: 7: 
EVKLQESGPGLVAPSQSLSVTCTVSGVSLPDYGVSWIRQPPRKGLEWLGVIWGSE
TTYYNSALKSRLTIIKDNSKSQVFLKMNSLQTDDTAIYYCAKHYYYGGSYAMDYWG
QGTSVTVSS;
SEQ **ID** NO: 8:

DIQMTQTTSSLSASLGDRVTISCRASQDISKYLNWYQQKPDGTVKLLIYHTSRLH
SGVPSRFSGSGSGTDYSLTISNLEQEDIATYFCQQGNTLPYTFGGGTKLEIT.

In some embodiments, the anti-CD19 antibody is from the hybridoma clone FMC63.

In some embodiments, the anti-CD19 antibody comprises a heavy chain variable region and a light chain variable region, wherein the heavy chain variable region (VH) comprises the amino acid sequence set forth in SEQ ID NO: 7, the light chain variable region (VL) comprises the amino acid sequence set forth in SEQ ID NO: 8, and the anti-CD19 antibody further comprises a human IgG4 antibody heavy chain constant region (CH) comprising S228P mutation and a human IgG4 antibody light chain constant region (CL).

In an optional specific example, the anti-CD19 antibody is FMC63, which comprises a heavy chain and a light chain, wherein the amino acid sequence of the heavy chain is as set forth in SEQ ID NO: 9, and the amino acid sequence of the light chain is as set forth in SEQ ID NO: 10.
SEQ ID NO: 9 
EVKLQESGPGLVAPSQSLSVTCTVSGVSLPDYGVSWIRQPPRKGLEWLGVIWGSE
TTYYNSALKSRLTIIKDNSKSQVFLKMNSLQTDDTAIYYCAKHYYYGGSYAMDYWG
QGTSVTVSSASTKGPSVFPLAPCSRSTSESTAALGCLVKDYFPEPVTVSWNSGALTSG
VHTFPAVLQSSGLYSLSSVVTVPSSSLGTKTYTCNVDHKPSNTKVDKRVESKYGPPCP
PCPAPPVAGGPSVFLFPPKPKDTLMISRTPEVTCVVVAVSQEDPEVQFNWYVDGVEVH
NAKTKPREEQFNSTYRVVSVLTVVHQDWLNGKEYKCKVSNKGLPSSIEKTISKAKGQ
PREPQVYTLPPSQEEMTKNQVSLTCLVKGFYPSDIAVEWESNGQPENNYKTTPPVLDS
DGSFFLYSKLTVDKSRWQEG NVFSCSVMHEALHNHYTQKSLSLSLGK;
SEQ ID NO: 10

DIQMTQTTSSLSASLGDRVTISCRASQDISKYLNWYQQKPDGTVKLLIYHTSRLH
SGVPSRFSGSGSGTDYSLTISNLEQEDIATYFCQQGNTLPYTFGGGTKLEITRTVAAPSV
FIFPPSDEQLKSGTASVVCLLNNFYPREAKVQWKVDNALQSGNSQESVTEQDSKDST

YSLSSTLTLSKADYEKHKVYACEVTHQGLSSPVTKSFNRGEC.

In some embodiments, the nucleotide sequence encoding the heavy chain of FMC63 is set forth in SEQ ID NO: 11, and the nucleotide sequence encoding the light chain of FMC63 is set forth in SEQ ID NO: 12.
SEQ ID NO: 11 
GAAGTGAAACTGCAGGAGAGCGGACCTGGCCTCGTGGCCCCTAGCCAGAGC
CTGAGCGTGACCTGTACCGTGTCCGGCGTGTCCCTGCCTGATTACGGCGTTTCTTG
GATCCGGCAGCCACCCAGAAAGGGCCTGGAATGGCTGGGCGTCATCTGGGGAAGC
GAGACAACCTACTACAACTCTGCTCTGAAAAGCAGACTGACCATCATCAAGGACA
ATAGCAAGTCCCAGGTGTTCCTGAAGATGAACAGCCTGCAAACAGATGACACCGC
CATCTACTACTGCGCCAAGCACTACTACTATGGCGGCAGCTACGCCATGGACTACT
GGGGCCAGGGCACCAGCGTGACAGTGTCTAGCGCTAGCACCAAGGGCCCCAGCG
TGTTCCCCCTGGCCCCCTGCAGCCGCAGCACCAGCGAGAGCACCGCCGCCCTGGG
CTGCCTGGTGAAGGACTACTTCCCCGAGCCCGTGACCGTGAGCTGGAACAGCGGC
GCCCTGACCAGCGGCGTGCACACCTTCCCCGCCGTGCTGCAGAGCAGCGGCCTGT
ACAGCCTGAGCAGCGTGGTGACCGTGCCCAGCAGCAGCCTGGGCACCAAGACCT
ACACCTGCAACGTGGACCACAAGCCCAGCAACACCAAGGTGGACAAGCGCGTGG
AGAGCAAGTACGGCCCCCCCTGCCCCCCCTGCCCCGCCCCCCCCGTGGCCGGCGG
CCCCAGCGTGTTCCTGTTCCCCCCCAAGCCCAAGGACACCCTGATGATCAGCCGC
ACCCCCGAGGTGACCTGCGTGGTGGTGGCCGTGAGCCAGGAGGACCCCGAGGTG
CAGTTCAACTGGTACGTGGACGGCGTGGAGGTGCACAACGCCAAGACCAAGCCC
CGCGAGGAGCAGTTCAACAGCACCTACCGCGTGGTGAGCGTGCTGACCGTGGTGC
ACCAGGACTGGCTGAACGGCAAGGAGTACAAGTGCAAGGTGAGCAACAAGGGCC
TGCCCAGCAGCATCGAGAAGACCATCAGCAAGGCCAAGGGCCAGCCCCGCGAGC
CCCAGGTGTACACCCTGCCCCCCAGCCAGGAGGAGATGACCAAGAACCAGGTGA
GCCTGACCTGCCTGGTGAAGGGCTTCTACCCCAGCGACATCGCCGTGGAGTGGGA
GAGCAACGGCCAGCCCGAGAACAACTACAAGACCACCCCCCCCGTGCTGGACAG
CGACGGCAGCTTCTTCCTGTACAGCAAGCTGACCGTGGACAAGAGCCGCTGGCAG
GAGGGCAACGTGTTCAGCTGCAGCGTGATGCACGAGGCCCTGCACAACCACTACA
CCCAGAAGAGCCTGAGCCTGAGCCTGGGCAAG;
SEQ ID NO: 12

GATATTCAGATGACACAGACCACCTCCAGCCTCTCTGCTAGCCTGGGCGACAG
AGTGACCATCTCTTGTAGAGCCAGCCAGGACATCAGCAAGTACCTGAACTGGTATC
AGCAGAAACCTGATGGCACAGTGAAGCTGCTGATCTACCACACCAGCCGGCTGCA
CAGCGGCGTGCCCAGCAGATTCAGCGGCAGCGGATCTGGCACCGACTACAGCCTG
ACAATCTCCAACCTGGAGCAGGAGGACATCGCCACCTACTTCTGCCAGCAAGGCA
ATACCCTGCCTTACACCTTTGGCGGAGGCACAAAGCTGGAAATCACCCGTACGGT
GGCCGCACCAAGCGTCTTCATCTTCCCGCCATCTGATGAGCAGTTGAAATCTGGAA
CTGCCTCTGTTGTGTGCCTGCTGAATAACTTCTATCCCAGAGAGGCCAAAGTACAG
TGGAAGGTGGATAACGCCCTCCAATCGGGTAACTCCCAGGAGAGTGTCACAGAGC
AGGACAGCAAGGACAGCACCTACAGCCTCAGCAGCACCCTGACGCTGAGCAAAG
CAGACTACGAGAAACACAAAGTCTACGCCTGCGAAGTCACCCATCAGGGCCTGAG
CTCGCCCGTCACAAAGAGCTTTAACAGAGGCGAGTGC.

In some embodiments, the targeting domain is an antibody that is an anti-CD5 antibody.

In some embodiments, the anti-CD5 antibody is from hybridoma clone 53-7.3(Biolegend, Cat. No. 100602).

In some embodiments, the targeting domain is an antibody that comprises Fc region.

In some embodiments, the targeting domain is an antibody, wherein the Fc region of the antibody is mutated, and the mutation causes the affinity of Fc to Fc receptor (FcR) to decrease or lose.

In some embodiments, the amino acid is represented by EU numbering, and the mutation sites of the Fc region are any one or more of 228, 233, 234, 235, 236, 237, 238, 239, 265, 267, 268, 270, 297, 298, 299, 309, 318, 328, 325, 329, 330, 331 and 409.

In some embodiments, the amino acid is represented by EU numbering, and the mutation of the Fc region is one or more of N297A, N297Q, N297G, L234F, L235E, P331S, L235A, G237A, E318A, L234A, L235A, S228P, L235E, G236R, L328R, S298G, T299A, P329G, D265A, E233P, L234V, G236del, S267K, H268Q, V309L, A330S, V234A, P238S, H268A, F234V, D265T, L309V, R409K, C226S, C229S, E233P, S267E, L328F, L235G, N325A, insertion of amino acid at 233, insertion of amino acid at 234, insertion of amino acid at 235, and insertion of amino acid at 237.

In some embodiments, the mutation of the Fc region is L234F/L235E/P331S of hIgG1, L235A/G237A/E318A of hIgG1, L234A/L235A (LALA) of hIgG1, S228P/L235E of hIgG4, G236R/L328R of hIgG1, S298G/T299A of hIgG1, L234A/L235A/P329G of hIgG1, L234F/L235E/D265AofhIgG1, E233P/L234V/L235A/G236del of hIgG2 and S267K of hIgG1, H268Q/V309L/A330S/P331S of hIgG2 (IgG2m4), V234A/G237A/P238S/ H268A/V309L/A330S/P331S of hIgG2 (IgG2c4d), S228P of hIgG4, S228P/E233P/F234V/L235A of hIgG4, S228P/E233P/F234V/L235A /D265A of hIgG4, S228P/E233P/F234V/L235A/D265T of hIgG4, S228P/E233P/F234V/L235A/ D265A/R409K of hIgG4, S228P/E233P/F234V/L235A/D265A/L309V/R409K of hIgG4, -C226S/C229S of IgG1, C226S/C229S/E233P/L234V/L235A of IgG1, IgG1-S267E/L328F, IgG2-V234A/G237A, L235G/G236R, N325A/L328R and the insertion of amino acid at 233, 234, 235, 237. It should be noted that "/" in "L234F/L235E/P331S" means "and", and other mutations are the same.

In some embodiments, the antibody comprised in the targeting domain comprises an antibody without Fc. In some embodiments, the antibody without Fc is selected from any one of Fab fragment, Fab' fragment, F(ab')₂ fragment, single-chain antibody (scFv), disulfide-stabilized antibody (dsFv), disulfide-stabilized single-chain antibody (ds-ScFv), diabody and single-domain antibody (sdAb).

In some embodiments, the targeting domain comprises nucleic acid and small molecule, nucleic acid and antibody, polypeptide and small molecule, or small molecule and antibody. In an optional specific example, the targeting domain is one or more of nucleic acid, polypeptide, antibody and small molecule.

### Coupling

In some embodiments, the targeting domain is coupled to the delivery carrier in a manner of covalent or non-covalent coupling. In some embodiments, the targeting domain is covalently coupled or non-covalently coupled to the polymer-lipid of the delivery carrier. It can be understood that in other embodiments, it is not limited that the targeting domain is coupled to the polymer-lipid of the delivery carrier, and can also be coupled to other components of the delivery carrier, such as helper lipid or structural lipid.

In some embodiments, the targeting domain has a first coupling group and the polymer-lipid has a second coupling group. The second coupling group can react with the first coupling group to form a covalent bond such that the targeting domain is covalently coupled to the polymer-lipid, or the second coupling group and the first coupling group can interact such that the targeting domain is non-covalently coupled to the polymer lipid. In some embodiments, the second coupling group is coupled to the lipid and/or polymer of the polymer-lipid. In an optional specific example, the polymer-lipid is polyethylene glycol-lipid, and the second coupling group is coupled to the lipid and/or polyethylene glycol of the polyethylene glycol-lipid.

The first coupling group can be coupled to the targeting domain using any method known to those skilled in the art. In some embodiments, the first coupling group (e.g., sulfhydryl) is coupled to the targeting domain via a chemical reaction, e.g. a reaction between free amino group and 2-iminothiane or N-succinimidyl S-acetylthioacetate (SATA). Likewise, the second coupling group may be coupled to the polymer or lipid in the polymer-lipid using any method known to those skilled in the art.

The first coupling group and the second coupling group may be any functional group capable of co-forming a covalent bond. For example, the first coupling group or the second coupling group is one or more derived from or selected from maleimides, N-hydroxysuccinimide (NHS) esters, carbodiimides, hydrazide, pentafluorophenyl (PFP) esters, phosphines, hydroxymethyl phosphines, psoralen, imidoesters, pyridyl disulfide, isocyanates, vinyl sulfones, alpha-haloacetyls, aryl azides, acyl azides, alkyl azides, diazirines, benzophenone, epoxides, carbonates, anhydrides, sulfonyl chlorides, cyclooctyne, aldehydes and sulfhydryl. In some embodiments, the first coupling group or the second coupling group is one or more derived from or selected from free amino (-NH₂), free sulfhydryl (-SH), free hydroxyl (-OH), carboxylates, hydrazides, and alkoxyamines.

In some embodiments, the targeting domain is chemically coupled to the polymer lipid by "click". Bioorthogonal "click" chemistry includes a reaction between a 1,3-dipole species (e.g., azide, nitrile oxide, nitrogen, isonitrile) and an olefin or alkyne dipolarophile. 1,3-Dipole is a three-atom formed 4π electron-chemical. Common 1,3-dipoles include azides, nitrile oxide, nitrone, isocyanides, diazo alkanes, and the like. In some embodiments, one of the targeting domain and the polymer-lipid comprises a 1,3-dipole and the other comprises an alkenyl or alkynyl group. Exemplary dipolarophiles include any strained cycloalkenes and cycloalkynes known to those skilled in the art, including but not limited to cyclooctyne, dibenzocyclooctyne, monofluorocyclooctyne, difluorocyclooctyne, and biaryl azacyclooctanone.

In some embodiments, the first coupling group and the second coupling group are independently selected from, or derived from, one or more of sulfhydryl, azido, amino, amido, a polypeptide whose amino acid sequence is LPXTG (X is any amino acid), carbonyl, avidin, a sulfhydryl-reactive functional group (i.e., a functional group capable of reacting with sulfhydryl), alkynyl, alkenyl, carboxyl, polyglycine, aminooxy (-O-NH₂), and biotin. In some embodiments, one of the first coupling group and the second coupling group is selected from or derived from one or more of sulfhydryl, azido, amino, amido, a polypeptide whose amino acid sequence is LPXTG, carbonyl and avidin, and the other of the first coupling group and the second coupling group is selected from or derived from one or more of a sulfhydryl reactive functional groups, alkynyl, alkenyl, carboxyl, amino, polyglycine, aminooxy (-O-NH₂), and biotin.

In some embodiments, the sulfhydryl-reactive functional group is selected from or derived from one or more of alkenyl, alkynyl, halogen substituent, an electron-withdrawing group, sulfonyl, and a functional group (e.g., 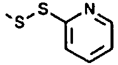 ) that can undergo a thiol-disulfide exchange reaction.

In some embodiments, the alkenyl is a linear alkenyl and/or a cycloalkenyl. In some embodiments, the alkenyl is derived from or selected from one or more of olefins, maleimides, divinylpyrimidines, 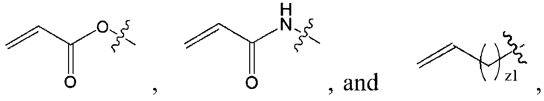 where z1 is 0 or any positive integer of 1-15. In some embodiments, the alkenyl is cycloalkenyl.

In some embodiments, the alkynyl is a linear alkynyl and/or a cycloalkynyl. In some embodiments, the alkynyl is a linear alkynyl. In some embodiments, the linear alkynyl is derived from or selected from one or more of linear alkynes, 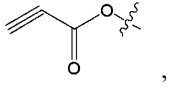 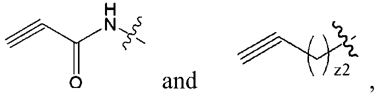 wherein z2 is 0 or any positive integer of 1-15. In an optional specific example, the linear alkynyl is

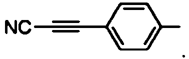

In other embodiments, the alkynyl is a cycloalkynyl. In some embodiments, the cycloalkynyl is cyclooctyne. In some embodiments, cyclooctynyl is derived from cyclooctyne (e.g., OCT), cyclooctyne without aromatic ring (e.g., ALO), cyclopropanecyclooctyne (BCN), dibenzocyclooctyne (DBCO), diphenylcyclooctyne (DIBO), monofluorinated cyclooctyne, difluorinated cyclooctyne (DIFO), or diaryl azacyclooctanone (BARAC).

In some embodiments, the halogen substituent is selected from one or more of -Br, -Cl, - I, haloaryl (e.g., halophenyl), halohydrocarbyl, acyl halide, dihalomaleimide (e.g., dibromomaleimide), and dihalopyridazinedione (e.g., dibromopyridazinedione). In some embodiments, the haloaryl is preferably chloroaryl, bromoaryl or iodoaryl. Halohydrocarbyl is preferably chlorohydrocarbyl, bromohydrocarbyl or iodohydrocarbyl. The acyl halide is preferably acyl chloride, acyl bromide or acyl iodide.

In some embodiments, the electron-withdrawing group is selected from one or more of: 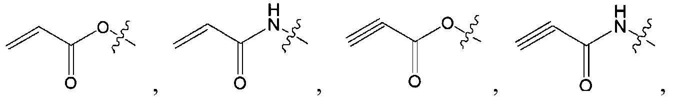 acyl halide, aldehyde and carbonyl.

In some embodiments, the avidin is streptavidin.

In some embodiments, the targeting domain is coupled to the delivery carrier in a manner of covalent coupling.

In some embodiments, one of the first coupling group and the second coupling group is sulfhydryl, and the other is a sulfhydryl reactive functional group. In this case, one of the targeting domain and the polymer-lipid comprises sulfhydryl and the other comprises a sulfhydryl-reactive functional group. The targeting domain and the polymer-lipid form a covalent bond between the sulfhydryl and the sulfhydryl-reactive functional group such that the targeting domain is coupled to the delivery carrier. In some embodiments, the targeting domain is covalently coupled to the polymer-lipid by a thiol-alkene click reaction, a thiol-alkyne click reaction, an interchain disulfide bond, a nucleophilic substitution reaction of sulfhydryl with a halo group, or a thiol-disulfide exchange reaction.

In some embodiments, the first coupling group is sulfhydryl and the second coupling group is a sulfhydryl reactive functional group.

In some embodiments, the first coupling group is sulfhydryl and the second coupling group is selected from or derived from one or more of alkenyl, alkynyl, halogen substituents, electron-withdrawing groups, sulfonyl, and functional groups (e.g., 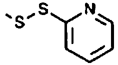 ) that can undergo a thiol-disulfide exchange reaction.

In some embodiments, the alkenyl is a linear alkenyl and/or a cycloalkenyl. In some embodiments, the alkenyl is derived from or selected from one or more of olefins, maleimides, divinylpyrimidines, 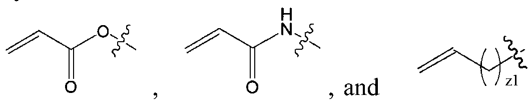 , where z1 is 0 or any positive integer of 1-15. In some embodiments, the alkenyl is cycloalkenyl.

In some embodiments, the targeting domain is coupled to the delivery carrier in a manner of covalent coupling, wherein the first coupling group is sulfhydryl and the second coupling group is alkenyl.

In some embodiments, the targeting domain is coupled to the delivery carrier in a manner of covalent coupling, wherein the first coupling group is sulfhydryl which is introduced by reacting SATA with an antibody, and the second coupling group is alkenyl which is derived from a maleimide functional group in DSPE-PEG-Mal.

In some embodiments, the alkynyl is a linear alkynyl and/or a cycloalkynyl. In some embodiments, the alkynyl is a linear alkynyl. In some embodiments, the linear alkynyl is derived from or selected from one or more of linear alkynes, 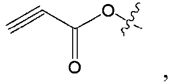 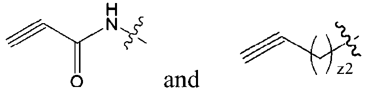 , wherein z2 is 0 or any positive integer of 1-15. In an optional specific example, the linear alkynyl is

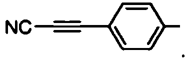

In other embodiments, the alkynyl is cycloalkynyl. In some embodiments, the cycloalkynyl is cyclooctyne. In some embodiments, cyclooctynyl is derived from cyclooctyne (e.g., OCT), cyclooctyne without aromatic ring (e.g., ALO), cyclopropanecyclooctyne (BCN), dibenzocyclooctyne (DBCO), diphenylcyclooctyne (DIBO), monofluorinated cyclooctyne, difluorinated cyclooctyne (DIFO), or diaryl azacyclooctanone (BARAC).

In some embodiments, the halogen substituent is selected from one or more of -Br, -Cl, - I, haloaryl (e.g., halophenyl), halohydrocarbyl, acyl halide, dihalomaleimide (e.g., dibromomaleimide), and dihalopyridazinedione (e.g., dibromopyridazinedione). In some embodiments, the haloaryl is preferably chloroaryl, bromoaryl or iodoaryl. Halohydrocarbyl is preferably chlorohydrocarbyl, bromohydrocarbyl or iodohydrocarbyl. The acyl halide is preferably acyl chloride, acyl bromide or acyl iodide.

In some embodiments, the electron-withdrawing group is selected from one or more of: 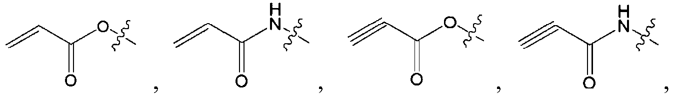 acyl halide, aldehyde and carbonyl.

In some embodiments, the targeting domain comprises an antibody having a free sulfhydryl group. In an optional specific example, the targeting domain is an antibody having a free sulfhydryl group. It can be understood that in other embodiments, the targeting domain is not limited to an antibody having a free sulfhydryl group, and may also be other substances having a free sulfhydryl group.

In addition, the free sulfhydryl group of the targeting domain may be a free sulfhydryl group of a natural amino acid (such as cysteine), or may be a free sulfhydryl group introduced by N-succinimidyl S-acetylthioacetate (SATA, or "N-succinimidyl-S-acetylthioacetate"), or a free sulfhydryl group formed by reduction of a disulfide bond between natural amino acids (cysteine) (such as a free sulfhydryl group is introduced with IdeS protease or DTT reduction of a disulfide bond after digestion with IdeZ protease, or DTT direct reduction of a disulfide bond), and an additional cysteine may also be introduced to generate a free sulfhydryl group (for example, cysteine is inserted at the C-terminus of antibody by genetic engineering technology).

In addition, it can be understood that in other embodiments, it can also be that the first coupling group is a sulfhydryl-reactive functional group and the second coupling group is sulfhydryl. In this case, the targeting domain comprises a sulfhydryl-reactive functional group, and the polymer-lipid comprises sulfhydryl.

In some embodiments, one of the first coupling group and the second coupling group is azido, and the other is alkynyl or alkenyl. In this case, one of the targeting domain and the polymer-lipid comprises azido, and the other comprises alkynyl or alkenyl. The targeting domain interacts and the polymer-lipid undergo click reaction through azido with alkynyl or alkenyl such that the targeting domain is covalently coupled to the delivery carrier.

In some embodiments, the first coupling group is azido, and the second coupling group is alkynyl or alkenyl. In some embodiments, the second coupling group is linear alkynyl or cycloalkynyl. In this case, the azido of the targeting domain and the alkynyl of the polymer-lipid undergo copper-catalyzed click chemistry (CuAAC) or copper-free catalytic click chemistry (SPAAC) to cyclize to form a covalent bond, so that the targeting domain is covalently coupled to the polymer-lipid. In an optional specific example, the second coupling group is cycloalkynyl. In some embodiments, cyclooctynyl is derived from cyclooctyne (e.g., OCT), cyclooctyne without aromatic ring (e.g., ALO), cyclopropanecyclooctyne (BCN), dibenzocyclooctyne (DBCO), diphenylcyclooctyne (DIBO), monofluorinated cyclooctyne, difluorinated cyclooctyne (DIFO), or diaryl azacyclooctanone (BARAC).

In some embodiments, the targeting domain is an azido containing antibody. It can be understood that the targeting domain is not limited to an antibody comprising an azido group, and may also be another substance containing an azido group, such as a polypeptide specifically targeting a surface antigen of an immune cell.

The azido in the targeting domain can be obtained by introducing (e.g., by genetic engineering techniques) a non-natural amino acid having an azido group (e.g., azidomethyl-L-phenylalanine (pAMF) or azidolysine), or by reacting the targeting domain with an azide compound. In some embodiments, the azide compound that reacts with the targeting domain is selected from one or more of NHS-azide, NHS-polyethylene glycol-azide, amino-polyethylene glycol-azide. In an optional specific example, the NHS-azide is 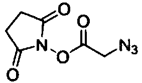 ; the NHS-polyethylene glycol-azide is 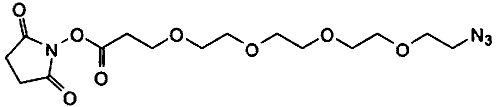 ; the amino-polyethylene glycol-azide is

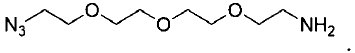

In addition, when the targeting domain is an antibody having a glutamine, azido groups in the targeting domain may also be introduced by the action of Microbial transglutaminase (MTGase). MTGase mainly catalyzes acyl transfer reactions between the amido in glutamine in protein or polypeptide (e.g., Q295 of antibody) and amino (e.g., primary amino group), participates in cross-linking between proteins and proteins or small molecules to form isopeptide bonds. For example, the targeting domain is an antibody having a glutamine, the glycan chain connected to the glutamine in the targeting domain is removed under the action of peptide N-glycosidase F (PNGase F), then an azide containing amino is added, so azido is introduced under the action of MTGase.

In addition, azido in the targeting domain may also be introduced under the action of β-galactosidase and β-1,4-galactosyltransferase.

In addition, it can be understood that in other embodiments, it can also be that the first coupling group is alkynyl or alkenyl, and the second coupling group is azido. In this case, the targeting domain comprises alkynyl or alkenyl, and the polymer-lipid comprises azido.

In some embodiments, one of the first coupling group and the second coupling group is amino, and the other is an amino reactive functional group (i.e., a functional group capable of reacting with amino). In some embodiments, the amino reactive functional group is carboxyl, alkenyl, alkynyl, a halogen substituent, an electron-withdrawing group, sulfonyl and a functional group (e.g., 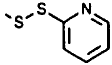 ) that can undergo a thiol-disulfide exchange reaction.

In some embodiments, the second coupling group is derived from a substance having one or more groups of

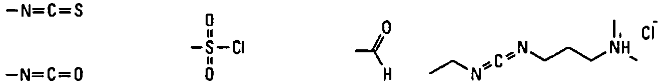

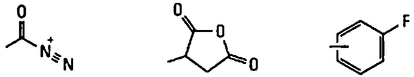

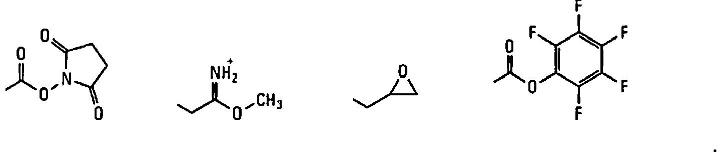

In some embodiments, the second coupling group is derived from one or more of isothiocyanate, isocyanate, sulfonyl chloride, aldehyde, carbodiimide, acyl azide, anhydride, fluorobenzene, carbonate, NHS ester, imidoester, epoxide and Fluorophenyl ester.

In this case, one of the targeting domain and the polymer-lipid comprises an amino group and the other comprises an amino reactive functional group. The targeting domain reacts with the polymer-lipid through the amino group and the amino-reactive functional group to produce a covalent bond such that the targeting domain is covalently coupled to the delivery carrier.

In some embodiments, one of the first coupling group and the second coupling group is amino, and the other is carboxyl. In this case, one of the targeting domain and the polymer-lipid comprises amino, and the other comprises carboxyl or activated carboxyl. The targeting domain and the polymer-lipid form an amide bond by reaction of the amino with the carboxyl or activated carboxyl such that the targeting domain is covalently coupled to the delivery carrier. In some embodiments, one of the first coupling group and the second coupling group is the free amino of lysine, and the other is activated carboxyl. In some embodiments, the first coupling group is the free amino of lysine, and the second coupling group is activated carboxyl. In this case, the targeting domain comprises lysine with a free amino and the polymer-lipid comprises activated carboxyl (for example, a carboxylate obtained by activating carboxyl using STP (4-Sulfo-2,3,5,6-tetrafluorophenyl), a carboxylate obtained by activating carboxyl with 1-ethyl-(3-dimethylaminopropyl) carbodiimide (EDC), or a carboxylate obtained by activating carboxyl by complexing EDC with N-hydroxysulfosuccinimide (Sulfo-NHS)).

In some embodiments, the targeting domain comprises an antibody having a lysine having a free amino, and the polymer-lipid comprises activated carboxyl (e.g., carboxylate). In an optional specific example, the targeting domain is an antibody comprising a lysine with a free amino, and the polymer-lipid comprises activated carboxyl. It can be understood that in other embodiments, the targeting domain is not limited to an antibody comprising a lysine having a free amino, and can be other substances comprising a lysine having a free amino or other substances comprising a free amino, such as a polypeptide specifically targeting a surface antigen of an immune cell.

In other embodiments, the first coupling group is an amino-reactive functional group and the second coupling group is amino. In this case, the targeting domain comprises an amino reactive functional group, and the polymer-lipid comprises amino. The targeting domain and the polymer-lipid form a covalent bond through the amino of the polymer-lipid with the amino reactive functional group of the targeting domain such that the targeting domain is coupled to the delivery carrier.

The carboxyl activation of the targeting domain may be performed in a manner known in the art (e.g., using STP, NHS, EDC, or the coordination of EDC with Sulfo-NHS). In some embodiments, the targeting domain comprises an antibody having a carboxyl group or an activated carboxyl group, and the polymer-lipid comprises amino. It can be understood that in other embodiments, the targeting domain is not limited to comprise an antibody having carboxyl or activated carboxyl, and may also be other substances having carboxyl or activated carboxyl, such as a polypeptide specifically targeting a surface antigen of an immune cell.

In some embodiments, one of the first coupling group and the second coupling group is carbonyl and the other is aminooxy (-O-NH₂). In some embodiments, the carbonyl is aldehyde or ketone. In this case, one of the targeting domain and the polymer-lipid comprises carbonyl and the other comprises aminooxy (-O-NH₂). The targeting domain is covalently coupled to the polymer-lipid through the carbonyl to form an oxime bond with aminooxy (-O-NH₂).

In some embodiments, the targeting domain comprises an amino acid comprising carbonyl. In an optional specific example, the amino acid comprising carbonyl includes acetylphenylalanine (pAF). The carbonyl containing amino acid of the targeting domain can be obtained by introducing a non-natural amino acid (e.g., acetylphenylalanine (pAF)).

In some embodiments, the targeting domain comprises an antibody comprising carbonyl. The carbonyl-containing antibody can be obtained by introducing a non-natural amino acid containing carbonyl through genetic engineering technology. When the targeting domain is an antibody containing an aldehyde group and having Fc fragment, the antibody containing an aldehyde group can also be obtained by introducing sialic acid (SA) at the end of the N-glycan and oxidizing SA through β-1,4-galactosyltransferase (GalT) and α-2,6-sialyltransferase (SialT). It can be understood that the targeting domain is not limited to an antibody comprising carbonyl, and may also be other carbonyl-containing substances, such as a polypeptide specifically targeting a surface antigen of an immune cell. In addition, the targeting domain is not limited to an amino acid containing carbonyl, and may also be other substances containing carbonyl and capable of reacting with aminooxy (-O-NH₂) to form an oxime.

In addition, it can be understood that in other embodiments, it can also be that the first coupling group is aminooxy (-O-NH₂), the second coupling group is carbonyl, and in this case, the targeting domain comprises aminooxy (-O-NH₂), and the polymer-lipid comprises carbonyl.

In some embodiments, one of the first coupling group and the second coupling group is amido, and the other is amino. In this case, one of the targeting domain and the polymer-lipid comprises amido and the other comprises amino. The targeting domain and the polymer-lipid can form a covalent bond through MTGase such that the targeting domain is covalently coupled to the polymer-lipid, such that the targeting domain is covalently coupled to the delivery carrier. In some embodiments, the first coupling group is amido, and the second coupling group is amino.

In some embodiments, one of the first coupling group and the second coupling group is the amido group of glutamine, and the other is amino (e.g., primary amino). In this case, one of the targeting domain and the polymer-lipid comprises glutamine and the other comprises amino.

In some embodiments, the first coupling group is the amido group of glutamine, and the second coupling group is amino (e.g., primary amino). In this case, the targeting domain comprises glutamine, and the polymer-lipid comprises amino. In some embodiments, the targeting domain is an antibody having a glutamine, and the polymer-lipid comprises amino. In some embodiments, the targeting domain is an antibody that is an Fc fragment having a glutamine. In this case, after the glycan chain of the Fc fragment of the targeting domain is removed by using PNGase F, the polymer-lipid is covalently coupled to the targeting domain under the action of MTGase. It can be understood that the targeting domain is not limited to an antibody having a glutamine, and may also be another substance comprising glutamine, such as a polypeptide specifically targeting a surface antigen of an immune cell.

In addition, in other embodiments, the first coupling group may be amino, and the second coupling group may be the amido group of glutamine. In this case, the targeting domain comprises amino, and the polymer-lipid comprises glutamine.

In some embodiments, one of the first coupling group and the second coupling group is a polypeptide whose amino acid sequence is LPXTG (X is any amino acid), and the other is polyglycine. In this case, one of the targeting domain and the polymer-lipid comprises a polypeptide whose amino acid sequence is LPXTG, and the other comprises polyglycine. The targeting domain forms a covalent bond with the polymer-lipid through a sortase such that the targeting domain is covalently coupled to the polymer-lipid. Sortase is an enzyme isolated from Staphylococcus aureus and can be used for transpeptide-mediated coupling. The sortase can specifically recognize the LPXTG sequence in protein, specifically cleave the threonine-glycine (T-G) binding site, form an acyl-substrate complex, and the polyglycine-bearing nucleophilic group of the polymer-lipid attacks the complex so that the targeting domain is covalently coupled to the polymer-lipid. For example, the sortase is sortase A (Srt A). Sortase A can specifically recognize LPETG, lyse peptide bonds between threonine and glycine to form an enzyme-substrate complex, then the polyglycine-bearing polymer-lipid reacts with the complex, eventually covalently coupling the targeting domain to the polymer-lipid.

In some embodiments, the first coupling group is a polypeptide whose amino acid sequence is LPXTG (X is any amino acid), and the second coupling group is polyglycine. In some embodiments, the polyglycine is tetrameric glycine (GGGG), pentameric glycine (GGGGG), hexameric glycine (GGGGGG), or heptameric glycine (GGGGGGG).

In some embodiments, the targeting domain is an antibody comprising LPXTG. It can be understood that the targeting domain is not limited to an antibody comprising LPXTG, and may also be other substances comprising LPXTG.

In addition, in other embodiments, the first coupling group may be polyglycine, and the second coupling group may be a polypeptide whose amino acid sequence is LPXTG. In this case, the targeting domain comprises polyglycine, and the polymer-lipid comprises a polypeptide whose amino acid sequence is LPXTG.

In other embodiments, the targeting domain is coupled to the delivery carrier in a manner of non-covalent coupling. In some embodiments, non-covalent coupling includes one or more of electrostatic interaction, van der Waals force, ring stacking, and hydrophobic interaction.

In some embodiments, one of the first coupling group and the second coupling group is avidin, and the other is biotin. In this case, one of the targeting domain and the polymer-lipid comprises avidin and the other comprises biotin. The targeting domain and the polymer-lipid of the delivery carrier couple the targeting domain to the delivery carrier by interaction of biotin with avidin. In some embodiments, the avidin is streptavidin.

In some embodiments, the first coupling group is avidin and the second coupling group is biotin. In this case, the targeting domain comprises avidin and the polymer-lipid comprises biotin. In some embodiments, the first coupling group is streptavidin and the second coupling group is biotin. In this case, the targeting domain comprises an antibody conjugated with streptavidin, and the polymer-lipid is a polymer-lipid conjugated with biotin. In an optional specific example, the targeting domain is an antibody conjugated with streptavidin, and the polymer-lipid is a polymer-lipid conjugated with biotin.

In some embodiments, the streptavidin-conjugated antibody is obtained by coupling streptavidin to an antibody containing a free sulfhydryl group by SMCC. In some embodiments, a biotin-coupled polymer-lipid is obtained by reacting activated biotin (e.g., maleimide-activated biotin) with an amino-containing polymer-lipid to form an amide bond. It may be understood that the method for coupling streptavidin to antibody and the solution for coupling biotin to the polymer-lipid are not limited thereto, and may also be other methods.

It can be understood that in other embodiments, the targeting domain is not limited to an antibody conjugated with streptavidin, and may also be other substances coupled with streptavidin, such as a polypeptide or a nucleic acid that specifically targets a surface antigen of an immune cell. In addition, in other embodiments, it may also be that the first coupling group is biotin, and the second coupling group is avidin or streptavidin. In this case, the targeting domain comprises biotin, and the polymer-lipid comprises avidin or streptavidin.

In some embodiments, the polymer-lipid is selected from one or more of: 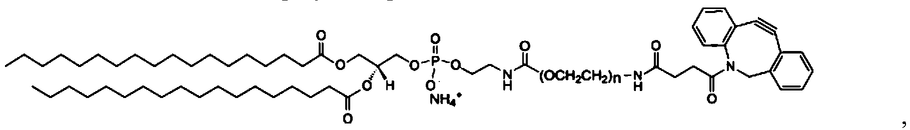 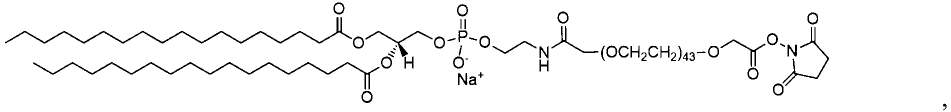 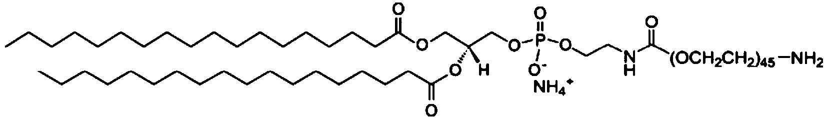 and

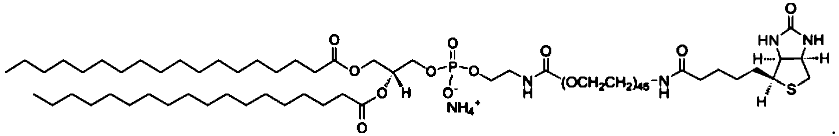

In some embodiments, the coupling of the targeting domain to the delivery carrier is a reversible coupling such that the delivery carrier may be separated from the targeting domain upon exposure to certain conditions or chemical reagents. In other embodiments, the coupling of the targeting domain to the delivery carrier is an irreversible coupling such that the delivery carrier is not separated from the targeting domain under normal conditions.

In some embodiments, the delivery carrier is a lipid nanoparticle. In some embodiments, the lipid nanoparticle comprises a PEG-lipid, and the targeting domain is coupled to a PEG-lipid.

In some embodiments, the PEG-lipid is a modified PEG-lipid.

In some embodiments, the PEG-lipid is a maleimide modified PEG-lipid.

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD19 antibody, and the delivery carrier is a lipid nanoparticle comprising PEG-lipid which is a modified PEG-lipid, wherein the anti-CD19 antibody has a first coupling group which is sulfhydryl, and the modified PEG-lipid has a second coupling group which is alkenyl, and the anti-CD19 antibody and the lipid nanoparticle are covalently coupled by the first coupling group and the second coupling group, wherein the anti-CD19 antibody comprises a heavy chain variable region comprising HCDR1 whose amino acid sequence is set forth in SEQ ID NO: 1, HCDR2 whose amino acid sequence is set forth in SEQ ID NO: 2 and HCDR3 whose amino acid sequence is set forth in SEQ ID NO: 3, and a light chain variable region comprising LCDR1 whose amino acid sequence is set forth in SEQ ID NO: 4, LCDR2 whose amino acid sequence is set forth in SEQ ID NO: 5 and LCDR3 whose amino acid sequence is set forth in SEQ ID NO: 6.

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD19 antibody, and the delivery carrier is a lipid nanoparticle comprising a maleimide (Mal) modified PEG-lipid, wherein the anti-CD19 antibody has a first coupling group which is sulfhydryl introduced by the reaction of SATA with the anti-CD19 antibody, and the maleimide (Mal) modified PEG-lipid has a second coupling group which is alkenyl derived from a maleimide functional group in DSPE-PEG-Mal, wherein the anti-CD19 antibody comprises a heavy chain variable region comprising HCDR1 whose amino acid sequence is set forth in SEQ ID NO: 1, HCDR2 whose amino acid sequence is set forth in SEQ ID NO: 2 and HCDR3 whose amino acid sequence is set forth in SEQ ID NO: 3, and a light chain variable region comprising LCDR1 whose amino acid sequence is set forth in SEQ ID NO: 4, LCDR2 whose amino acid sequence is set forth in SEQ ID NO: 5 and LCDR3 whose amino acid sequence is set forth in SEQ ID NO: 6.

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD19 antibody, and the delivery carrier is a lipid nanoparticle comprising DSPE-PEG-Mal, wherein the anti-CD19 antibody has a first coupling group which is sulfhydryl introduced by the reaction of SATA with the anti-CD19 antibody, and DSPE-PEG-Mal in the lipid nanoparticle has a second coupling group which is alkenyl derived from a maleimide functional group in DSPE-PEG-Mal, wherein the anti-CD19 antibody and the lipid nanoparticle are covalently coupled by the first coupling group and the second coupling group, wherein the anti-CD19 antibody comprises a heavy chain variable region comprising HCDR1 whose amino acid sequence is set forth in SEQ ID NO: 1, HCDR2 whose amino acid sequence is set forth in SEQ ID NO: 2 and HCDR3 whose amino acid sequence is set forth in SEQ ID NO: 3, and a light chain variable region comprising LCDR1 whose amino acid sequence is set forth in SEQ ID NO: 4, LCDR2 whose amino acid sequence is set forth in SEQ ID NO: 5 and LCDR3 whose amino acid sequence is set forth in SEQ ID NO: 6.

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD19 antibody, and the delivery carrier is a lipid nanoparticle comprising DSPE-PEG-Mal, a cationic lipid, a helper lipid and a structural lipid, wherein the anti-CD19 antibody has a first coupling group which is sulfhydryl introduced by the reaction of SATA with the anti-CD19 antibody, and DSPE-PEG-Mal in the lipid nanoparticle has a second coupling group which is alkenyl derived from a maleimide functional group in DSPE-PEG-Mal, wherein the anti-CD19 antibody and the lipid nanoparticle are covalently coupled by the first coupling group and the second coupling group, wherein the anti-CD19 antibody comprises a heavy chain variable region comprising HCDR1 whose amino acid sequence is set forth in SEQ ID NO: 1, HCDR2 whose amino acid sequence is set forth in SEQ ID NO: 2 and HCDR3 whose amino acid sequence is set forth in SEQ ID NO: 3, and a light chain variable region comprising LCDR1 whose amino acid sequence is set forth in SEQ ID NO: 4, LCDR2 whose amino acid sequence is set forth in SEQ ID NO: 5 and LCDR3 whose amino acid sequence is set forth in SEQ ID NO: 6, the cationic lipid is ALC-0315, the helper lipid is DSPC, and the structural lipid is cholesterol.

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD19 antibody and the delivery carrier is a lipid nanoparticle comprising DSPE-PEG-Mal, a cationic lipid, a helper lipid and a structural lipid, wherein the anti-CD19 antibody has a first coupling group which is sulfhydryl introduced by the reaction of SATA with the anti-CD19 antibody, and DSPE-PEG-Mal in the lipid nanoparticle has a second coupling group which is alkenyl derived from a maleimide functional group in DSPE-PEG-Mal, wherein the anti-CD19 antibody and the lipid nanoparticle are covalently coupled by the first coupling group and the second coupling group, wherein the anti-CD19 antibody comprises a heavy chain variable region comprising HCDR1 whose amino acid sequence is set forth in SEQ ID NO: 1, HCDR2 whose amino acid sequence is set forth in SEQ ID NO: 2 and HCDR3 whose amino acid sequence is set forth in SEQ ID NO: 3, and a light chain variable region comprising LCDR1 whose amino acid sequence is set forth in SEQ ID NO: 4, LCDR2 whose amino acid sequence is set forth in SEQ ID NO: 5 and LCDR3 whose amino acid sequence is set forth in SEQ ID NO: 6, the cationic lipid is ALC-0315, the helper lipid is DSPC, the structural lipid is cholesterol, and based on the total amount of the cationic lipid, the helper lipid, the structural lipid and the DSPE-PEG-Mal, the molar ratio of the cationic lipid, the helper lipid, the structural lipid and the DSPE-PEG-Mal is (25-75): (5-45): (0-50): (0.5-5).

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD5 antibody, and the delivery carrier is a lipid nanoparticle comprising a PEG-lipid which is a modified PEG-lipid, wherein the anti-CD5 antibody has a first coupling group which is sulfhydryl and the modified PEG-lipid has a second coupling group which is alkenyl, wherein the anti-CD5 antibody and the lipid nanoparticle are covalently coupled by the first coupling group and the second coupling group, and the anti-CD5 antibody is from hybridoma clone 53-7.3 (e.g., Biolegend, Cat. No. 100602).

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD5 antibody, and the delivery carrier is a lipid nanoparticle comprising a maleimide (Mal) modified PEG-lipid, wherein the anti-CD5 antibody has a first coupling group which is sulfhydryl introduced by the reaction of SATA with the anti-CD5 antibody, and the maleimide (Mal) modified PEG-lipid has a second coupling group which is alkenyl derived from a maleimide functional group in DSPE-PEG-Mal, wherein the anti-CD5 antibody is from hybridoma clone 53-7.3 (e.g., Biolegend, Cat. No. 100602).

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD5 antibody, and the delivery carrier is a lipid nanoparticle comprising DSPE-PEG-Mal, wherein the anti-CD5 antibody has a first coupling group which is sulfhydryl introduced by the reaction of SATA with the anti-CD5 antibody, and DSPE-PEG-Mal in the lipid nanoparticle has a second coupling group which is alkenyl derived from a maleimide functional group in DSPE-PEG-Mal, wherein the anti-CD5 antibody and the lipid nanoparticle are covalently coupled by the first coupling group and the second coupling group, and the anti-CD5 antibody is from hybridoma clone 53-7.3 (e.g., Biolegend, Cat. No. 100602).

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD5 antibody and the delivery carrier is a lipid nanoparticle comprising DSPE-PEG-Mal, a cationic lipid, a helper lipid and a structural lipid, wherein the anti-CD5 antibody has a first coupling group which is sulfhydryl introduced by reaction of SATA with the anti-CD5 antibody, and DSPE-PEG-Mal in the lipid nanoparticle has a second coupling group which is alkenyl derived from a maleimide functional group in DSPE-PEG-Mal, wherein the anti-CD5 antibody and the lipid nanoparticle are covalently coupled by the first coupling group and the second coupling group, the anti-CD5 antibody is from hybridoma clone 53-7.3 (e.g., Biolegend, Cat. No. 100602), the cationic lipid is ALC-0315, the helper lipid is DSPC, and the structural lipid is cholesterol.

In some embodiments, the present invention provides a modified delivery carrier comprising a delivery carrier for loading an agent and a targeting domain for specifically targeting a surface antigen of an immune cell, wherein the targeting domain is covalently coupled to the delivery carrier, wherein the targeting domain is an anti-CD5 antibody, and the delivery carrier is a lipid nanoparticle comprising DSPE-PEG-Mal, a cationic lipid, a helper lipid and a structural lipid, wherein the anti-CD5 antibody has a first coupling group which is sulfhydryl introduced by the reaction of SATA with the anti-CD5 antibody, and DSPE-PEG-Mal in the lipid nanoparticle has a second coupling group which is alkenyl derived from a maleimide functional group in DSPE-PEG-Mal, wherein the anti-CD5 antibody and the lipid nanoparticle are covalently coupled by the first coupling group and the second coupling group, the anti-CD5 antibody is from hybridoma clone 53-7.3 (e.g., Biolegend, Cat. No. 100602), the cationic lipid is ALC-0315, the helper lipid is DSPC, the structural lipid is cholesterol, and based on the total amount of the cationic lipid, the helper lipid, the structural lipid and the DSPE-PEG-Mal, the molar ratio of the cationic lipid, the helper lipid, the structural lipid and the DSPE-PEG-Mal is (25-75): (5-45): (0-50): (0.5-5).

In some embodiments, the delivery carrier is loaded with an agent. In some embodiments, the agent is encapsulated within the delivery carrier. For example, a hydrophilic agent is wrapped in a cavity formed by lipid bilayer, and a hydrophobic agent, for example, is embedded between lipid bilayer. In some alternative specific examples, the agent is fully encapsulated within the delivery carrier. In other alternative embodiments, a portion of the agent is encapsulated within the delivery carrier and a portion is exposed outside the delivery carrier.

In some embodiments, the agent loaded in the delivery carrier includes one or more of a therapeutic agent, an imaging agent, a diagnostic agent, a contrast agent, a labeling agent, a detection agent and a disinfectant. In some embodiments, the agent loaded in the delivery carrier may also include a biologically active substance, such as flavorant, sweetener, flavorant and odorant, pH adjuster, effervescent agent, emollient, filler, soluble organic salt, osmotic agent, antioxidant, and the like, which are not generally considered to be active ingredient.

In some embodiments, the agent loaded in the delivery carrier is a therapeutic agent. The therapeutic agent of the present invention is not particularly limited, including any suitable therapeutic agent that may be included in a delivery carrier. Exemplary therapeutic agents include, but not limited to, antiviral agents, antibacterial agents, antioxidants, thrombolytic agents, chemotherapeutic agents, anti-inflammatory agents, immunogenic agents, preservatives, anesthetics, analgesics, pharmaceutical agents, small molecules, peptides, proteins, antibodies, nucleic acids, and the like.

In some embodiments, the agent loaded in the delivery carrier comprises a therapeutic agent and the active ingredient of the therapeutic agent comprises a nucleic acid, for example, DNA or RNA. In some embodiments, the nucleic acid comprised in the agent loaded in the delivery carrier comprises one or more modified nucleotides.

In some embodiments, the agent loaded in the delivery carrier comprises a therapeutic agent and the active ingredient of the therapeutic agent comprises RNA. When the active ingredient in the therapeutic agent is RNA, since RNA is limited to cytoplasm and cannot be integrated into the genome, and the instability of RNA, the safety of the therapeutic agent whose active ingredient is RNA is high.

In some embodiments, the agent loaded in the delivery carrier comprises a therapeutic agent, the active ingredient of the therapeutic agent comprising a nucleic acid comprising DNA (e.g., plasmid) and/or RNA (e.g., mRNA).

In some embodiments, the nucleic acid comprised in the therapeutic agent loaded in the delivery carrier comprises one or more of: mRNA, nucleic acid based on RNAi technology, and sgRNA. In some embodiments, the nucleic acid based on RNAi technology is selected from one or more of siRNA, antisense oligonucleotide, and miRNA.

In some embodiments, the DNA or mRNA comprised in the therapeutic agent loaded in the delivery carrier includes one or more of DNA or RNA encoding a Cas protein (e.g., Cas9), DNA (e.g., plasmid) or mRNA encoding a CAR, DNA (e.g., plasmid) or mRNA encoding TCR, and DNA (e.g., plasmid) or mRNA encoding a therapeutic protein or polypeptide. In some embodiments, the therapeutic protein is an antibody, or the therapeutic protein is a protein or polypeptide for treating a rare disease.

In some embodiments, the mRNA comprised in the therapeutic agent loaded in the delivery carrier is mRNA encoding a Cas protein, mRNA of CAR, or mRNA of TCR.

In some embodiments, the DNA comprised in the therapeutic agent loaded in the delivery carrier is DNA (e.g., plasmid) encoding a therapeutic protein or polypeptide.

In some embodiments, the nucleic acid comprised in the therapeutic agent loaded in the delivery carrier comprises gRNA and mRNA encoding a Cas protein.

In some embodiments, the nucleic acid comprised in the therapeutic agent loaded in the delivery carrier comprises siRNA. In an optional specific example, the nucleic acid comprised in the therapeutic agent loaded in the delivery carrier is siRNA.

In some embodiments, the RNA comprised in the therapeutic agent loaded in the delivery carrier is a modified RNA. For example, the RNA is an RNA comprising one or more modified nucleotides. In some embodiments, the modified nucleotide of the RNA has a base modification and/or a sugar modification. In some embodiments, the modified nucleotide of the RNA also has a phosphate group modification. In some embodiments, the modified nucleotide of the RNA has a base modification.

In some embodiments, the RNA comprised in the agent loaded in the delivery carrier comprises one or more modified nucleosides. In some embodiments, the RNA comprises at least one of a modified uridine, a modified cytidine, a modified adenosine and a modified guanosine.

In some embodiments, the modified nucleoside in the RNA comprised in the agent loaded in the delivery carrier is a modified uridine. In some embodiments, 0.1% to 100% of uridines in the RNA are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99% or 100% uridines in the RNA comprised in the agent loaded in the delivery carrier are modified. In some embodiments, 80% - 100% of uridines are modified. In some embodiments, 100% of uridines are modified. Exemplary modified uridine comprises pseudouridine (ψ), N1-methylpseudouridine, pyridin-4-one ribonucleoside, 5-aza-uridine, 6-aza-uridine, 2-thio-5-aza-uridine, 2-thiouridine (s2U), 4-thiouridine (s4U), 4-thiopseudouridine, 2-thiopseudouridine, 5-hydroxyuridine (ho5U), 5-aminoallyluridine, 5-halouridine (e.g., 5-iodouridine or 5-bromouridine), 3-methyluridine (m3U), 5-methoxyuridine (mo5U), uridine-5-oxyacetic acid (cmo5U), methyl uridine-5-oxyacetate (mcmo5U), 5-carboxymethyluridine (cm5U), 1-carboxymethylpseudouridine, 5-carboxyhydroxymethyluridine (chm5U), 5-carboxyhydroxymethyluridine methyl ester (mchm5U), 5-methoxycarbonylmethyluridine (mcm5U), 5-methoxycarbonylmethyl-2-thiouridine (mcm5s2U), 5-aminomethyl-2-thiouridine (nm5s2U), 5-methylaminomethyluridine (mnm5U), 5-methylaminomethyl-2-thiouridine (mnm5s2U), 5-methylaminomethyl-2-selenouridine (mnm5se2U), 5-carbamoylmethyluridine (ncm5U), 5-carboxymethylaminomethyluridine (cmnm5U), 5-carboxymethylaminomethyl-2-thiouridine (cmnm5s2U), 5-propynyluridine, 1-propynylpseudouridine, 5-taurinomethyl-uridine (τm5U), 1-taurinomethylpseudouridine, 5-taurinomethyl-2-thiouridine (τm5s2U), 1-taurinomethyl-4-thiopseudouridine, 5-methyluridine (m5U , i.e., with the nucleobase deoxythymidine), 1-methylpseudouridine (m1ψ), 5-methyl-2-thiouridine (m5s2U), 1-methyl-4-thiopseudouridine (m1s4ψ), 4-thio-1-methylpseudouridine, 3-methylpseudouridine (m3ψ), 2-thio-1-methylpseudouridine, 1-methyl-1-deazapseudouridine, 2-thio-1-methyl-1-deazapseudouridine, dihydrouridine (D), dihydropseudouridine, 5,6-dihydrouridine, 5-methyldihydrouridine (m5D), 2-thiodihydrouridine, 2-thiodihydropseudouridine, 2-methoxyuridine, 2-methoxy-4-thiouridine, 4-methoxypseudouridine, 4-methoxy-2-thiopseudouridine, N1-methylpseudouridine, 3-(3-amino-3-carboxypropyl) uridine (acp3U), 1-methyl-3-(3-amino-3-carboxypropyl) pseudouridine (acp3ψ), 5-(isopentenylaminomethyl) uridine (inm5U), 5-(isopentenylaminomethyl)-2-thiouridine (inm5s2U), α-thiouridine, 2'-O-methyluridine (Um), 5,2'-O-dimethyluridine (m5Um), 2'-O-methylpseudouridine (ψm), 2-thio-2'-O-methyluridine (s2Um), 5-methoxycarbonylmethyl-2'-O-methyluridine (mcm5Um), 5-carbamoylmethyl-2'-O-methyluridine (ncm5Um), 5-carboxymethylaminomethyl-2'-O-methyluridine (cmnm5Um), 3,2'-O-dimethyluridine (m3Um), and 5-(isopentenylaminomethyl)-2'-O-methyluridine (inm5Um), 1-thiouridine, deoxythymidine, 2'-F-arabino-uridine (2'-F-ara-uridine), 2'-F-uridine, 2'-OH-arabino-uridine, 5-(2-carbomethoxyvinyl)uridine and 5-[3-(1-E-propenylamino)] uridine.

In some embodiments, the modified uridines in the RNA comprised in the agent loaded in the delivery carrier are of the same kind. For example, the RNA comprised in the agent loaded in the delivery carrier comprises a plurality of modified uridines, all of which are N1-methylpseudouridine. In other embodiments, the modified uridines in the RNA comprised in the agent loaded in the delivery carrier are of various kinds. For example, the RNA comprised in the agent loaded in the delivery carrier comprises a plurality of modified uridines that are at least two selected from the above exemplary modified uridines (e.g. pseudouridine and N1-methylpseudouridine).

In some embodiments, the modified nucleoside in the RNA comprised in the agent loaded in the delivery carrier is a modified cytidine. In some embodiments, 0.1%-100% of cytidines in the RNA are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99%, or 100% of cytidines in the RNA comprised in the agent loaded in the delivery carrier are modified. In some embodiments, 80%-100% of cytidines are modified. In some embodiments, 100% of cytidines are modified. Exemplary modified cytidine comprises 5-aza-cytidine, 6-aza-cytidine, pseudoisocytidine, 3-methylcytidine (m3C), N4-acetylcytidine (ac4C), 5-formylcytidine (f5C), N4-methylcytidine (m4C), 5-methylcytidine (m5C), 5-halocytidine (e.g., 5-iodocytidine), 5-hydroxymethylcytidine (hm5C), 1-methylpseudoisocytidine, pyrrolo-cytidine, pyrrolo-pseudoisocytidine, 2-thiocytidine (s2C), 2-thio-5-methylcytidine, 4-thiopseudoisocytidine, 4-thio-1-methylpseudoisocytidine, 4-thio-1-methyl-1-deazapseudoisocytidine, 1-methyl-1-deazapseudoisocytidine, zebularine, 5-aza-zebularine, 5-methylzebularine, 5-aza-2-thio-zebularine, 2-thio-zebularine, 2-methoxycytidine, 2-methoxy-5-methylcytidine, 4-methoxypseudoisocytidine, 4-methoxy-1-methylpseudoisocytidine, lysidine (k2C), α-thiocytidine, 2'-O-methylcytidine (Cm), 5,2'-O-dimethylcytidine (m5Cm), N4-acetyl-2'-O-methylcytidine (ac4Cm), N4,2'-O-dimethylcytidine (m4Cm), 5-formyl-2'-O-methylcytidine (f5Cm), N4,N4,2'-O-trimethylcytidine (m42Cm), 1-thiocytidine, 2'-F-arabino-cytidine, 2'-F-cytidine and 2'-OH-arabino-cytidine.

In some embodiments, the modified cytidines in the RNA comprised in the agent loaded in the delivery carrier are of the same kind. For example, the RNA comprised in the agent loaded in the delivery carrier comprises a plurality of modified cytidines, all of which are 5-aza-cytidine. In other embodiments, the modified cytidines of the RNA comprised in the agent loaded in the delivery carrier are of various kinds. For example, the RNA comprised in the agent loaded in the delivery carrier comprises a plurality of modified cytidines that are at least two selected from the above exemplary modified cytidines (e.g., 5-aza-cytidine and 6-aza-cytidine).

In some embodiments, the modified nucleoside in the RNA comprised in the agent loaded in the delivery carrier is a modified adenosine. In some embodiments, 0.1%-100% of adenosines in the RNA are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99%, or 100% of adenosines in the RNA comprised in the agent loaded in the delivery carrier are modified. In some embodiments, 80%-100% of adenosines are modified. In some embodiments, 100% of adenosines are modified. Exemplary modified adenosine comprises 2-amino-purine, 2,6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenine, 7-deaza-8-aza-adenine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m1A), 2-methyl-adenine (m2A), N6-methyl-adenosine (m6A), 2-methylthio-N6-methyl-adenosine (ms2m6A), N6-isopentenyl-adenosine (i6A), 2-methylthio-N6-isopentenyl-adenosine (ms2i6A), N6-(cis-hydroxyisopentenyl) adenosine (io6A), 2-methylthio-N6-(cis-hydroxyisopentenyl) adenosine (ms2io6A), N6-glycylcarbamoyl-adenosine (g6A), N6-threonylcarbamoyl-adenosine (t6A), N6-methyl-N6-threonylcarbamoyl-adenosine (m6t6A), 2-methylthio-N6-threonylcarbamoyl-adenosine (ms2g6A), N6,N6-dimethyl-adenosine (m62A), N6-hydroxynorvalylcarbamoyl-adenosine (hn6A), 2-methylthio-N6-hydroxynorvalylcarbamoyl-adenosine (ms2hn6A), N6-acetyl-adenosine (ac6A), 7-methyl-adenine, 2-methylthio-adenine, 2-methoxy-adenine, α-thio-adenosine, 2'-O-methyl-adenosine (Am), N6,2'-O-dimethyl-adenosine (m6Am), N6,N6,2'-O-trimethyl-adenosine (m62Am), 1,2'-O-dimethyl-adenosine (m1Am), 2'-O-ribosyladenosine (phosphate) (Ar(p)), 2-amino-N6-methyl-purine, 1-thio-adenosine, 8-azido-adenosine, 2'-F-arabino-adenosine, 2'-F-adenosine, 2'-OH-arabino-adenosine and N6-(19-amino-pentaoxanonadecyl)-adenosine.

In some embodiments, the modified adenosines in the RNA comprised in the agent loaded in the delivery carrier are of the same kind. For example, the RNA comprised in the agent loaded in the delivery carrier comprises a plurality of modified adenosines, all of which are 2-amino-purine. In other embodiments, the modified adenosines in the RNA comprised in the agent loaded in the delivery carrier are of various kinds. For example, the RNA comprised in the agent loaded in the delivery carrier comprises a plurality of modified adenosines that are at least two selected from the above exemplary modified adenosines (e.g. 2-amino-purine and 2,6 - diaminopurine).

In some embodiments, the modified nucleoside in the RNA comprised in the agent loaded in the delivery carrier is a modified guanosine. In some embodiments, 0.1%-100% of guanosines in the RNA are modified. For example, at least 0.1%, at least 0.5%, at least 1%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 88%, at least 90%, at least 92%, at least 95%, at least 97%, at least 99%, or 100% of guanosines in the RNA comprised in the agent loaded in the delivery carrier are modified. In some embodiments, 80%-100% of guanosines are modified. In some embodiments, 100% of guanosines are modified. Exemplary modified guanosine comprises inosine (I), 1-methyl-inosine (m1I), wyosine (imG), methylwyosine (mimG), 4-demethyl-wyosine (imG-14), isowyosine (imG2), wybutosine (yW), peroxywybutosine (o2yW), hydroxywybutosine (OHyW), undermodified hydroxywybutosine (OHyW*), 7-deaza-guanosine, queuosine (Q), epoxyqueuosine (oQ), galactosyl-queuosine (galQ), mannosyl-queuosine (manQ), 7-cyano-7-deaza-guanosine (preQ0), 7-aminomethyl-7-deaza-guanosine (preQ1), archaeosine (G+), 7-deaza-8-aza-guanosine, 6-thio-guanosine, 6-thio-7-deaza-guanosine, 6-thio-7-deaza-8-aza-guanosine, 7-methyl-guanosine (m7G), 6-thio-7-methyl-guanosine, 7-methyl-inosine, 6-methoxy-guanosine, 1-methyl-guanosine (m1G), N2-methyl-guanosine (m2G), N2,N2-dimethyl-guanosine (m22G), N2,7-dimethyl-guanosine (m2,7G), N2,N2,7-trimethyl-guanosine (m2,2,7G), 8-oxo-guanosine, 7-methyl-8-oxo-guanosine, 1-methyl-6-thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m2Gm), N2,N2-dimethyl-2'-O-methyl-guanosine (m22Gm), 1-methyl-2'-O-methyl-guanosine (m1Gm), N2,7-dimethyl-2'-O-methyl-guanosine (M2,7Gm), 2'-O-methyl-inosine (Im), 1,2'-O-dimethyl-inosine (m1Im), 2'-O-ribosylguanosine (phosphate) (Gr(p)), 1-thio-guanosine, O6-methyl-guanosine, 2'-F-arabino-guanosine and 2'-F-guanosine.

In some embodiments, the modified guanosines in the RNA comprised in the agent loaded in the delivery carrier are of the same kind. For example, the RNA comprised in the agent loaded in the delivery carrier comprises a plurality of modified guanosines, all of which are inosine. In other embodiments, the modified guanosines of the RNA comprised in the agent loaded in the delivery carrier are of various kinds. For example, the RNA comprised in the agent loaded in the delivery carrier comprises a plurality of modified guanosines that are at least two selected from the above exemplary modified guanosines (e.g. inosine and 1-methyl-inosine).

In addition, the modified nucleotide in the RNA comprised in the agent loaded in the delivery carrier comprises an isotope-containing nucleotide. In some embodiments, the RNA comprises a nucleotide comprising an isotope of hydrogen. The isotope of hydrogen is not limited to deuterium, tritium. In addition, in some embodiments, the RNA further comprises or contains a nucleotide containing an isotope of other element than hydrogen, wherein the element includes, but not limited to, carbon, oxygen, nitrogen, and phosphorus.

In some embodiments, the agent loaded in the delivery carrier comprises mRNA. In some embodiments, the mRNA comprised in the agent loaded in the delivery carrier is a modified mRNA.

In some embodiments, the modified mRNA comprises one or more of a modified mRNA encoding a chimeric antigen receptor, a modified mRNA encoding a T cell receptor, a modified mRNA encoding a therapeutic polypeptide or protein, and a modified mRNA encoding an antigen.

In some embodiments, the modified mRNA comprises a modified mRNA encoding a chimeric antigen receptor or a T cell receptor.

In some embodiments, the modified mRNA comprises one or more modified nucleosides. In some embodiments, the modified mRNA comprises at least one of a modified uridine, a modified cytidine, a modified adenosine, and a modified guanosine.

In some embodiments, the modified mRNA comprises a modified uridine. In some embodiments, 0.1%-100% of uridines in the modified RNA are modified. In some embodiments, 80%-100% of uridines are modified. In some embodiments, 100% of uridines are modified. Exemplary modified uridine is described as above.

In some embodiments, the modified mRNA comprises a modified cytidine. In some embodiments, 0.1%-100% of cytidines in the modified RNA are modified. In some embodiments, 80%-100% of cytidines are modified. In some embodiments, 100% of cytidines are modified. Exemplary modified cytidine is described as above.

In some embodiments, the modified mRNA comprises a modified adenosine. In some embodiments, 0.1%-100% of adenosines in the modified RNA are modified. In some embodiments, 80%-100% of adenosines are modified. In some embodiments, 100% of adenosines are modified. Exemplary modified adenosine is described as above.

In some embodiments, the modified mRNA comprises a modified guanosine. In some embodiments, 0.1%-100% of guanosines in the modified RNA are modified. In some embodiments, 80%-100% of guanosines are modified. In some embodiments, 100% of guanosines are modified. Exemplary modified guanosine is described as above.

In addition, the modified nucleotide comprised in the modified mRNA comprises an isotope-containing nucleotide. In some embodiments, the mRNA comprises a nucleotide comprising an isotope of hydrogen. The isotope of hydrogen is not limited to deuterium, tritium. In addition, in some embodiments, the mRNA further comprises or contains a nucleotide containing an isotope of other element than hydrogen, wherein the element includes, but not limited to, carbon, oxygen, nitrogen, and phosphorus.

It will be appreciated that in other embodiments, the active ingredient in the agent loaded in the delivery carrier is not limited to nucleic acid, but may be other substances.

### Regulatory Element

In some embodiments, the active ingredient of the therapeutic agent in the modified delivery carrier comprises mRNA. In an optional specific example, the active ingredient of the therapeutic agent in the modified delivery carrier is mRNA. In some embodiments, the mRNA in the modified delivery carrier comprises a regulatory element for regulating the expression of the mRNA therein. For example, it is used to increase or decrease the expression of mRNA therein.

In some embodiments, the regulatory element is microRNA binding site for regulating the expression of mRNA in the modified delivery carrier, for example, to increase or decrease the expression of mRNA therein, preferably for reducing the expression of mRNA in the modified delivery carrier in undesired cells and/or tissues. It can be understood that in other embodiments, the regulatory element is not limited to microRNA binding site, and may be other elements capable of regulating mRNA expression, such as untranslated region (UTR).

The microRNA (or referred to as miRNA) is small non-coding RNA, which can cause silencing after transcription of a specific gene in a cell by inhibiting translation or by degrading targeted mRNA. The microRNA has a seed sequence which is generally a nucleotide sequence of positions 2-8 at the 5' end of the microRNA. Exemplary microRNAs and their sequences can be found in US2005261218 and US2005059005.

The microRNA binding site is a polynucleotide capable of binding to microRNA, which can achieve the binding of microRNA to mRNA in which the microRNA binding site is located.

In some embodiments, the microRNA binding site has sufficient complementarity to the microRNA.

That the microRNA binding site has sufficient complementarity to the microRNA means that the complementarity of the microRNA to the microRNA binding site is sufficient to achieve microRNA-mediated mRNA regulation, for example, the inhibition or degradation of the microRNA-mediated translation of mRNA. In some embodiments, that the microRNA binding site has sufficient complementarity to the microRNA means that the complementarity of the microRNA binding site to the microRNA binding site is sufficient to achieve microRNA-mediated mRNA degradation, for example, microRNA-guided RISC-mediated mRNA lysis. For example, the microRNA binding site is complementary to microRNAs of 19-25 or 19-23 (e.g., 22) nucleotides. In some embodiments, the microRNA binding site is only complementary to a portion of the microRNA, for example, complementary to a portion that is 1, 2, 3 or 4 nucleotides shorter than a naturally occurring microRNA. Completely complementary (e.g., completely complementary to all or significant portions of native microRNA) is preferred when the desired regulation is mRNA degradation.

In some embodiments, the microRNA binding site comprises a polynucleotide having sufficient complementarity (e.g., partial or complete complementarity) to a microRNA sequence. In some embodiments, the microRNA binding site comprises a sequence having complete complementarity to a microRNA sequence.

In some embodiments, the microRNA binding site comprises a polynucleotide having sufficient complementarity (e.g., partial or complete complementarity) to a seed sequence of microRNA. In some embodiments, the microRNA binding site comprises a polynucleotide having complete complementarity to the microRNA seed sequence.

In some embodiments, the microRNA binding site has complete complementarity to the microRNA sequence except for 1, 2 or 3 nucleotide substitutions, terminal addition and/or truncation.

The microRNA binding site is a polynucleotide or a variant thereof that is complementarily paired with a full-length microRNA or partial microRNA, wherein the variant retains the ability of the microRNA binding site to bind to microRNA, and can achieve the binding of microRNA to mRNA in which the microRNA binding site is located. The microRNA binding site may be completely complementary paired with a full-length microRNA or partial microRNA, or may not be completely complementary paired with a full-length microRNA or partial microRNA.

In some embodiments, the microRNA binding site comprises a polynucleotide that is complementarily paired with a seed sequence of microRNA or does not comprise a polynucleotide that is complementarily paired with a seed sequence (in whole or in part) of microRNA. In some embodiments, the microRNA binding site is a polynucleotide that is completely complementarily paired with a full-length microRNA.

In some embodiments, the active ingredient of the therapeutic agent in the modified delivery carrier is mRNA which comprises one or more microRNA binding sites. For example, the mRNA comprises one, two, three, four, five, six, seven, eight, nine or ten microRNA binding sites. In some embodiments, the mRNA in the therapeutic agent comprises one microRNA binding site. In some embodiments, the mRNA in the therapeutic agent comprises three microRNA binding sites.

In some embodiments, the regulatory element of mRNA in the modified delivery carrier further comprises UTR. In some embodiments, the mRNA in the modified delivery carrier comprises one or both of 5'-UTR and 3'-UTR.

In some embodiments, the mRNA in the modified delivery carrier comprises 3'-UTR and a microRNA binding site located in 3'-UTR. In some embodiments, the mRNA in the modified delivery carrier comprises 3'-UTR, and the position of the microRNA binding site is selected from one or more of a 5' end close to 3'-UTR, a region between 5' and 3' ends of 3'-UTR, and a 3' end close to 3'-UTR.

In some embodiments, the mRNA in the modified delivery carrier comprises 5'-UTR and a microRNA binding site located in 5'-UTR.

In some embodiments, the mRNA in the modified delivery carrier comprises 5'-UTR, and the position of the microRNA binding site is selected from one or more of a 5' end close to 5'-UTR, a region between 5' end and the 3' end of 5'-UTR, and a 3' end close to 5'-UTR.

In some embodiments, the mRNA in the modified delivery carrier comprises 5'-UTR, 3'-UTR, and a microRNA binding site located in 5'-UTR and/or 3'-UTR.

In some embodiments, the mRNA in the modified delivery carrier comprises 5'-UTR, 3'-UTR and a microRNA binding site, wherein the position of the microRNA binding site is selected from one of a 5' end close to 3'-UTR, a region between the 5' and 3' ends of 3'-UTR, a 3' end close to 3'-UTR, a 5' end close to 5'-UTR, a region between the 5' and 3' ends of the 5'-UTR, and a 3' end close to 5'-UTR.

In some embodiments, the mRNA in the modified delivery carrier comprises 5'-UTR, 3'-UTR and a plurality of microRNA binding sites, wherein the positions of the plurality of microRNA binding sites are selected from one or more of a 5' end close to 3'-UTR, a region between 5' and 3' ends of 3'-UTR, a 3' end close to 3'-UTR, a 5' end close to 5'-UTR, a region between 5' and 3' ends of 5'-UTR, and a 3' end close to 5'-UTR. For example, each of the plurality of microRNA binding sites is close to 5' end of 3'-UTR, is located in the region between 5' end and 3' end of 3'-UTR, is located in the region between 5' end and 3' end of 5'-UTR, or is close to 3' end of 5'-UTR. For another example, some of the plurality of microRNA binding sites are close to 5' end of 5'-UTR, and the left are close to 5' end of 3'-UTR. For another example, some of the plurality of microRNA binding sites are close to 5' end of 5'-UTR, some are located in the region between 5' end and 3' end of 5'-UTR, and the left are close to 3' end of 3'-UTR. It may be understood that the position selection of the plurality of microRNA binding sites is not limited to the above examples, and may also be a combination of other positions.

In some embodiments, the active ingredient of the therapeutic agent in the modified delivery carrier is mRNA comprising a plurality of microRNA binding sites, wherein the plurality of microRNA binding sites are the same or different.

In some embodiments, the active ingredient of the therapeutic agent in the modified delivery carrier is mRNA comprising a plurality of microRNA binding sites, wherein the plurality of microRNA binding sites are the same. For example, in some embodiments, the mRNA comprises three microRNA binding sites capable of specifically binding to miR-122-3p, wherein the three microRNA binding sites capable of specifically binding to miR-122-3p are exactly the same.

In some embodiments, the active ingredient of the therapeutic agent in the modified delivery carrier is mRNA comprising a plurality of microRNA binding sites, wherein the plurality of microRNA binding sites are different. The plurality of microRNA binding sites bind to one microRNA or to different microRNAs.

In some embodiments, the active ingredient of the therapeutic agent in the modified delivery carrier is mRNA comprising a plurality of microRNA binding sites, wherein the plurality of microRNA binding sites are different and bind to the same microRNA.

For example, in some embodiments, the mRNA comprises three microRNA binding sites capable of specifically binding to miR-122-3p, wherein the first of the three microRNA binding sites capable of specifically binding to miR-122-3p can specifically bind to 5' end of miR-122-3p, the second can specifically bind to 3' end of miR-122-3p, and the third is exactly identical to, partially the same as, or completely different from one of the foregoing two.

In some embodiments, the active ingredient of the therapeutic agent in the modified delivery carrier is mRNA comprising a plurality of microRNA binding sites, wherein the plurality of microRNA binding sites are different, wherein the plurality of microRNA binding sites bind to different microRNAs from the same cell and/or tissue, or from different cells and/or tissues. The plurality of microRNA binding sites are used to increase or decrease the expression of mRNA where they are present in a specific cell or tissue, or to increase or decrease the expression of mRNA where they are present in several specific cells and/or tissues.

In some embodiments, the plurality of microRNA binding sites comprise different microRNA binding sites capable of specifically binding to different microRNAs expressed in the same tissue or the same cell. For example, in some embodiments, the mRNA comprises three microRNA binding sites, each of which is capable of specifically binding to a microRNA specifically expressed in liver. For example, the first of the three microRNA binding sites can specifically bind to miR-107 specifically expressed in liver, the second can specifically bind to miR-122-5p specifically expressed in liver, and the third can specifically bind to miR-107 specifically expressed in liver, miR-122-5p specifically expressed in liver or other microRNAs specifically expressed in liver.

In some embodiments, the plurality of microRNA binding sites are capable of specifically binding to at least two different microRNAs expressed in different tissues or different classes of cells. For example, the mRNA comprises three microRNA binding sites, wherein the first of the three microRNA binding sites can specifically bind to miR-107 expressed in liver, and the second can specifically bind to hsa-let-7a-2-3p expressed in immune cells.

In some embodiments, microRNAs expressed in immune cells include, but not limited to, one or more of hsa-let-7a-2-3p, hsa-let-7a-3p, hsa-7a-5p, hsa-let-7c, hsa-let-7e-3p, hsa-let-7e-5p, hsa-let-7g-3p, hsa-let-7g-5p, hsa-let-7i-3p, hsa-let-7i-5p, miR-10a-3p, miR-10a-5p, miR-1184, hsa-let-7f-1-3p, hsa-let-7f-2~5p, hsa-let-7f-5p, miR-125b-l-3p, miR-125b-2-3p, miR-125b-5p, miR-1279, miR-130a-3p, miR-130a-5p, miR-132-3p, miR-132-5p, miR-142-3p, miR-142-5p, miR-143-3p, miR-143-5p, miR-146a-3p, miR-146a-5p, miR-146b-3p, miR-146b-5p, miR-147a, miR-147b, miR-148a-5p, miR-148a-3p, miR-150-3p, miR-150-5p, miR-151b, miR-155-3p, miR-155-5p, miR-15a-3p, miR-15a-5p, miR-15b-5p, miR-15b-3p, miR-16-l-3p, miR-16-2-3p, miR-16-5p, miR-17-5p, miR-181a-3p, miR-181a-5p, miR-181a-2-3p, miR-182-3p, miR-182-5p, miR-197-3p, miR-197-5p, miR-21-5p, miR-21-3p, miR-214-3p, miR-214-5p, miR-223-3p, miR-223-5p, miR-221-3p, miR-221-5p, miR-23b-3p, miR-23b-5p, miR-24-l-5p, miR-24-2-5p, miR-24-3p, miR-26a-l-3p, miR-26a-2-3p, miR-26a-5p, miR-26b-3p, miR-26b-5p, miR-27a-3p, miR-27a-5p, miR-27b-3p, miR-27b-5p, miR-28-3p, miR-28-5p, miR-2909, miR-29a-3p, miR-29a-5p, miR-29b-l-5p, miR-29b-2-5p, miR-29c-3p, miR-29c-5p, miR-30e-3p, miR-30e-5p, miR-331-5p, miR-339-3p, miR-339-5p, miR-345-3p, miR-345-5p, miR-346, miR-34a-3p, miR-34a-5p, miR-363-3p, miR-363-5p, miR-372, miR-377-3p, miR-377-5p, miR-493-3p, miR-493-5p, miR-542, miR-548b-5p, miR548c-5p, miR-548i, miR-548j, miR-548n, miR-574-3p, miR-598, miR-718, miR-935, miR-99a-3p, miR-99a-5p, miR-99b-3p and miR-99b-5p. In addition, microRNAs expressed in immune cells also include microRNAs identified by Jima DD et al, Blood, 2010, 116: e118-e127 and Vaz C et al, BMC Genomics, 2010, 11, 288.

In some embodiments, microRNAs expressed in liver include, but not limited to, one or more of miR-107, miR-122-3p, miR-122-5p, miR-1228-3p, miR-1228-5p, miR-1249, miR-129-5p, miR-1303, miR-151a-3p, miR-151a-5p, miR-152, miR-194-3p, miR-194-5p, miR-199a-3p, miR-199a-5p, miR-199b-3p, miR-199b-5p, miR-296-5p, miR-557, miR-581, miR-939-3p and miR-939-5p.

In some embodiments, microRNAs expressed in lung include, but not limited to, one or more of let-7a-2-3p, let-7a-3p, let-7a-5p, miR-126-3p, miR-126-5p, miR-127-3p, miR-127-5p, miR-130a-3p, miR-130a-5p, miR-130b-3p, miR-130b-5p, miR-133a, miR-133b, miR-134, miR-18a-3p, miR-18a-5p, miR-18b-3p, miR-18b-5p, miR-24-l-5p, miR-24-2-5p, miR-24-3p, miR-296-3p, miR-296-5p, miR-32-3p, miR-337-3p, miR-337-5p, miR-381-3p, miR-381-5p.

In some embodiments, microRNAs expressed in heart include, but not limited to, one or more of miR-1, miR-133a, miR-133b, miR-149-3p, miR-149-5p, miR-186-3p, miR-186-5p, miR-208a, miR-208b, miR-210, miR-296-3p, miR-320, miR-451a, miR-451b, miR-499a-3p, miR-499a-5p, miR-499b-3p, miR-499b-5p, miR-744-3p, miR-744-5p, miR-92b-3p and miR-92b-5p.

In some embodiments, microRNAs expressed in nervous system include, but not limited to, one or more of miR-124-5p, miR-125a-3p, miR-125a-5p, miR-125b-l-3p, miR-125b-2-3p, miR-125b-5p, miR-1271-3p, miR-1271-5p, miR-128, miR-132-5p, miR-135a-3p, miR-135a-5p, miR-135b-3p, miR-135b-5p, miR-137, miR-139-5p, miR-139-3p, miR-149-3p, miR-149-5p, miR-153, miR-181c-3p, miR-181c-5p, miR-183-3p, miR-183-5p, miR-190a, miR-190b, miR-212-3p, miR-212-5p, miR-219-1-3p, miR-219-2-3p, miR-23a-3p, miR-23a-5p, miR-30a-5p, miR-30b-3p, miR-30b-5p, miR-30c-1-3p, miR-30c-2-3p, miR-30c-5p, miR-30d-3p, miR-30d-5p, miR-329, miR-342-3p, miR-3665, miR-3666, miR-380-3p, miR-380-5p, miR-383, miR-410, miR-425-3p, miR-425-5p, miR-454-3p, miR-454-5p, miR-483, miR-510, miR-516a-3p, miR-548b-5p, miR-548c-5p, miR-571, miR-7-1-3p, miR-7-2-3p, miR-7-5p, miR-802, miR-922, miR-9-3p and miR-9-5p.

In some embodiments, microRNAs specifically expressed in neurons include, but not limited to, one or more of miR-132-3p, miR-132-3p, miR-148b-3p, miR-148b-5p, miR-151a-3p, miR-151a-5p, miR-212-3p, miR-212-5p, miR-320b, miR-320e, miR-323a-3p, miR-323a-5p, miR-324-5p, miR-325, miR-326, miR-328 and miR-922. MicroRNAs specifically expressed in glial cells include, but not limited to, one or more of miR-1250, miR-219-1-3p, miR-219-2-3p, miR-219-5p, miR-23a-3p, miR-23a-5p, miR-3065-3p, miR-3065-5p, miR-30e-3p, miR-30e-5p, miR-32-5p, miR-338-5p and miR-657.

In some embodiments, microRNAs expressed in pancreas include, but not limited to, one or more of miR-105-3p, miR-105-5p, miR-184, miR-195-3p, miR-195-5p, miR-196a-3p, miR-196a-5p, miR-214-3p, miR-214-5p, miR-216a-3p, miR-216a-5p, miR-30a-3p, miR-33a-3p, miR-33a-5p, miR-375, miR-7-1-3p, miR-7-2-3p, miR-493-3p, miR-493-5p and miR-944.

In some embodiments, microRNAs expressed in kidney include, but not limited to, one or more of miR-122-3p, miR-145-5p, miR-17-5p, miR-192-3p, miR-192-5p, miR-194-3p, miR-194-5p, miR-20a-3p, miR-20a-5p, miR-204-3p, miR-204-5p, miR-210, miR-216a-3p, miR-216a-5p, miR-296-3p, miR-30a-3p, miR-30a-5p, miR-30b-3p, miR-30b-5p, miR-30c-1-3p, miR-30c-2-3p, miR30c-5p, miR-324-3p, miR-335-3p, miR-335-5p, miR-363-3p, miR-363-5p and miR-562.

In some embodiments, microRNAs expressed in muscle include, but not limited to, one or more of let-7g-3p, let-7g-5p, miR-1, miR-1286, miR-133a, miR-133b, miR-140-3p, miR-143-3p, miR-143-5p, miR-145-3p, miR-145-5p, miR-188-3p, miR-188-5p, miR-206, miR-208a, miR-208b, miR-25-3p and miR-25-5p.

In some embodiments, microRNAs expressed in endothelial cell include, but not limited to, one or more of let-7b-3p, let-7b-5p, miR-100-3p, miR-100-5p, miR-101-3p, miR-101-5p, miR-126-3p, miR-126-5p, miR-1236-3p, miR-1236-5p, miR-130a-3p, miR-130a-5p, miR-17-5p, miR-17-3p, miR-18a-3p, miR-18a-5p, miR-19a-3p, miR-19a-5p, miR-19b-1-5p, miR-19b-2-5p, miR-19b-3p, miR-20a-3p, miR-20a-5p, miR-217, miR-210, miR-21-3p, miR-21-5p, miR-221-3p, miR-221-5p, miR-222-3p, miR-222-5p, miR-23a-3p, miR-23a-5p, miR-296-5p, miR-361-3p, miR-361-5p, miR-421, miR-424-3p, miR-424-5p, miR-513a-5p, miR-92a-1-5p, miR-92a-2-5p, miR-92a-3p, miR-92b-3p and miR-92b-5p, and in some embodiments, microRNAs expressed in endothelial cell include one or more in Voellenkle C et al., RNA, 2012, 18, 472-484.

In some embodiments, microRNAs expressed in epithelial cell include, but not limited to, one or more of let-7b-3p, let-7b-5p, miR-1246, miR-200a-3p, miR-200a-5p, miR-200b-3p, miR-200b-5p, miR-200c-3p, miR-200c-5p, miR-338-3p, miR-429, miR-451a, miR-451b, miR-494, miR-802. In some embodiments, microRNAs specifically expressed in respiratory cilia epithelial cell include, but not limited to, one or more of miR-34a, miR-34b-5p, miR-34c-5p, miR-449a, miR-449b-3p, miR-449b-5p. MicroRNAs specifically expressed in lung epithelial cell include, but not limited to, one or more of let-7 family, miR-133a, miR-133b and miR-126. MicroRNAs specifically expressed in renal tubular epithelial cell include, but not limited to, one or more of miR-382-3p and miR-382-5p. MicroRNAs specifically expressed in corneal epithelial cell include, but not limited to, miR-762.

In some embodiments, microRNAs expressed in embryonic stem cell include, but not limited to, one or more of let-7a-2-3p, let-a-3p, let-7a-5p, let7d-3p, let-7d-5p, miR-103a-2-3p, miR-103a-5p, miR-106b-3p, miR-106b-5p, miR-1246, miR-1275, miR-138-1-3p, miR-138-2-3p, miR-138-5p, miR-154-3p, miR-154-5p, miR-200c-3p, miR-200c-5p, miR-290, miR-301a-3p, miR-301a-5p, miR-302a-3p, miR-302a-5p, miR-302b-3p, miR-302b-5p, miR-302c-3p, miR-302c-5p, miR-302d-3p, miR-302d-5p, miR-302e, miR-367-3p, miR-367-5p, miR-369-3p, miR-369-5p, miR-370, miR-371, miR-373, miR-380-5p, miR-423-3p, miR-423-5p, miR-486-5p, miR-520c-3p, miR-548e, miR-548f, miR-548g-3p, miR-548g-5p, miR-548i, miR-548k, miR-5481, miR-548m, miR-548n, miR-548o-3p, miR-548o-5p, miR-548p, miR-664a-3p, miR-664a-5p, miR-664b-3p, miR-664b-5p, miR-766-3p, miR-766-5p, miR-885-3p, miR-885-5p, miR-93-3p, miR-93-5p, miR-941, miR-96-3p, miR-96-5p, miR-99b-3p and miR-99b-5p. In some embodiments, microRNAs expressed in embryonic stem cell include, but not limited to, one or more mentioned in Morin RD et al, Genome Res, 2008, 18, 610-621; Goff LA et al, PLoS One, 2009, 4:e7192; and Bar M et al, Stem cells, 2008, 26, 2496-2505.

Some microRNAs are abnormally overexpressed in certain abnormal cells (such as cancer cells), while others are under-expressed in certain abnormal cells. For example, cells, tissues or diseases with differentially expressed microRNAs include: cancer cells (WO2008/154098, US2013/0059015, US2013/0042333, WO2011/157294), cancer stem cells (US2012/0053224), pancreatic cancer and diseases (US2009/0131348, US201 1/0171646, US2010/0286232, US8389210), Asthma and Inflammation (US8415096), prostate cancer (US2013/0053264), hepatocellular carcinoma (WO2012/151212, US2012/0329672, WO2008/054828, US8252538), lung cancer cells (WO2011/076143, WO2013/033640, WO2009/070653, US2010/0323357), cutaneous T-cell lymphoma (WO2013/011378), colorectal cancer cells (WO2011/0281756, WO2011/076142), cancer-positive lymph nodes (WO2009/100430 , US2009/0263803), nasopharyngeal carcinoma (EP2112235), chronic obstructive pulmonary disease (US2012/0264626, US2013/0053263), thyroid cancer (WO2013/066678), ovarian cancer cells (US2012/0309645, WO2011/095623), breast cancer cells (WO2008/154098, WO2007/081740, US2012/0214699), leukemia and lymphoma (WO2008/073915, US2009/0092974, US2012/0316081, US2012/0283310, WO2010/018563).

In some embodiments, the microRNA binding site includes one or more of a microRNA binding site specifically binding microRNA expressed in immune cells, a microRNA binding site specifically binding microRNA expressed in liver, a microRNA binding site specifically binding microRNA expressed in lung, a microRNA binding site specifically binding microRNA expressed in heart, a microRNA binding site specifically binding microRNA expressed in nervous system, a microRNA binding site specifically binding microRNA expressed in pancreas, a microRNA binding site specifically binding microRNA expressed in kidney, a microRNA binding site specifically binding microRNA expressed in muscle, a microRNA binding site specifically binding microRNA expressed in endothelial cell, a microRNA binding site specifically binding microRNA expressed in epithelial cell, and a microRNA binding site specifically binding microRNA expressed in embryonic stem cell.

In some embodiments, the microRNA binding site comprises a microRNA binding site specifically binding microRNA expressed in immune cells and one of a microRNA binding site specifically binding microRNA expressed in liver, a microRNA binding site specifically binding microRNA expressed in lung, a microRNA binding site specifically binding microRNA expressed in heart, a microRNA binding site specifically binding microRNA expressed in nervous system, a microRNA binding site specifically binding microRNA expressed in pancreas, a microRNA binding site specifically binding microRNA expressed in kidney, a microRNA binding site specifically binding microRNA expressed in muscle, a microRNA binding site specifically binding microRNA expressed in endothelial cell, a microRNA binding site specifically binding microRNA expressed in epithelial cell, and a microRNA binding site specifically binding microRNA expressed in embryonic stem cell.

In some embodiments, the microRNA binding site includes one or more of a microRNA binding site specifically binding microRNA expressed in liver, a microRNA binding site specifically binding microRNA expressed in lung, a microRNA binding site specifically binding microRNA expressed in heart, a microRNA binding site specifically binding microRNA expressed in nervous system, a microRNA binding site specifically binding microRNA expressed in pancreas, a microRNA binding site specifically binding microRNA expressed in kidney, a microRNA binding site specifically binding microRNA expressed in muscle, a microRNA binding site specifically binding microRNA expressed in endothelial cell, a microRNA binding site specifically binding microRNA expressed in epithelial cell, and a microRNA binding site specifically binding microRNA expressed in embryonic stem cell.

In some embodiments, the modified delivery carrier comprises a targeting domain coupled to the delivery carrier.

In some embodiments, the modified delivery carrier comprises a plurality of targeting domains coupled to the delivery carrier. When the modified delivery carrier comprises a plurality of targeting domains, the modified delivery carrier is capable of targeting at least one immune cell having at least one surface antigen capable of binding to at least one of the plurality of targeting domains of the modified delivery carrier.

In some embodiments, the modified delivery carrier comprises two, three, four or five targeting domains.

In one alternative specific example, the modified delivery carrier comprises two targeting domains. In some embodiments, the two targeting domains may be two monoclonal antibodies which are coupled to the delivery carrier, respectively, or bispecific antibodies which are coupled to the delivery carrier.

In another alternative specific example, the modified delivery carrier comprises three targeting domains. In some embodiments, the three targeting domains may be three monoclonal antibodies, or a combination of one bispecific antibody and one monoclonal antibody. Three monoclonal antibodies or a combination of one bispecific antibody and one monoclonal antibody can be coupled to the delivery carrier via a coupling manner at least comprising one of (1) coupling three monoclonal antibodies respectively to the delivery carrier, (2) independently coupling one of the three monoclonal antibodies to the delivery carrier, coupling one of the left two monoclonal antibodies to the delivery carrier, and coupling the other to the monoclonal antibody coupled to the delivery carrier, (3) coupling the bispecific antibody to the delivery carrier, or coupling one of the bispecific antibody and the monoclonal antibody to the delivery carrier, and the other to the antibody coupled to the delivery carrier.

In another alternative specific example, the modified delivery carrier comprises four targeting domains. In some embodiments, three targeting domains may be four monoclonal antibodies, may be two bispecific antibodies, may be a combination of one bispecific antibody and two monoclonal antibodies, or may be a combination of one monoclonal antibody and one tri-specific antibody (tribody), or may also be one tetra-specific antibody (tetrabody). Tri-specific antibody is an antibody having three specific antigen-binding sites. Tetra-specific antibody is an antibody having four specific antigen-binding sites. The coupling manner between the four targeting domains and the delivery carrier may be analogized with reference to the coupling manner between the three targeting domains and the delivery carrier.

In some embodiments, a plurality of targeting domains of the modified delivery carrier are each independently coupled to the same delivery carrier. In other embodiments, at least one of the plurality of targeting domains of the modified delivery carrier is coupled to the delivery carrier, and at least one of the left targeting domains is coupled to the targeting domain coupled to the delivery carrier.

Compared with traditional cell immunotherapy, the above prevention or treatment method using the above modified delivery carrier has at least the following advantages: there is no need to collect immune cells from the body, nor to modify the collected immune cells in vitro, for example, it can be directly injected, the operation is simpler, and the patient comfort is higher. In addition, due to the action of the targeting domain, the modified delivery carrier described above can specifically target a certain type or several types of immune cells, which can reduce non-specific delivery, thereby improving the effective delivery rate of therapeutic agent and reducing the dose of therapeutic agent, thereby reducing the immunological side effects of therapeutic agent.

The present invention also provides another modified delivery carrier that differs from the modified delivery carrier described above in that the targeting domain of the modified delivery carrier is different from the targeting domain of the modified delivery carrier described above, wherein the targeting domain of the modified delivery carrier is capable of binding to a surface antigen of non-immune cell.

In some embodiments, the targeting domain of the modified delivery carrier can specifically bind to surface antigens of one or more of the following cells: liver cells, kidney cells, spleen cells, lung cells, cardiomyocytes and fibroblasts. It can be understood that the type of the surface antigen capable of specifically binding to the targeting domain of the modified delivery carrier is not limited to one, and may be several, such as two, three or four.

In some embodiments, the targeting domain of the modified delivery carrier is not bound to a surface antigen of immune cell.

### III. Method for preparing modified delivery carrier and kit for preparing modified delivery carrier

The present invention further provides a preparation method of a modified delivery carrier, comprising step S1 and step S2. Specifically:
Step S1: mixing raw material for preparing a delivery carrier with an agent to be loaded into carrier to prepare a delivery carrier loaded with agent.

The raw materials for preparing a delivery carrier can be selected according to the delivery carrier to be prepared. In some embodiments, the type or specific composition of the delivery carrier to be prepared is as described above in respect of the modified delivery carrier, and the raw materials for preparing a delivery carrier are selected according to the delivery carrier to be prepared. For example, when the delivery carrier to be prepared is a lipid nanoparticle comprising cationic lipid, DSPC, cholesterol and polymer-lipid (e.g., PEG-lipid), the raw materials for preparing the delivery carrier comprise cationic lipid, DSPC, cholesterol and polymer-lipid, and the amount of each component corresponds to the delivery carrier to be prepared.

The agent to be loaded into delivery carrier in step S1 can be selected according to the use of the modified delivery carrier.

In some embodiments, the agent to be loaded into the carrier in step S1 is as described above in parts of the modified delivery carrier, which will not be repeated here.

In some embodiments, mixing manner in step S1 is microfluidic. In some embodiments, the raw materials for preparing a delivery carrier and the agent to be loaded into the delivery carrier are prepared by microfluidic technology to prepare a delivery carrier loaded with agent.

Step S2: coupling the targeting domain with a delivery carrier loaded with an agent to prepare a modified delivery carrier, wherein the targeting domain can specifically target a surface antigen of a cell.

In some embodiments, the targeting domain in step S2 can specifically target a surface antigen of an immune cell or non-immune cell. In some embodiments, the targeting domain in step S2 is as described above in parts of the modified delivery carrier. In some embodiments, the coupling manner in step S2 is as described above in parts of the modified delivery carrier, which will not be repeated here. It can be understood that in other embodiments, the manner in which the targeting domain is coupled to the delivery carrier loaded with an agent is not limited to the above description, and may be other ways.

Compared with the traditional preparation of related agents (such as monocytes and CAR-T) in cell immunotherapy in which autologous immune cells need to be modified and cultured in vitro, the preparation method of a modified delivery carrier is simple to operate, does not need to collect immune cells from the body, and does not need to engineer the collected immune cells in vitro, with higher patient comfort.

In addition, the present invention also provides a kit for preparing a modified delivery carrier, the kit comprising: reagents for preparing a delivery carrier; an agent comprising or preparing a targeting domain which is capable of specifically targeting a surface antigen of an immune cell or a non-immune cell; and a linking agent for coupling the targeting domain with the delivery carrier.

In some embodiments, the reagents for preparing a delivery carrier correspond to the carrier to be prepared. The specific composition of the reagents for preparing a delivery carrier needs to be correspondingly selected according to the type of delivery carrier to be prepared. In some embodiments, the delivery carrier to be prepared is a lipid nanoparticle. The reagents for preparing delivery carrier comprise a cationic lipid. In some embodiments, the reagents for preparing delivery carrier also include one or more of a helper lipid, a structural lipid and a polymer-lipid (e.g., PEG-lipid). Cationic lipid, helper lipid, structural lipid and polymer-lipid are as described above in parts of the modified delivery carrier.

In some embodiments, the agent for preparing the targeting domain comprises a vector or host cell that encodes the gene of the targeting domain.

In some embodiments, the linking agent is selected according to the delivery carrier and the targeting domain, including, but not limited to, the description at which the delivery carrier is coupled with the targeting domain.

The kit for preparing a modified delivery carrier described above can prepare the modified delivery carrier described above, and is convenient and fast.

### IV. Pharmaceutical Composition

The present invention also provides a pharmaceutical composition comprising the modified delivery carrier according to any of the above embodiments.

In some embodiments, the pharmaceutical composition described above comprises one modified delivery carrier as described above.

In some embodiments, the pharmaceutical composition described above comprises at least two of the modified delivery carriers described above, and the various modified delivery carriers are loaded with different agents. When the agents loaded on the modified delivery carrier are different and used as different components in the pharmaceutical composition, the agents loaded in different modified delivery carriers can cooperate with each other in vivo to achieve the preset biological function. In an optional specific example, the above pharmaceutical composition comprises two, three, four or five modified delivery carriers described above. In some embodiments, the pharmaceutical composition described above comprises a first modified delivery carrier and a second modified delivery carrier, wherein the agents loaded in the first modified delivery carrier and the second modified delivery carrier are different. For example, the agent loaded in the first modified delivery carrier comprises an mRNA encoding Cas9, and the agent loaded in the second modified delivery carrier comprises gRNA.

In some embodiments, the above-mentioned pharmaceutical composition comprises at least two of the above-mentioned modified delivery carriers, and the targeting domains of each modified delivery carriers are different. When the targeting domains of the modified delivery carriers are different and as different components of the pharmaceutical composition, the agents loaded in different modified delivery carriers can specifically target different immune cells (improving therapeutic effect by activating different immune cells) or the same immune cell (improving therapeutic effect by increasing the binding rate of the modified delivery carrier to immune cells by different targets). In some embodiments, the pharmaceutical composition described above comprises a first modified delivery carrier and a second modified delivery carrier, wherein the targeting domains of the first modified delivery carrier and the second delivery carrier are different. In an optional specific example, the targeting domain of the first modified delivery carrier can specifically bind to CD19, and the targeting domain of the second modified delivery carrier can specifically bind to CD20.

In some embodiments, the above-mentioned pharmaceutical composition comprises at least two of the above-mentioned modified delivery carriers, wherein the targeting domains of various modified delivery carriers are different, and the agents of each modified delivery carrier are also different. In some embodiments, the pharmaceutical composition described above comprises a first modified delivery carrier and a second modified delivery carrier, wherein the targeting domains of the first modified delivery carrier and the second modified delivery carrier are different, and the agents loaded in the first modified delivery carrier and the second modified delivery carrier are different.

In some embodiments, the above pharmaceutical composition further comprises a pharmaceutically acceptable excipient. As used herein, the term "pharmaceutically acceptable" refers to being approved by a regulatory agency (e.g., National Food and Drug Administration (CFDA), United States Food and Drug Administration (FDA)) or recognized Pharmacopoeia (e.g., Chinese Pharmacopoeia, European Pharmacopoeia) for use in animals and/or humans. The term "excipient" refers to a substance that can be administered with the modified delivery carrier or lipid nanoparticle of the present invention, including but not limited to diluents, sweeteners, flavoring agents, wetting agents, adjuvants, glidants, preservatives, dyes/colorants, surfactants, dispersing agents, suspending agents, stabilizers, isotonic agents, solvents or emulsifiers.

The above pharmaceutical composition comprises the modified delivery carrier described above, and has corresponding advantages.

### V. Use of the modified delivery carrier or a pharmaceutical composition thereof in the preparation of gene-edited immune cells.

The present invention further provides use of the modified delivery carrier according to any one of the above embodiments or the pharmaceutical composition according to any one of the above embodiments in the preparation of gene-edited immune cells.

In some embodiments, the gene-edited immune cells include one or more of B cells, T cells, NK cells, DC cells, neutrophils, eosinophils, basophils, mast cells, monocytes and macrophages; in some embodiments, the gene-edited immune cells are B cells, T cells or NK cells.

In some embodiments, the gene-edited immune cells are CAR-T cells, TCR-T cells or CAR-NK cells, and the modified delivery carrier is loaded with a nucleic acid encoding a chimeric antigen receptor (CAR) or a T cell receptor (TCR). In some embodiments, the nucleic acid encoding a CAR is mRNA or DNA encoding a CAR (e.g., plasmid). In some embodiments, the nucleic acid encoding a TCR is mRNA or DNA encoding a TCR (e.g., plasmid).

In some embodiments, the modified delivery carrier is loaded with nucleic acids required for gene editing.

In some embodiments, the modified delivery carrier is loaded with nucleic acids required for gene editing based on CRISPR/CAS system, zinc finger nuclease system or TALEN system.

In some embodiments, the modified delivery carrier is loaded with an sgRNA and a nucleic acid encoding a Cas protein (e.g., Cas9) (e.g., mRNA or plasmid).

In some embodiments, the modified delivery carrier is loaded with an sgRNA, a nucleic acid encoding a Cas protein (e.g., Cas9) (e.g., mRNA or plasmid) and a donor DNA.

In some embodiments, the gene-edited immune cell is a B cell, and the modified delivery carrier is loaded with nucleic acids required for gene editing.

In some embodiments, the gene-edited immune cell is a B cell, and the modified delivery carrier is loaded with nucleic acids required for gene editing based on CRISPR/CAS system, zinc finger nuclease system or TALEN system.

In some embodiments, the gene-edited immune cell is a B cell, and the modified delivery carrier is loaded with an sgRNA and a nucleic acid encoding a Cas protein (e.g., Cas9) (e.g., mRNA or plasmid).

In some embodiments, the gene-edited immune cell is a B cell, and the modified delivery carrier is loaded with an sgRNA, a nucleic acid encoding a Cas protein (e.g., Cas9) (e.g., mRNA or plasmid) and a donor DNA.

In some embodiments, the gene-edited immune cell is a B cell, and the modified delivery carrier is loaded with an sgRNA, a nucleic acid encoding a Cas protein (e.g., Cas9) (e.g., mRNA or plasmid) and a nucleic acid encoding a therapeutic protein.

In some embodiments, the therapeutic protein is an antibody or a protein for the treatment of a rare disease.

In some embodiments, the therapeutic protein is an antibody.

In some embodiments, the nucleic acid encoding a Cas protein is mRNA or DNA encoding a Cas protein (e.g., plasmid), and the nucleic acid encoding a therapeutic protein is DNA.

The present invention further provides a method for preparing a gene-edited immune cell, comprising a step of:
contacting the modified delivery carrier according to any of the above embodiments or the pharmaceutical composition according to any of the above embodiments with an immune cell to prepare a gene-edited immune cell,

In some embodiments, the immune cell in contact with the modified delivery carrier or pharmaceutical composition in the method described above includes one or more of B cell, T cell, NK cell, DC cell, neutrophil, eosinophil, basophil, mast cell, monocyte and macrophage. In some embodiments, the immune cell in contact with the modified delivery carrier or pharmaceutical composition in the above method is B cell, T cell or NK cell.

In some embodiments, the method described above is performed in vitro. In some embodiments, the above method comprises contacting the modified delivery carrier according to any of the above embodiments or the pharmaceutical composition according to any of the above embodiments with an ex vivo immune cell to prepare a gene-edited immune cell.

In some embodiments, the method described above is performed in vivo. In some embodiments, the above method comprises administering the modified delivery carrier according to any one of the above embodiments or the pharmaceutical composition according to any one of the above embodiments into body (such as human body or animal body) so that it is in contact with immune cells in the body to prepare gene-edited immune cells.

In addition, the present invention also provides a gene-edited immune cell prepared by the method for preparing a gene-edited immune cell according to any of the above embodiments.

### VI. Use of a modified delivery carrier or a pharmaceutical composition thereof in the preparation of a modified immune cell.

The present invention further provides use of the modified delivery carrier according to any one of the above embodiments or the pharmaceutical composition according to any one of the above embodiments in the preparation of a modified immune cell.

In some embodiments, the modified immune cell includes one or more of B cell, T cell, NK cell, DC cell, neutrophil, eosinophil, basophil, mast cell, monocyte and macrophage. In some embodiments, the modified immune cell is B cell, T cell or NK cell.

In some embodiments, the modified immune cell is CAR-T cell, TCR-T cell or CAR-NK cell, and the modified delivery carrier is loaded with a nucleic acid encoding a CAR or TCR.

In some embodiments, the nucleic acid encoding a CAR or TCR is an mRNA or DNA encoding a CAR or TCR (e.g., a plasmid).

In some embodiments, the modified immune cell is B cell, and the modified delivery carrier is loaded with a nucleic acid encoding a therapeutic protein. In some embodiments, the therapeutic protein is an antibody. In some embodiments, the nucleic acid encoding a therapeutic protein is an mRNA or DNA (e.g., a plasmid).

In addition, the present invention also provides a method for preparing a modified immune cell, comprising the step of contacting the modified delivery carrier according to any of the above embodiments or the pharmaceutical composition according to any of the above embodiments with an immune cell.

In some embodiments, the immune cell in contact with the modified delivery carrier or pharmaceutical composition in the method described above includes one or more of B cell, T cell, NK cell, DC cell, neutrophil, eosinophil, basophil, mast cell, monocyte and macrophage. In some embodiments, the immune cell in contact with the modified delivery carrier or pharmaceutical composition in the above method is B cell, T cell or NK cell.

In some embodiments, the method described above is performed in vitro. In some embodiments, the above method comprises contacting the modified delivery carrier according to any of the above embodiments or the pharmaceutical composition according to any of the above embodiments with an ex vivo immune cell to prepare a modified immune cell.

In some embodiments, the method described above is performed in vivo. In some embodiments, the above method comprises administering the modified delivery carrier according to any of the above embodiments or the pharmaceutical composition according to any one of the above embodiments into body (e.g., human body or animal body) to make it in contact with an immune cell in the body to produce a modified immune cell.

In addition, the present invention also provides a modified immune cell prepared by the method for preparing a modified immune cell according to any of the above embodiments.

### VII. Use of the modified delivery carrier, the pharmaceutical composition, the gene-edited immune cell, or the modified immune cell in the preparation of a medicament.

The present invention further provides use of the modified delivery carrier or pharmaceutical composition according to any one of the above embodiments in the preparation of a medicament.

The present invention further provides use of the modified delivery carrier according to any of the above embodiments, the pharmaceutical composition according to any of the above embodiments, the gene-edited immune cell prepared by the method for preparing a gene-edited immune cell according to any of the above embodiments, or the modified immune cell prepared by the method for preparing a modified immune cell according to any of the above embodiments in the preparation of a medicament.

In some embodiments, the medicament is used for treating and/or preventing a disease.

In some embodiments, the medicament is used for treating and/or preventing an immune cell-associated disease.

In some embodiments, the medicament is used for treating and/or preventing a B cell-associated disease. In some embodiments, the modified delivery carrier targets B-cells, and the medicament is used for the treatment of a B-cell tumor. The modified carrier is loaded with a therapeutic agent for treating a B-cell tumor, wherein the therapeutic agent comprises one or more of a nucleic acid, a polypeptide, a protein and a small molecule. In some embodiments, the therapeutic agent has a tumorcidal effect. In some embodiments, the modified delivery carrier is loaded with a nucleic acid comprising one or both of RNA and DNA (e.g., a plasmid). In some embodiments, the modified delivery carrier is loaded with a nucleic acid comprising RNA. In some embodiments, the modified delivery carrier is loaded with a nucleic acid comprising an mRNA or a nucleic acid based on RNAi technology. In some embodiments, the nucleic acid based on RNAi technology is one or more selected from siRNA, antisense oligonucleotide (ASO) and miRNA. It should be noted that in other embodiments, the RNA loaded in the modified delivery carrier is not limited to the above mRNA, siRNA, miRNA and ASO, and may also be other RNAs.

In some embodiments, the gene-edited immune cell is a gene-edited B cell.

In some embodiments, the medicament is used to treat and/or prevent a T cell-associated disease.

In some embodiments, the modified delivery carrier targets T cell, and the medicament is used for the treatment of a T cell tumor. The modified carrier is loaded with a therapeutic agent for treating a T cell tumor, wherein the therapeutic agent comprises one or more of a nucleic acid, a polypeptide, a protein and a small molecule. In some embodiments, the therapeutic agent has a tumorcidal effect. In some embodiments, the modified delivery carrier is loaded with a nucleic acid comprising one or both of RNA and DNA (e.g., a plasmid). In some embodiments, the modified delivery carrier is loaded with a nucleic acid comprising RNA. In some embodiments, the modified delivery carrier is loaded with a nucleic acid comprising an mRNA or a nucleic acid based on RNAi technology. In some embodiments, the nucleic acid based on RNAi technology is one or more selected from siRNA, antisense oligonucleotide (ASO) and miRNA. It should be noted that in other embodiments, the RNA loaded in the modified delivery carrier is not limited to the above mRNA, siRNA, miRNA and ASO, and may also be other RNAs.

In some embodiments, the gene-edited immune cell is a gene-edited T cell.

In some embodiments, the modified immune cell is CAR-T cell or TCR-T cell.

In some embodiments, the medicament is used to treat and/or prevent a NK cell-associated disease.

In some embodiments, the modified delivery carrier targets NK cells, and the medicament is used for the treatment of a NK cell tumor. The modified carrier is loaded with a therapeutic agent for treating a NK cell tumor, wherein the therapeutic agent comprises one or more of a nucleic acid, a polypeptide, a protein and a small molecule. In some embodiments, the therapeutic agent has a tumorcidal effect. In some embodiments, the modified delivery carrier is loaded with a nucleic acid comprising one or both of RNA and DNA (e.g., a plasmid). In some embodiments, the modified delivery carrier is loaded with a nucleic acid comprising RNA. In some embodiments, the modified delivery carrier is loaded with a nucleic acid comprising an mRNA or a nucleic acid based on RNAi technology. In some embodiments, the nucleic acid based on RNAi technology is one or more selected from siRNA, antisense oligonucleotide (ASO) and miRNA. It should be noted that in other embodiments, the RNA loaded in the modified delivery carrier is not limited to the above mRNA, siRNA, miRNA and ASO, and may also be other RNAs.

In some embodiments, the gene-edited immune cell is a gene-edited NK cell.

In some embodiments, the modified immune cell is a CAR-NK cell.

In other embodiments, the modified delivery carrier targets B cells, and the modified delivery carrier is loaded with an sgRNA, a nucleic acid encoding a Cas protein, and a nucleic acid encoding a therapeutic protein. In some embodiments, the therapeutic protein is an antibody or a protein for treating a rare disease. In some embodiments, the nucleic acid encoding a Cas protein is an mRNA encoding a Cas protein, or the nucleic acid encoding a therapeutic protein is DNA (e.g., a plasmid).

In some embodiments, the medicament is used to treat and/or prevent an autoimmune disease.

In some embodiments, the modified delivery carrier targets B cells, and the modified delivery carrier is loaded with a nucleic acid, a polypeptide, a protein and a small molecule that inhibits autoimmunity.

In some embodiments, the modified delivery carrier targets B cells, and the modified delivery carrier is loaded with a nucleic acid encoding a protein (e.g., antibody) that inhibits autoimmunity.

In some embodiments, the protein inhibiting autoimmunity is an antibody. In some embodiments, the nucleic acid encoding a protein that inhibits autoimmunity is mRNA or DNA (e.g., a plasmid).

In some embodiments, the gene-edited immune cell is a gene-edited B cell.

In some embodiments, the modified delivery carrier targets T cells, and the modified delivery carrier is loaded with a nucleic acid encoding a protein of interest. In some embodiments, the nucleic acid is mRNA. In some embodiments, the protein of interest is a CAR or TCR. In some embodiments, the modified delivery carrier is loaded with an mRNA encoding a CAR or TCR.

In some embodiments, the gene-edited immune cell is a gene-edited T cell.

In some embodiments, the modified immune cell is CAR-T cell or TCR-T cell.

In some embodiments, the modified delivery carrier targets NK cells, and the modified delivery carrier is loaded with a nucleic acid encoding a protein of interest. In some embodiments, the nucleic acid is mRNA. In some embodiments, the protein of interest is a CAR. In some embodiments, the modified delivery carrier is loaded with an mRNA encoding a CAR.

In some embodiments, the gene-edited immune cell is a gene-edited NK cell.

In some embodiments, the modified immune cell is a CAR-NK cell.

In some embodiments, the disease is selected from the group consisting of rare diseases, infectious diseases, cancers, hereditary diseases, autoimmune diseases, diabetes, neurodegenerative diseases, cardiovascular diseases, renal vascular diseases and metabolic diseases.

In some embodiments, the cancers include one or more of lung cancer, gastric cancer, liver cancer, esophageal cancer, colon cancer, pancreatic cancer, brain cancer, lymph cancer, blood cancer and prostate cancer. Hereditary diseases include one or more of hemophilia, thalassemia and Gaucher's disease. Rare diseases include one or more of Brittle Bone Disease, Wilson Disease, Spinal Muscular Atrophy (SMA), Huntingdon's Disease, Rett Syndrome, Amyotrophic Lateral Sclerosis (ALS), Duchenne Type Muscular dystrophy, Friedrichs Ataxia, methylmalonic academia (MMA), Cystic Fibrosis (CF), glycogen storage disease 1a (GSD1a), glycogen storage disease III (GSD III), Crigler-Najjar syndrome, ornithine transcarbamylase deficiency (OTCD), propionic academia (PA), phenylketonuria (PKU), hemophilia A, hemophilia B, β -thalassemia, Lafora disease, Dravet syndrome (DS), Alexander disease, Leber's congenital amaurosis (LCA), myelodysplastic syndromes (MDS), Homocystinuria due to CBS deficiency. In addition, the rare diseases in www.orpha.net/consor/cgi-bin/DiseaseSearch List.php and rarediseases.info.nih.gov/diseases are also incorporated herein.

### VIII. Method for Preventing or Treating Diseases

In addition, the present invention also provides a method for preventing or treating a disease, comprising administering to a subject in need thereof the modified delivery carrier or pharmaceutical composition comprising a therapeutic agent according to any of the above embodiments.

In some embodiments, the method comprises administering to a subject in need thereof the modified delivery carrier or pharmaceutical composition comprising a therapeutic agent and targeting an immune cell according to any of the embodiments described above. The method is carried out by administering (e.g., injecting intravenously) the modified delivery carrier comprising a therapeutic agent to a subject in need thereof, wherein the carrier of the modified delivery carrier interacts with a targeting domain such that the modified delivery carrier is capable of targeting a particular immune cell, thereby activating immunomodulation for prophylactic or therapeutic purposes. Compared with the method for preventing or treating diseases using viral vectors, the above method for preventing or treating a disease does not need to collect immune cells from body, and does not need to engineer the collected immune cells in vitro, and the operation is simpler and the patient comfort is higher. In addition, due to the action of the targeting domain, the above method for preventing or treating a disease can specifically target a certain type or several types of immune cells, thereby reducing the non-specific delivery of the delivery carrier, thereby improving the effective delivery rate of the therapeutic agent, reducing the dose of the therapeutic agent and reducing side effects of the therapeutic agent. In some embodiments, the disease or disorder refers to the above description of the present invention describing the use of the modified delivery carrier or pharmaceutical composition in the preparation of a medicament.

In addition, the present invention also provides a method for treating and/or preventing an immune cell-associated disease, comprising the steps of: administering (for example, intravenously injecting) to a subject in need thereof the modified delivery carrier or pharmaceutical composition comprising a therapeutic agent according to any of the above embodiments, a gene-edited immune cell prepared by the method for preparing a gene-edited immune cell according to any of the above embodiments, a modified immune cell prepared by the method for preparing a modified immune cell according to any of the above embodiments.

In addition, the present invention also provides a method for treating and/or preventing a B-cell tumor, comprising the steps of: administering (for example, intravenously injecting) to a subject in need thereof the modified delivery carrier or pharmaceutical composition comprising a therapeutic agent and targeting B cell according to any of the above embodiments, a gene-edited B cell prepared by the method for preparing a gene-edited immune cell according to any of the above embodiments, a modified B cell prepared by the method for preparing a modified immune cell according to any of the above embodiments.

In some embodiments, the method of treating and/or preventing a B-cell tumor comprises: contacting the modified delivery carrier or pharmaceutical composition comprising a therapeutic agent and targeting B cell according to any of the embodiments described above with ex vivo isolated B cells of a subject in need thereof; and infusing the contacted ex vivo B cells back into the subject in need thereof.

In addition, the present invention also provides a method for treating and/or preventing a T-cell tumor, comprising administering (for example, intravenously injecting) to a subject in need thereof the modified delivery carrier or pharmaceutical composition comprising a therapeutic agent and targeting T cell according to any of the above embodiments, a gene-edited T cell prepared by the method for preparing a gene-edited immune cell according to any of the above embodiments, a modified T cell prepared by the method for preparing a modified immune cell according to any of the above embodiments.

In some embodiments, the method of treating and/or preventing a T-cell tumor comprises: contacting the modified delivery carrier or pharmaceutical composition comprising a therapeutic agent and targeting T cell according to any of the embodiments described above with an ex vivo T-cell of a subject in need thereof; and infusing the contacted ex vivo T cell back into the subject in need thereof.

In addition, the present invention also provides a method for treating and/or preventing a NK cell tumor, comprising administering (for example, intravenously injecting) to a subject in need thereof the modified delivery carrier or pharmaceutical composition comprising a therapeutic agent and targeting NK cell according to any of the above embodiments, a gene-edited NK cell prepared by the method for preparing a gene-edited immune cell according to any of the above embodiments, a modified NK cell prepared by the method for preparing a modified immune cell according to any of the above embodiments.

In some embodiments, the method for treating and/or preventing a NK cell tumor comprises: contacting the modified delivery carrier or pharmaceutical composition comprising a therapeutic agent and targeting NK cell according to any of the above embodiments with an ex vivo NK cell of a subject in need thereof; and infusing the contacted ex vivo NK cell back to the subject in need thereof.

In addition, the present invention also provides a method for treating and/or preventing an autoimmune disease, comprising administering (for example, intravenously injecting) to a subject in need thereof the modified delivery carrier or pharmaceutical composition comprising a therapeutic agent capable of preventing or treating an autoimmune disease, a gene-edited immune cell capable of preventing or treating an autoimmune disease prepared by the method for preparing a gene-edited immune cell according to any of the above embodiments, a modified immune cell capable of preventing or treating an autoimmune disease prepared by the method for preparing a modified immune cell according to any of the above embodiments.

### Examples

To make the objectives, technical solutions and advantages of the present invention clearer, the technical solutions of the present invention are described in detail below. Apparently, the described examples are merely some but not all of the embodiments of the present invention. All other embodiments obtained by a person of ordinary skill in the art based on the examples of the present invention without creative works shall fall within the protection scope of the present invention. In the following examples, ALC-0315 (CAS: 2036272-55-4) is commercially available.

### Example 1: Preparation of LNPs loaded with eGFP mRNA

An ethanol solution of one volume of lipid mixture (ALC-0315, DSPC, cholesterol, and DLSPE-PEG-Mal (Avanti Polar Lipids, catalog number 880126) with a molar ratio of 48:10:40.5:1.5) and an acetic acid-sodium acetate buffer of three volumes of eGFP mRNA (mRNA and lipid in 1:4 P/N ratio) were mixed by microfluidic to obtain a LNP mixed solution loaded with eGFP, and the obtained LNP mixed solution was dialyzed in Tris buffer saline (pH 7.4) to remove ethanol.

### Example 2: Preparation of Anti-CD19 Antibody-Modified LNPs

(1) Preparation of an antibody with sulfhydryl:
   (a) preparing a gene sequence (SEQ ID NO: 11) encoding the heavy chain variable region (VH, SEQ ID NO: 7) and a gene sequence (SEQ ID NO: 12) encoding the light chain variable region (VL, SEQ ID NO: 8) of the anti-CD19 antibody (FMC63) by gene synthesis method, splicing the gene sequence of the heavy chain variable region (VH) and the gene sequence of the heavy chain constant region (CH) of human IgG4 antibody containing S228P mutation by gene recombination, splicing the light chain variable region (VL) and the gene sequence of the light chain constant region (CL) of human IgG4 antibody, and then respectively cloning into the eukaryotic cell expression vector pCDNA3.0 to obtain the antibody heavy chain expression vector and the antibody light chain expression vector. Then, the antibody heavy chain expression vector and the antibody light chain expression vector were transfected into expression host cell HEK293 at a certain ratio for protein expression. After 1 week of host cell culture, the culture supernatant was collected, centrifuged to remove cells, the supernatant was filtered and loaded into a purification column containing Protein A for antibody purification and concentration, and then the anti-CD19 antibody (FMC63) was obtained, and the concentration was determined and stored.
   (b) 3.6 mg of the anti-CD19 antibody (FMC63) and 55.5µg of SATA (Sigma-Aldrich) were incubated at room temperature for half an hour, then 100µL of 0.5M hydroxylamine hydrochloride solution was added for deprotection of SATA, and after two hours, the antibody was purified with a dextran desalting column to obtain an antibody having an introduced sulfhydryl.
(2) Coupling the anti-CD19 antibody having an introduced sulfhydryl to LNPs loaded with eGFP mRNA: adding the anti-CD19 antibody after introducing sulfhydryl into 300µL of the LNP mixed solution prepared in Example 1, incubating at room temperature for 2 hours to allow sulfhydryl on the antibody to react with a maleimide functional group of LNPs. After the reaction was completed, the free antibody protein was isolated by using a Sepharose CL-4B gel filtration column to obtain anti-CD19 antibody modified LNPs, and finally the mRNA content and the encapsulation rate of the anti-CD19 antibody modified LNPs were detected by RiboGreen RNA quantification method.

By detection, the mRNA content of the anti-CD19 antibody modified LNPs was 59.03 ng/µL, and the encapsulation rate was 72.3%.

### Example 3: Targeted Delivery of Anti-CD19 Antibody-Modified LNPs

The experimental group (referred to as "anti-CD19/LNP-eGFP") was that the LNPs were encapsulated with eGFP mRNA and coupled to the anti-CD19 FMC63 antibody, the LNPs modified by the anti-CD19 antibody were diluted in PBS buffer, the diluted mRNA concentration was 50 ng/µL, and each of huHSC-NCG mice (GemPharmatech Co., Ltd, strain number: T037620) was injected in tail vein with 200uL of the anti-CD19 antibody modified LNPs, and after 9 hours, the peripheral blood and spleen of the mice were collected. The lymphocytes in the peripheral blood of mice were isolated to obtain a single cell suspension, the spleen was ground and then subjected to red blood cell lysis treatment to obtain a single cell suspension, a certain amount of the single cell suspension was blocked for Fc antibody, and stained with a fluorescent antibody (shown in Table 1 below) for 30 min, and after the cells were washed, the cells were fixed by formalin, and the eGFP expression of different cell types was analyzed by flow cytometry.

Control groups include LNP group without antibody modification (abbreviated as "LNP-eGFP") and LNP blank group (abbreviated as "LNP"), wherein LNP group without antibody modification refers to LNP encapsulating eGFP mRNA but not coupled to antibody, and LNP blank group refers to LNP neither encapsulating eGFP mRNA nor coupled to antibody. Except for the above differences, the composition of each of the control groups had no difference. In addition, the operation of each of the control groups was the same as that of the experimental group.

**Table 1**

| Antibody or Kit Name | Manufacturer | Catalog Number |
|---|---|---|
| BV510 anti-human CD3 | Biolegend | 300448 |
| Per/Cy5.5 anti-human CD4 | Biolegend | 300530 |
| BV650 anti-human CD8a | Biolegend | 301042 |
| BV421 anti-human CD22 | Biolegend | 302524 |
| BV785 anti-human CD19 | Biolegend | 363028 |
| APC anti-human CD45 | Biolegend | 304012 |
| Zombie NIR^{™} Fixable Viability Kit | Biolegend | 423105 |

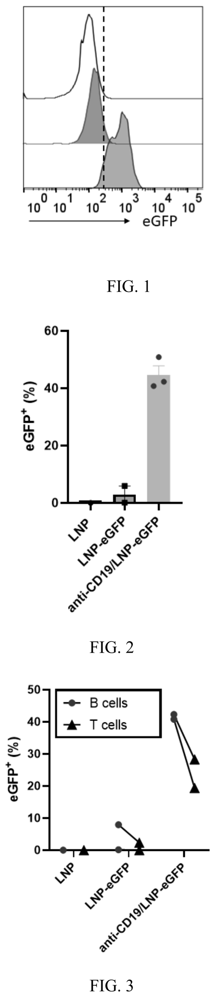

Results analysis: 9 hours after tail vein injection, the expression level of eGFP in CD45⁺CD22⁺ B cells in PBMC was analyzed by flow cytometry, and the results were shown in FIG. 1, and the result analysis of percentage (%) of eGFP⁺ cells in CD45⁺CD22⁺ B cells was shown in FIG. 2. Meanwhile, the expression level of eGFP in T cells (CD45⁺CD3⁺) and the expression level of eGFP in B cells of the same animal were compared, and the results were shown in FIG. 3.

It can be seen from FIG. 2 that compared with the LNP group and the LNP-eGFP group, eGFP⁺% in B cells in the anti-CD19/LNP-eGFP group was increased to more than 40%. It can be seen from FIG. 3 that the eGFP expression in B cells was significantly higher than that in T cells in the anti-CD19/LNP-eGFP group. It can be seen that the anti-CD19 antibody modified LNPs had significant B cell-targeting delivery characteristics.

### Example 4: Preparation of LNPs loaded with Cre mRNA

One volume of the lipid mixture (ALC-0315, DSPC, cholesterol, and DLSPE-PEG-Mal at a molar ratio of 48:10:40.5:1.5) in ethanol solution and 3 volumes of Cre mRNA (P/N ratio of 1:4) in acetic acid-sodium acetate buffer were mixed by microfluidic to obtain a LNP mixed solution loaded with Cre mRNA. The obtained LNP mixed solution was dialyzed in Tris buffer saline (pH 7.4) to remove ethanol.

### Example 5: Preparation of Anti-CD5 Antibody-Modified LNPs

(1) Preparation of an antibody having sulfhydryl:
   0.5 mg of an anti-CD5 antibody (hybridoma clone 53-7.3, Biolegend, catalog number 100602) and 7.7µg of SATA (Sigma-Aldrich) were incubated at room temperature for half an hour, then 100µL of 0.5 M hydroxylamine hydrochloride solution was added to deprotect SATA, and after two hours, the antibody was purified using a dextran desalting column to obtain an antibody having an introduced sulfhydryl.
(2) Coupling the anti-CD5 antibody having an introduced sulfhydryl to LNPs loaded with Cre mRNA: the anti-CD5 antibody after introducing sulfhydryl was added to 300µL of the LNP mixed solution prepared in Example 4, and incubated at room temperature for 2 hours to allow the sulfhydryl on the antibody to react with a maleimide functional group of LNPs. After the reaction was completed, free antibody protein was isolated by using a Sepharose CL-4B gel filtration column to obtain anti-CD5 antibody modified LNPs, and finally the mRNA content and the encapsulation rate of the anti-CD5 antibody modified LNPs were detected by RiboGreen RNA quantification method.

By detection, the mRNA content of the anti-CD5 antibody modified LNPs was 49.42 ng/µL, and the encapsulation rate was 76.1%.

### Example 6: Targeted Delivery of Anti-CDS Antibody-Modified LNPs

The experimental group (abbreviated as "T-LNP-Cre") refers to LNPs encapsulating Cre mRNA and coupled to anti-CD5 antibody (hybridoma clone 53-7.3, Biolegend, cat. no. 100602), the T-LNP-Cre was diluted in PBS buffer, the diluted mRNA concentration was 38 ng/µL, each of TdTomato mice was injected in tail vein with 200µL of the anti-CD5 antibody modified LNPs, and the peripheral blood and spleen of the mice were collected after 48 hours. The lymphocytes of the peripheral blood of mice were isolated to obtain a single cell suspension, the spleen was ground and then subjected to red blood cell lysis treatment to obtain a single cell suspension, a certain amount of the single cell suspension was blocked for Fc antibody, and simultaneously stained using a fluorescent antibody (shown in Table 2 below) for 30 min, and after the cells were washed, the cells were fixed by formalin, and the TdTomato expression of different cell types was analyzed by flow cytometry.

Control groups include LNP group without antibody modification (abbreviated as "LNP-Cre") and LNP blank group (abbreviated as "LNP"), wherein LNP group without antibody modification refers to LNP encapsulating Cre mRNA but not coupled to antibody, and LNP blank group refers to LNP neither encapsulating Cre mRNA not coupled to antibody. Except for the above differences, the composition of each of the control groups had no differences, and in addition, the operation of each of the control groups was the same as that of the experimental group.

**Table 2**

| Antibody or Kit Name | Manufacturers | Catalog Number |
|---|---|---|
| BV 510 anti-mouse CD3ε | Biolegend | 100353 |
| APC anti-mouse CD45 | Biolegend | 103111 |
| Zombie NIR^{™} Fixable Viability Kit | Biolegend | 423105 |

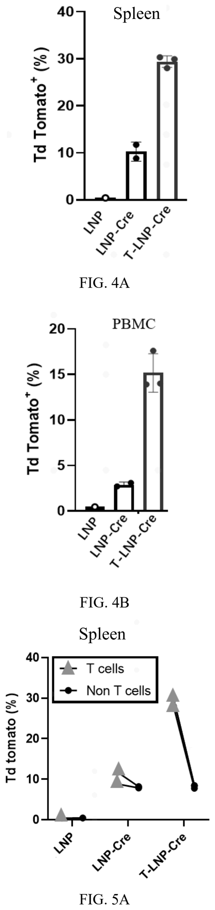

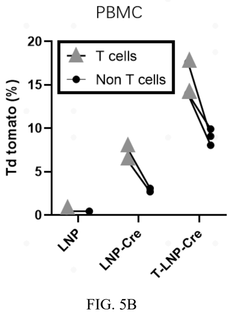

Results analysis: 48 hours after tail vein injection, the expression level of TdTomato in CD45⁺CD3⁺ T cells in spleen and PBMC was analyzed by flow cytometry, and the result analysis of the percentage (%) of TdTomato⁺ cells in CD45⁺CD3⁺ T cells were shown in FIGs. 4A-4B. Meanwhile, the expression level of TdTomato in T cells (CD45⁺CD3⁺) and the expression level of TdTomato in non-T cells of the same animal were compared, and the results were shown in FIGs. 5A-5B.

It can be seen from FIG. 4A to FIG. 4B that compared with the LNP group and the LNP-Cre group, TdTomato⁺ % in the spleen T cells in the T-LNP-Cre group was increased to 30%, and TdTomato⁺% in T cells of the PBMC were increased to more than 15%. It can be seen from FIG. 5A to FIG. 5B that the expression of TdTomato in T cells of spleen and PBMC in the T-LNP-Cre group was significantly higher than that in non-T cells. It can be seen that the anti-CD5 antibody modified LNPs had significant T cell targeting delivery characteristics.

## Claims

1. A modified delivery carrier comprising a delivery carrier and a targeting domain coupled to the delivery carrier, wherein the delivery carrier is for loading an agent and the targeting domain is capable of specifically targeting a surface antigen of an immune cell.

2. The modified delivery carrier of claim 1, wherein the delivery carrier is one selected from the group consisting of a lipid nanoparticle, a liposome, a cationic protein, a vesicle, a microparticle, a polymer and a micelle.

3. The modified delivery carrier of any one of claims 1-2, wherein the immune cell comprises one or more of T cell, B cell, NK cell, DC cell, neutrophil, eosinophil, basophil, mast cell, monocyte and macrophage; preferably, the immune cell is B cell, T cell or NK cell.

4. The modified delivery carrier of any one of claims 1-3, wherein the surface antigen comprises one or more of:
a pan-T cell antigen, a pan-B cell antigen, a pan-NK cell antigen, a pan-DC cell antigen, a pan-neutrophil antigen, a pan-eosinophil antigen, a pan-basophil antigen, a pan-monocyte antigen, and a pan-macrophage antigen; preferably, the surface antigen is a pan-B cell antigen.

5. The modified delivery carrier of any one of claims 1-4, wherein the surface antigen comprises one or more of:
CD1, CD2, CD5, CD6, CD9, CD10, CD11, CD17, CD18, CD19, CD20, CD21, CD22, CD23, CD24, CD25, CD26, CD27, CD29, CD30, CD31, CD32a, CD32b, CD35, CD37, CD38, CD39, CD40, CD44, CD45, CD45RA, CD45RB, CD45RC, CD46, CD47, CD48, CD49b, CD49c, CD49d, CD50, CD52, CD53, CD54, CD55, CD58, CD60a, CD62L, CD63, CD68, CD69, CD70, CD72, CD73, CD74, CD75, CD75S, CD77, CD79a, CD79b, CD80, CD81, CD82, CD83, CD84, CD85E, CD85I, CD85J, CD85L, CD85M, CD86, CD92, CD95, CD97, CD98, CD99, CD100, CD102, CD108, CD119, CD120, CD121, CD122, CD124, CD125, CD126, CD127, CD130, CD132, CD137, CD138, CD139, CD147, CD148, CD150, CD151, CD152, CD162, CD164, CD166, CD167a, CD170, CD171, CD175, CD175s, CD179, CD180, CD184, CD185, CD192, CD196, CD197, CD200, CD205, CD210, CD212, CD213a1, CD213a2, CD215, CD217, CD218, CD220, CD221, CD222, CD224, CD225, CD226, CD227, CD229, CD230, CD232, CD245, CD252, CD253, CD257, CD258, CD261, CD262, CD263, CD264, CD267, CD268, CD269, CD270, CD272, CD274, CD275, CD277, CD279, CD283, CD289, CD290, CD295, CD298, CD300a, CD300c, CD305, CD306, CD307, CD314, CD315, CD316, CD317, CD319, CD321, CD327, CD328, CD329, CD338, CD351, CD352, CD353, CD354, CD355, CD357, CD358, CD360, CD361, CD362, CD363, CD367, CD369 and GPRC5D;
preferably, the surface antigen comprises one or more of CD10, CD19, CD20, CD21, CD22, CD23, CD27, CD32b, CD38, CD40, CD49d, CD52, CD79a, CD79b, CD80, CD86, CD126, CD138, CD267, CD268, CD269, CD275, CD351, CD360 and GPRC5D; preferably, the surface antigen comprises one or two of CD19 and CD22.

6. The modified delivery carrier of any one of claims 1-5, wherein the targeting domain comprises one or more of a nucleic acid, a polypeptide, an antibody and a small molecule.

7. The modified delivery carrier of claim 6, wherein the targeting domain is an antibody;
preferably, the antibody is one or more selected from the group consisting of an anti-CD10 antibody, an anti-CD19 antibody, an anti-CD20 antibody, an anti-CD21 antibody, an anti-CD22 antibody, an anti-CD23 antibody, an anti-CD27 antibody, an anti-CD32b antibody, an anti-CD38 antibody, an anti-CD40 antibody, an anti-CD49d antibody, CD52 antibody, an anti-CD79a antibody, an anti-CD79b antibody, an anti-CD80 antibody, an anti-CD86 antibody, an anti-CD126 antibody, an anti-CD138 antibody, an anti-CD267 antibody, an anti-CD268 antibody, an anti-CD269 antibody, an anti-CD275 antibody, an anti-CD351 antibody, an anti-CD360 antibody and an anti-GPRCSD antibody; preferably, the targeting domain is one or two of an anti-CD19 antibody and an anti-CD22 antibody.

8. The modified delivery carrier of any one of claims 1-7, wherein the targeting domain is an antibody which is an anti-CD19 antibody.

9. The modified delivery carrier of claim 8, wherein the anti-CD19 antibody comprises a heavy chain variable region comprising an HCDR1 whose amino acid sequence is set forth in SEQ ID NO: 1, an HCDR2 whose amino acid sequence is set forth in SEQ ID NO: 2 and an HCDR3 whose amino acid sequence is set forth in SEQ ID NO: 3, and a light chain variable region comprising an LCDR1 whose amino acid sequence is set forth in SEQ ID NO: 4, an LCDR2 whose amino acid sequence is set forth in SEQ ID NO: 5 and an LCDR3 whose amino acid sequence is set forth in SEQ ID NO: 6.

10. The modified delivery carrier of claim 9, wherein the heavy chain variable region comprises the amino acid sequence as set forth in SEQ ID NO: 7, and the light chain variable region comprises the amino acid sequence as set forth in SEQ ID NO: 8.

11. The modified delivery carrier of any one of claims 1-6, wherein the targeting domain is an antibody which is an anti-CD5 antibody.

12. The modified delivery carrier of any one of claims 1-11, wherein the targeting domain and the delivery carrier are coupled in a coupling manner of covalent coupling or non-covalent coupling;
preferably, the non-covalent coupling comprises one or more of electrostatic interaction, van der Waals force, ring stacking and hydrophobic interaction.

13. The modified delivery carrier of claim 12, wherein the targeting domain has a first coupling group and the delivery carrier has a second coupling group, wherein the first coupling group and the second coupling group are independently selected from or derived from one or more of sulfhydryl, azido, amino, amido, a polypeptide whose amino acid sequence is LPXTG, carbonyl, avidin, a sulfhydryl-reactive functional group, alkynyl, alkenyl, an amino reactive functional group, polyglycine, aminooxy (-O-NH₂) and biotin, wherein X is any amino acid, preferably the avidin is streptavidin;
preferably, one of the first coupling group and the second coupling group is selected from or derived from one or more of sulfhydryl, azido, amino, amido, a polypeptide whose amino acid sequence is LPXTG, carbonyl and avidin, and the other is selected from or derived from one or more of sulfhydryl reactive functional group, alkynyl, alkenyl, amino reactive functional group, polyglycine, aminooxy (-O-NH₂) and biotin;
preferably, one of the first coupling group and the second coupling group is sulfhydryl, and the other is a sulfhydryl reactive functional group; or one of the first coupling group and the second coupling group is azido, and the other is alkenyl or alkynyl; or one of the first coupling group and the second coupling group is amino, and the other is an amino reactive functional group; or one of the first coupling group and the second coupling group is amido, and the other is amino; or one of the first coupling group and the second coupling group is a polypeptide whose amino acid sequence is LPXTG, and the other is polyglycine; or one of the first coupling group and the second coupling group is avidin or streptavidin, and the other is biotin; or one of the first coupling group and the second coupling group is carbonyl, and the other is aminooxy (-O-NH₂).

14. The modified delivery carrier of claim 13, wherein the targeting domain and the delivery carrier are coupled in a coupling manner of covalent coupling, wherein the first coupling group is sulfhydryl, and the second coupling group is alkenyl.

15. The modified delivery carrier of any one of claims 1-14, wherein the delivery carrier is a lipid nanoparticle;
preferably, the lipid nanoparticle comprises a polymer-lipid, and the targeting domain is coupled to the polymer-lipid;
preferably, the polymer for forming the polymer-lipid comprises one or two of a hydrophilic polymer and an amphoteric polymer;
preferably, the hydrophilic polymer comprises one or more of polyethylene glycol, polyoxazoline, polyglycerol, poly(hydroxypropyl methacrylate), poly(2-hydroxyethyl methacrylate), poly(N-(2-hydroxypropyl) methacrylamide), poly(vinylpyrrolidone), poly(N,N-dimethylacrylamide), poly(N-acryloyl morpholine), glycosaminoglycan, heparin, hyaluronic acid, poly(sialic acid), elastin like polypeptide, serum albumin and CD47;
and/or, the amphoteric polymer comprises one or more of poly(carboxybetaine), poly(sulfobetaine), phosphobetaine-based polymer, and phosphorylcholine polymer.

16. The modified delivery carrier of claim 15, wherein the polymer-lipid is DSPE-PEG-Mal.

17. The modified delivery carrier of claim 16, wherein the sulfhydryl is introduced by reaction of SATA with an antibody and the alkenyl is derived from a maleimide functional group in the DSPE-PEG-Mal.

18. The modified delivery carrier of any of claims 15-17, wherein the lipid nanoparticle comprises a cationic lipid, a helper lipid, a structural lipid and a polymer-lipid;
preferably, the cationic lipid is one or two of ALC-0315 and SM-102;
preferably, the molar ratio of the cationic lipid, the helper lipid, the structural lipid and the polymer-lipid is (25-75): (5-45): (0-50): (0.5-5) based on the total amount of the cationic lipid, the helper lipid, the structural lipid and the polymer-lipid.

19. The modified delivery carrier of claim 18, wherein the cationic lipid is ALC-0315, the helper lipid is DSPC, the structural lipid is cholesterol, and the polymer-lipid is DSPE-PEG-Mal.

20. The modified delivery carrier of any one of claims 1-19, wherein the delivery carrier is loaded with an agent; preferably, the agent is encapsulated within the delivery carrier;
preferably, the agent comprises one or more of a therapeutic agent, an imaging agent, a diagnostic agent, a contrast agent, a labelling agent, a detection agent and a disinfectant.

21. The modified delivery carrier of claim 20, wherein the agent comprises a therapeutic agent comprising one or more of a nucleic acid, a polypeptide, a protein, an antibody, and a small molecule.

22. The modified delivery carrier of claim 21, wherein the agent comprises a therapeutic agent, wherein the active ingredient of the therapeutic agent comprises a nucleic acid;
preferably, the nucleic acid comprises DNA and/or RNA; preferably, the nucleic acid comprises one or more of an mRNA, a nucleic acid based on RNAi technology and an sgRNA; preferably, the nucleic acid based on RNAi technology is one or more selected from an siRNA, an antisense oligonucleotide and an miRNA;
preferably, the DNA or the mRNA comprises one or more of a DNA or mRNA encoding a Cas protein, a DNA or mRNA encoding a CAR, a DNA or mRNA encoding a TCR, and a DNA or mRNA encoding a therapeutic protein or polypeptide;
preferably, the mRNA is an mRNA encoding a Cas protein, an mRNA encoding a CAR or an mRNA encoding a TCR;
preferably, the DNA is a DNA encoding a therapeutic protein or polypeptide;
preferably, the therapeutic protein is an antibody, or the therapeutic protein is a protein or polypeptide for treating a rare disease;
preferably, the nucleic acid comprises a gRNA and an mRNA encoding a Cas protein;
preferably, the Cas protein is Cas9;
preferably, the nucleic acid comprises a modified nucleotide.

23. The modified delivery carrier of claim 22, wherein the mRNA comprised in the agent further comprises a regulatory element;
preferably, the regulatory element comprises an microRNA binding site;
preferably, the mRNA comprised in the agent comprises one or more microRNA binding sites; preferably, the microRNA binding sites are the same or at least partially different;
preferably, the microRNA binding sites are different, and the microRNA binding sites bind to the same microRNA or different microRNAs;
preferably, the microRNA binding sites bind to different microRNAs, and the different microRNAs are from the same cell and/or tissue, or the different microRNAs are from different cells and/or tissues;
preferably, the mRNA in the modified delivery carrier comprises 3'-UTR, and the position of the microRNA binding site is one or more selected from 5' end close to 3'-UTR, a region between 5' end and 3' end of 3'-UTR, and 3' end close to 3'-UTR;
preferably, the mRNA in the modified delivery carrier comprises 5'-UTR, and the position of the microRNA binding site is one or more selected from 5' end close to 5'-UTR, a region between 5' end and 3' end of 5'-UTR, and 3' end close to 5'-UTR;
preferably, the mRNA in the modified delivery carrier comprises 5'-UTR, 3'-UTR and a microRNA binding site, wherein the position of the microRNA binding site is one selected from a 5' end close to the 3'-UTR, a region located between the 5' end and the 3' end of the 3'-UTR, a 3' end close to the 3'-UTR, a 5' end close to the 5'-UTR, a region between the 5' end and the 3' end of the 5'-UTR, and a 3' end close to the 5'-UTR;
preferably, the mRNA in the modified delivery carrier comprises 5'-UTR, 3'-UTR and microRNA binding sites, and the positions of the microRNA binding sites are one or more selected from a 5' end close to 3'-UTR, a region between 5' end and 3' end of 3'-UTR, a 3' end close to 3'-UTR, a 5' end close to 5'-UTR, a region between 5' end and 3' end of 5'-UTR, and a 3' end close to 5'-UTR.

24. The modified delivery carrier of any one of claims 1-23, wherein the modified delivery carrier comprises a plurality of targeting domains coupled with the delivery carrier;
preferably, the plurality of targeting domains are independently coupled to the delivery carrier, or at least one of the plurality of targeting domains is coupled to the delivery carrier, and at least one of the remaining targeting domains is coupled to the targeting domain coupled to the delivery carrier.

25. A method of preparing the modified delivery carrier of any one of claims 1-24, comprising steps of:
mixing raw materials for preparing a delivery carrier with an agent to prepare a delivery carrier loaded with the agent; and
coupling a targeting domain with the agent-loaded delivery carrier to produce a modified delivery carrier, wherein the targeting domain is capable of specifically targeting a surface antigen of an immune cell.

26. A pharmaceutical composition comprising the modified delivery carrier of any one of claims 1-24.

27. The pharmaceutical composition of claim 26, wherein the pharmaceutical composition comprises at least two the modified delivery carriers, wherein the modified delivery carriers are loaded with different agents or the targeting domains of the delivery carriers are different.

28. Use of the modified delivery carrier of any one of claims 1-24 or the pharmaceutical composition of any one of claims 26-27 in the preparation of a gene-edited immune cell;
preferably, the gene-edited immune cell comprises one or more of B cell, T cell, NK cell, DC cell, neutrophil, eosinophil, basophil, mast cell, monocyte and macrophage; preferably, the gene-edited immune cell is B cell, T cell or NK cell;
preferably, the gene-edited immune cell is CAR-T cell, TCR-T cell or CAR-NK cell, and the delivery carrier of the modified delivery carrier is loaded with a nucleic acid encoding a CAR or TCR; preferably, the nucleic acid encoding a CAR or TCR is an mRNA encoding a CAR or TCR;
or, the gene-edited immune cell is B cell wherein the modified delivery carrier is loaded with an sgRNA and a nucleic acid encoding a Cas protein; or, the gene-edited immune cell is B cell wherein the modified delivery carrier is loaded with an sgRNA, a nucleic acid encoding a Cas protein, and a donor DNA; or, the gene-edited immune cell is B cell wherein the modified delivery carrier is loaded with an sgRNA, a nucleic acid encoding a Cas protein, and a nucleic acid encoding a therapeutic protein; preferably, the therapeutic protein is a protein for treating a rare disease, or the therapeutic protein is an antibody; preferably, the nucleic acid encoding a Cas protein is an mRNA encoding a Cas protein, and the nucleic acid encoding a therapeutic protein is DNA.

29. Use of the modified delivery carrier of any one of claims 1-24 or the pharmaceutical composition of any one of claims 26-27 in the preparation of a medicament;
preferably, the medicament is used for treating and/or preventing a disease;
preferably, the modified delivery carrier targets B cell, and the medicament is used for treating a B-cell tumor;
or the modified delivery carrier targets B cell, and the medicament is used for treating an autoimmune disease;
or the modified delivery carrier targets T cell, and the medicament is used for treating a T cell tumor;
or the modified delivery carrier targets NK cell, and the medicament is used for treating an NK cell tumor;
preferably, the modified delivery carrier comprises one or more of a nucleic acid, a polypeptide, a protein and a small molecule;
preferably, the nucleic acid comprises one or two of RNA and DNA;
Preferably, the nucleic acid comprises mRNA, or the nucleic acid comprises a nucleic acid based on RNAi technology; preferably, the nucleic acid based on RNAi technology is one or more selected from siRNA, antisense oligonucleotide and miRNA.

30. Use of the modified delivery carrier of any one of claims 1-24 or the pharmaceutical composition of any one of claims 26-27 in the preparation of a medicament;
preferably, the medicament is used for treating and/or preventing a disease;
preferably, the modified delivery carrier targets B cell, and the modified delivery carrier is loaded with an sgRNA, a nucleic acid encoding a Cas protein and a nucleic acid encoding a therapeutic protein; preferably, the therapeutic protein is a protein for treating a rare disease, or the therapeutic protein is an antibody;
preferably, the nucleic acid encoding a Cas protein is an mRNA encoding a Cas protein; and/or the nucleic acid encoding a therapeutic protein is DNA.

31. Use of the modified delivery carrier of any one of claims 1-24 or the pharmaceutical composition of any one of claims 26-27 in the preparation of a medicament;
preferably, the medicament is used for treating and/or preventing a disease;
preferably, the modified delivery carrier targets T cell, and the modified delivery carrier is loaded with a nucleic acid encoding a desired protein; preferably, the nucleic acid is an mRNA, preferably, the desired protein is a CAR or TCR; furthermore, the modified delivery carrier is loaded with an mRNA encoding a CAR or TCR;
or, the modified delivery carrier targets NK cell, and the modified delivery carrier is loaded with a nucleic acid encoding a desired protein; preferably, the nucleic acid is an mRNA, preferably, the desired protein is a CAR; furthermore, the modified delivery carrier is loaded with an mRNA encoding a CAR.

32. The use of any one of claims 29-31, wherein the medicament is used for treating and/or preventing one or more of rare disease, cancer, infectious disease, autoimmune disease, metabolic disease, neuropathic disease, cardiovascular disease, transplant rejection response, inflammatory response, hereditary disease and musculoskeletal disease;
preferably, the rare disease comprises one or more of Brittle Bone Disease, Wilson disease, spinal muscular atrophy, Huntington's disease, Rett syndrome, amyotrophic lateral sclerosis, Duchenne Type Muscular Dystrophy, Friedrichs ataxia, Methylmalonic Acidemia, cystic fibrosis, glycogen storage disease 1a, glycogen storage disease III, Crigler-Najjar syndrome, ornithine transcarbamylase deficiency, Propionic Acidemia, phenylketonuria, hemophilia A, hemophilia B, β-thalassemia, Lafora disease, Dravet syndrome, Alexander disease, Leber's congenital amaurosis, myelodysplastic syndromes, and Homocystinuria due to CBS deficiency;
the cancer comprises one or more of hematological malignancy, lung cancer, liver cancer, kidney cancer, head and neck cancer, esophageal cancer, gastric cancer, colorectal cancer, pancreatic cancer, brain cancer, prostate cancer, gallbladder cancer, ovarian cancer, breast cancer, cervical cancer, endometrial cancer, bladder cancer and melanoma;
the infectious disease comprises a disease caused by infection with one or more of virus, fungus, and bacterium;
the autoimmune disease comprises one or more of acute idiopathic thrombocytopenic purpura, chronic idiopathic thrombocytopenic purpura, systemic lupus erythematosus, rheumatoid arthritis, psoriasis, inflammatory bowel disease, multiple sclerosis, celiac disease, type 1 diabetes mellitus, and diffuse toxic goiter;
the hereditary disease comprises one or more of hemophilia, thalassemia, and Gaucher's disease;
the neuropathic disease comprises one or more of amyotrophic lateral sclerosis, Alzheimer's disease, and glioma.

33. A kit for preparing the modified delivery carrier of any one of claims 1-24, comprising:
reagents for preparing a delivery carrier;
an agent comprising or preparing a targeting domain, wherein the targeting domain is capable of specifically targeting a surface antigen of an immune cell; and
a linking agent for coupling the targeting domain to the delivery carrier.

34. Use of the modified delivery carrier of any one of claims 1-24 or the pharmaceutical composition of any one of claims 26-27 in preparing a modified immune cell;
preferably, the modified immune cell comprises one or more of B cell, T cell, NK cell, DC cell, neutrophil, eosinophil, basophil, mast cell, monocyte and macrophage; preferably, the modified immune cell is B cell, T cell or NK cell;
preferably, the modified immune cell is a CAR-T cell, TCR-T cell or CAR-NK cell, and the delivery carrier of the modified delivery carrier is loaded with a nucleic acid encoding a CAR or TCR; preferably, the nucleic acid encoding a CAR or TCR is an mRNA encoding a CAR or TCR.
